# EUROPÄISCHE PATENTSCHRIFT

(11) **EP 3 653 176 B1**
(45) Veröffentlichungstag und Bekanntmachung des Hinweises auf die Patenterteilung: **21.10.2026**
(21) Anmeldenummer: 19208645.2
(22) Anmeldetag: 26.02.2016
(51) Int. Cl.: A61F 2/76, A61F 2/30, A61F 2/78, A61F 2/80, A61F 2/60, A61F 2/50

(54) **VORRICHTUNG, SET UND VERFAHREN ZUM ERSTELLEN EINES GIPSABDRUCKS EINES KÖRPERGLIEDSTUMPFS EINES PATIENTEN ZUM FERTIGEN EINES PROTHESENSCHAFTS SOWIE ADAPTER**
DEVICE, SET AND METHOD FOR CREATING A PLASTER IMPRESSION OF A BODYPART STUMP OF A PATIENT FOR THE MANUFACTURE OF A PROSTHESIS SHAFT AND ADAPTER
DISPOSITIF, ENSEMBLE ET PROCÉDÉ DE PRODUCTION D'UNE IMPRESSION EN PLÂTRE D'UN MOIGNON DE MEMBRE CORPOREL D'UN PATIENT PERMETTANT DE FABRIQUER UNE PROTHÈSE AINSI QU'ADAPTATEUR

(30) Priorität: 26.02.2015 DE 102015102798; 16.03.2015 DE 102015103864; 18.03.2015 DE 102015104070; 15.04.2015 DE 102015105775; 28.04.2015 DE 102015106551; 30.04.2015 DE 102015106821; 10.06.2015 DE 102015109198; 24.06.2015 DE 102015110156; 26.06.2015 DE 102015110355; 28.08.2015 DE 102015114383; 25.01.2016 DE 102016101257
(43) Veröffentlichungstag der Anmeldung: 20.05.2020
(62) Dokumentnummer(n) der früheren Anmeldung(en) nach Art. 76 EPÜ: 16706645.5 / 3 261 587
(73) Patentinhaber: Romedis GmbH, 83115 Neubeuern (DE)
(72) Erfinder: RADSPIELER, Andreas, 83115 Neubeuern (DE)
(74) Vertreter: Pfenning, Meinig & Partner mbB

(56) Entgegenhaltungen:
- GB-A- 2 232 598
- US-A- 4 735 754
- US-A1- 2002 043 738

## Beschreibung

Die vorliegende Erfindung betrifft eine Vorrichtung gemäß dem Oberbegriff des Anspruchs 1. Sie betrifft ferner ein Set gemäß Anspruch 7, Verfahren gemäß den Ansprüchen 8, 9 und 10, sowie eine Einheit gemäß Anspruch 11.

Beinamputierte Personen können mittels Beinprothesen Mobilität zurückgewinnen. Moderne Beinprothesen umfassen verschiedene Module (Prothesenschaft, Knie-, Unterschenkel-, und Fußmodule), welche kombiniert werden können, um die unterschiedlichsten Bedürfnisse der Prothesenträger (im Folgenden auch kurz als Träger oder Patienten bezeichnet) hinsichtlich Grundmobilität, sportlicher Betätigung und ästhetischen Vorstellungen zu erfüllen.

Einige der vorgenannten Module können standardisiert sein und ggf. in verschiedenen Größen, Längen oder anderen Ausgestaltungen vorliegen, aus welchen das jeweils geeignete Modul ausgewählt wird.

Dies gilt i. a. R. allerdings nicht für den Prothesenschaft, jenes Modul der Prothese, welches die Verbindung zwischen dem mechanischen Ersatz der Extremität und dem verbliebenen Körpergliedstumpf (im Folgenden auch kurz als Stumpf bezeichnet) des Prothesenträgers, beispielsweise einem Unterschenkelstumpf, einem Oberschenkelstumpf oder einem Armstumpf herstellt.

Der Prothesenschaft wird im Stand der Technik individuell an den Stumpf des späteren Trägers angepasst. Hierzu wird zunächst mittels einer feuchten Gipsbinde ein Gipsabdruck gefertigt, der die Grundlage des Prothesenschafts sein wird und dessen Form wesentlich bestimmt.

Das Fertigen des Schafts einer Prothese erfordert handwerkliches Geschick; die Frage, wie passend der Schaft später beim Tragen empfunden wird, hängt maßgeblich von der Geschicklichkeit und Erfahrung des Orthopädietechnikers und den weiteren Umständen, unter denen der Gipsabdruck gemacht wurde, ab.

Die Frage, wie passend der Schaft beim Tragen empfunden wird, hängt auch davon ab, wie sehr die Umstände, unter denen der Gipsabdruck gefertigt wurde, mit jenen übereinstimmen, unter welchen der Schaft später getragen wird. In der Praxis ist es üblich, den Gipsabdruck des Stumpfs am stehenden Patienten zu nehmen. Der i. d. R. einbeinige Patient steht dabei meist, wobei er regelmäßig gestützt werden muss. Der Orthopädietechniker kann die noch feuchte Gipsbinde, welche um den Stumpf gelegt ist, mit der Hand am stehenden Patienten ausformen. Er hat so die Möglichkeit, sich tastend ein Bild über anatomische Gegebenheiten und die Lage relevanter Knochenstrukturen, bezogen auf den Stumpf, zu machen und die feuchte Gipsbinde bzw. den langsam trocknenden Gipsabdruck entsprechend zu modellieren. Eine Vorrichtung nach der Präambel von Anspruch 1 ist aus GB 2 232 598 A bekannt.

Die vorliegende Erfindung ist in den unabhängigen Ansprüchen 1, 8, 10, 11 und 12 definiert. Somit sind die in weiterer Folge beschriebenen Alternativen der Beispiele, in denen die fluiddichte Membran und/oder der Verbinder als optional beschrieben werden, nicht Teil der vorliegenden Erfindung, und finden nur zur besseren Veranschaulichung Erwähnung.

Die vorliegende Erfindung betrifft das technische Gebiet des Herstellens eines Gipsabdrucks als Pause, Vorlage oder Modell für den späteren Prothesenschaft einer Prothese, vorzugsweise für die unteren Extremitäten, also für eine Beinprothese, insbesondere einer Unterschenkelprothese.

Eine Aufgabe der vorliegenden Erfindung kann es sein, eine Vorrichtung, ein System und Verfahren zur Verwendung beim Fertigen eines Gipsabdrucks für einen Prothesenschaft, oder zumindest für einen Außenschaft hiervon, insbesondere für die untere Extremität, vorzuschlagen.

Die erfindungsgemäße Aufgabe wird durch eine Vorrichtung mit den Merkmalen des Anspruchs 1 gelöst. Sie kann weiterhin gelöst werden durch ein Set mit den Merkmalen des Anspruchs 7, durch Verfahren mit den Merkmalen der Ansprüche 8 oder 10, ferner durch eine Berechnungsvorrichtung mit den Merkmalen des Anspruchs 11, sowie eine Einheit mit den Merkmalen des Anspruchs 12.

Sie kann auch gelöst werden durch das hierin beschriebene digitale Speichermedium, das Computerprogramm-Produkt und das Computerprogramm.

Erfindungsgemäß wird somit eine medizinische Vorrichtung (kurz: Vorrichtung) vorgeschlagen, welche bei der Anfertigung eines Gipsabdrucks eines Körpergliedstumpfs, insbesondere eines Unterschenkelstumpfs, verwendet werden kann. Diese kann beispielsweise als Grundlage beim Fertigen eines Unterschenkelprothesenschaftes, am nur vorzugsweise stehenden Patienten, verwendet werden.

Dabei weist die Vorrichtung einen Fluidbehälter oder Druckbehälter mit genau oder wenigstens einer Druckkammer oder genau oder wenigstens einer Fluidkammer auf. Diese kann ein optional unter Druck stehendes Fluid aufnehmen oder speichern. Der Druck liegt dabei oberhalb des Atmosphärendrucks. Bei dem Fluid handelt es sich um ein Gas oder eine Flüssigkeit, vorzugsweise Luft bzw. Wasser, da letztere jeweils billig und leicht verfügbar sind.

Der Druckbehälter weist eine Wandung auf, welche aus wenigstens oder genau einem ersten Material gefertigt ist oder wenigstens ein erstes Material aufweist.

Die Wandung des Druckbehälters begrenzt dessen Inneres. Unter dem Inneren des Druckbehälters wird erfindungsgemäß jener Raum oder jenes Volumen verstanden, welcher/s durch die Geometrie des Druckbehälters vorgegeben oder durch eine äußere Wandung des Druckbehälters umfasst oder umschrieben ist. Ist der Druckbehälter beispielsweise zylindrisch, so ist das Innere des Druckbehälters der von der zylindrischen Mantelfläche und den beiden Stirnflächen oder Stirnebenen begrenzte Raum. Ist der Druckbehälter in einem anderen Beispiel rechteckig, so wird der Raum des Inneren durch das Ergebnis der Multiplikation von Höhe, Breite und Tiefe des Rechtecks definiert. Es spielt bei der Bestimmung des Inneren keine Rolle, ob der Raum, der dem Inneren entspricht, fluiddicht begrenzt ist oder nicht. Das Innere beschreibt keinen fluiddicht abgeschlossenen Raum, sondern ein von der Wandung umschriebenes Volumen. Jener Raum, welcher nicht zum Inneren des Druckbehälters zählt, wird hierin als dessen Äußeres bezeichnet.

Der Druckbehälter weist eine Einführöffnung auf, durch welche hindurch der Körpergliedstumpf (gemeint sein kann hiermit in diesem Zusammenhang stets auch das distale Stumpfende anstelle des gesamten Stumpfs) in das Innere des Druckbehälters eingeführt werden kann. Die Einführöffnung kann beispielsweise eine offene Stirnfläche oder Stirnebene, eine Durchgangsöffnung in der Wandung oder eine Öffnung sein, welche die Wandung durchbricht oder unterbricht. Im Bereich der Einführöffnung ist das Innere somit nicht durch einen Abschnitt der Wandung von dem Äußeren des Druckbehälters getrennt. Die Einführöffnung kann in einer Eintrittsöffnung oder in einer Einführebene liegen, durch welche der Körpergliedstumpf in das Innere des Druckbehälters eingebracht wird.

Ferner weist der Druckbehälter wenigstens eine oder genau eine fluiddichte Membran auf. Alternativ weist der Druckbehälter keine solche und im Folgenden weiter beschriebene Membran, sondern nur entsprechend geeignete und/oder vorgesehene Aufnahmeeinrichtungen (wie den im Folgenden als optional beschriebenen Verbinder) zum Aufnehmen der Membran am Druckbehälter auf.

Die Membran ist aus einem zweiten Material gefertigt oder weist ein solches auf. Das erste und das zweite Material unterscheiden sich voneinander.

Die Wandung ist einteilig oder optional mehrteilig ausgestaltet.

Eine mehrteilige Wandung kann mehrere verschiebbare Abschnitte aufweisen, um beispielsweise für Transportzwecke ein kleines Packvolumen der Vorrichtung zu erzielen. Die erfindungsgemäße medizinische Vorrichtung mit der mehrteiligen Wandung kann als mobile medizinische Vorrichtung bezeichnet werden.

Der Adapter ist zur Verwendung bei der Anfertigung eines Gipsabdrucks oder eines Datenmodells eines Körpergliedstumpfs, insbesondere eines Unterschenkelstumpfs, vorgesehen und/oder vorbereitet. Der Adapter weist wenigstens eine Entlastungseinrichtung und/oder eine Einrichtung zum Übertragen von Zugkräften, welche mit einem Haftstrumpf in Kraft- und/oder Formschluss verbunden ist, und den Haftstrumpf auf. Die Entlastungseinrichtung kann vorteilhaft empfindliche Weichteile im Bereich des distalen Endes des Körpergliedstumpfs schützen.

In gewissen beispielhaften Ausführungsformen weist der Adapter eine Zugeinrichtung, insbesondere einen Gurt, und/oder eine Befestigungseinrichtung, insbesondere einen Befestigungsring, zum Verbinden des Adapters mit der Zugeinrichtung auf.

Das erfindungsgemäße Set weist wenigstens eine erfindungsgemäße medizinische Vorrichtung auf. Es weist ferner wenigstens zwei Membrane, welche sich in wenigstens einer Eigenschaft voneinander unterscheiden, und/oder wenigstens ein Gewicht zum vorübergehenden Erhöhen des Körpergewichts des Patienten bzw. des von diesem auf die Membran oder auf eine Waage aufgebrachten Gewichts auf. Der Druckbehälter der erfindungsgemäßen Vorrichtung kann somit durch einfachen Austausch der Membran gegen eine andere Membran an unterschiedliche Körperteile, Patienten, Patientengewichte, Patientengrößen usw. angepasst werden. Das Gewicht, etwa in Gestalt eines mit einem Gewicht oder mehreren Teilgewichten beschwerten Gürtels, Trägers, Rucksacks oder dergleichen kann dem Patienten bei der Anfertigung eines Gipsabdrucks mittels der erfindungsgemäßen Vorrichtung angehängt werden. Es macht ihn wie das Gewicht, das einem Taucher angehängt wird, auf einer Waage "schwerer" mit der Folge, dass der Körpergliedstumpf aufgrund des erhöhten, auf die Membran aufgebrachten Gewichts tiefer in die Vorrichtung eindringt. Es hat sich gezeigt, dass hierdurch noch exaktere Passformen für den gefertigten Gipsabdruck erzielbar sind. Das dem Patienten angehängte Gewicht kann individuell festgelegt werden und beispielsweise zwischen 5kg und 25kg oder zwischen 10kg und 20kg betragen. Es kann eine Anzahl von Teilgewichten von je 3kg, 4kg, 5kg oder dergleichen vorgesehen sein.

Der Haftstrumpf ist zum Überziehen über einen Körpergliedstumpf (KS) bei der Anfertigung eines Gipsabdrucks oder eines Datenmodells des Körpergliedstumpfs (KS) vorgesehen und/oder vorbereitet. Der mit dem Körpergliedstumpf verbundene Haftstrumpf ist kraftschlüssig, insbesondere reibschlüssig mit einer Oberfläche, insbesondere mit der Oberfläche einer Membran, an oder in einem Druckbehälter zum Anfertigen des Gipsabdrucks oder des Datenmodells verbunden.

Das erfindungsgemäße Set (hierin synonym auch als System bezeichnet) kann wenigstens eine erfindungsgemäße medizinische Vorrichtung und wenigstens eine Auflagefläche umfassen. Die Vorrichtung ist, relativ zur Auflagefläche und wenn sie auf der Auflagefläche aufliegt, vorzugsweise während der Anfertigung eines Gipsabdrucks oder eines Datenmodells eines Körpergliedstumpfs, kippbar, verdrehbar und/oder verschiebbar.

Ein erfindungsgemäßes Verfahren dient dem Anpassen eines Gipsabdrucks an einen Körpergliedstumpf eines Patienten oder dem Vermessen des Körpergliedstumpfs, beispielsweise seines Volumens, seiner Geometrie, seiner Oberfläche und/oder einer Kombination hiervon. Dabei wird eine erfindungsgemäße medizinische Vorrichtung bereitgestellt.

Ferner wird oder ist, jeweils optional, die Druckkammer mit einer Flüssigkeit befüllt, eine ballonartig geschlossene Membran im Inneren des Druckbehälters bereitgestellt oder der Flüssigkeitsspiegel innerhalb der Druckkammer derart eingestellt oder verändert, dass die Membran zumindest in Abschnitten hiervon um den gesamten Umfang dieser Abschnitte herum von Flüssigkeit bedeckt ist oder sich über die Einführöffnung hinaus in das Äußere des Druckbehälters wölbt.

Ein zweites erfindungsgemäßes Verfahren dient dem Anpassen eines Gipsabdrucks an einen Körpergliedstumpf eines Patienten. Dabei wird eine um den Körpergliedstumpf gewickelte feuchte Gipsbinde mit einem pro Flächeneinheit konstanten Druck beaufschlagt bis die Gipsbinde zumindest teilweise getrocknet ist.

Ein drittes Verfahren dient dem Fixieren des Körpergliedstumpfs im Druckbehälter. Dabei wird zunächst optional der Körpergliedstumpf mit einer feuchten Gipsbinde umwickelt, wobei die Umwicklung, die als Ummantelung bezeichnet werden kann, mit dem Adapter verbunden ist. Anschließend wird der optional mit einer feuchten Gipsbinde umwickelte Körpergliedstumpf mit dem Adapter in die Eintrittsöffnung oder die Membran eingeführt. Nachfolgend wird der eingeführte Körpergliedstumpf mittels einer mit dem Adapter verbundenen Halte- oder Verstelleinrichtung geführt oder gehalten, insbesondere mittels einer Zugvorrichtung. Das Führen erfolgt insbesondere von außerhalb des Druckbehälters und/oder der Druckkammer. Schließlich wird der mit dem Adapter verbundene Körpergliedstumpf in dem Druckbehälter mittels der Verstelleinrichtung derart fixiert, dass ein Herausbewegen des Körpergliedstumpfs aus der Eintrittsöffnung oder ein Bewegen in Richtung nach außen verhindert oder zumindest weitgehend unterbunden wird. Im Anschluss an diese Fixierung wird die Druckkammer mit Fluid, vorzugsweise Wasser, befüllt. Dabei kann vorzugsweise ein vorbestimmter Druck gezielt eingestellt werden. Der vorbestimmte Druck kann wie hierin beschrieben ermittelt werden oder ermittelt worden sein.

Ein weiteres Verfahren dient dem Fixieren oder Halten des Körpergliedstumpfs im Druckbehälter. Dabei wird in einem ersten Verfahrensschritt ein erfindungsgemäßer Haftstrumpf über den Körpergliedstumpf gezogen oder übergestülpt. Anschließend wird der mit dem Haftstrumpf überzogene Körpergliedstumpf in den Druckbehälter eingeführt. In dem anschließenden Verfahrensschritt erfolgt ein kraftschlüssiges, insbesondere ein reibschlüssiges, Verbinden des Haftstrumpfes mit einer Oberfläche, insbesondere mit der Oberfläche der Membran, in dem Druckbehälter. Ein reibschlüssiges Verbinden kann ein Anhaften in Form einer belastungsfähigen und reversiblen Verbindung des Haftstrumpfes mit einer Oberfläche in dem Druckbehälter sein.

Ein digitales, insbesondere nichtflüchtiges, Speichermedium, insbesondere in Form eines maschinenlesbaren Trägers, insbesondere in Form einer Diskette, CD, EPROM oder DVD, mit elektrisch auslesbaren Steuersignalen kann derart mit einem programmierbaren Computersystem zusammenwirken, dass die maschinellen Schritte wenigstens eines oder aller erfindungsgemäßen Verfahren(s) veranlasst werden.

Dabei können alle, einige oder manche der maschinell durchgeführten Schritte des oder der Verfahren(s) veranlasst werden, insbesondere im Zusammenwirken mit einer Rechenanlage und einer erfindungsgemäßen Vorrichtung.

Ein Computerprogramm-Produkt weist einen volatilen, flüchtigen oder auf einem maschinenlesbaren Träger gespeicherten Programmcode zur Veranlassung der maschinellen Schritte wenigstens eines oder aller erfindungsgemäßen Verfahren(s), wenn das Computerprogramm-Produkt auf einem Rechner abläuft, auf. Unter einem Computerprogramm-Produkt kann erfindungsgemäß beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte oder dergleichen sein.

Ein Computerprogramm weist einen Programmcode auf zur Veranlassung der maschinellen Schritte eines oder aller erfindungsgemäßen Verfahren(s), wenn das Computerprogramm auf einem Computer abläuft. Unter einem Computerprogramm kann beispielsweise ein physikalisches, vertriebsfähiges Software-Produkt verstanden werden, welches ein Programm aufweist.

Auch für das Computerprogramm-Produkt und das Computerprogramm gilt, dass alle, einige oder manche der maschinell durchgeführten Schritte des oder der Verfahren(s) veranlasst werden, insbesondere im Zusammenwirken mit einer Rechenanlage und einer erfindungsgemäßen Vorrichtung.

Die vorliegende Erfindung betrifft ferner eine medizinische Vorrichtung zur Verwendung bei der Anfertigung eines Gipsabdrucks oder eines Datenmodells eines Körpergliedstumpfs, insbesondere eines Unterschenkelstumpfs, wobei die Vorrichtung einen Fluidbehälter oder einen Druckbehälter zur Aufnahme eines Fluids mit einer Wandung, oder aus einem ersten Material, aufweist, wobei das Fluid Wasser ist, wobei eine erste Stirnseite der Wandung, insbesondere fluiddicht, geschlossen ist, wobei eine zweite Stirnseite der Wandung mit einer Membran aus oder mit einem zweiten Material fluiddicht verschlossen ist, wobei der Fluidbehälter oder der Druckbehälter, insbesondere in seiner Wandung, wenigstens einen Auslass für das Fluid hat, optional mit einem Ventil oder einem Sperrhahn zum Öffnen und Schließen des Auslasses, wobei die Membran sackartig (also mit einem toten Ende, im Gegensatz zu einem oben und unten offenen Schlauch) ist, wobei wenigstens ein, vorzugsweise zentraler oder mittlerer, Abschnitt der Membran mittels wenigstens einem Verbinder an einem Abschnitt der Wandung, vorzugsweise lösbar, befestigt ist, wobei die Membran vorzugsweise aus einem Material gefertigt ist oder ein solches aufweist, welches in einer ersten Richtung des Materials vorzugsweise keine Elastizität oder vorzugsweise keine Dehnbarkeit aufweist.

Die vorliegende Erfindung betrifft gleichwertig eine medizinische Vorrichtung zur Verwendung bei der Anfertigung eines Gipsabdrucks oder eines Datenmodells eines Körpergliedstumpfs, insbesondere eines Unterschenkelstumpfs, wobei die Vorrichtung einen, Fluidbehälter (kann ein Druckbehälter sein) für eine Flüssigkeit, etwa Wasser, aufweist. Der Fluidbehälter weist eine im Gebrauch der Vorrichtung obere, oder erste, Stirnseite und eine im Gebrauch untere, oder zweite, Stirnseite auf. Im Bereich der zweiten Stirnseite kann der Fluidbehälter fluiddicht verschlossen sein, beispielsweise mittels einer Bodenfläche. Die Vorrichtung weist eine Folie oder Membran zum Aufnehmen eines Abschnitts eines Körpergliedstumpfs auf. Die Membran ist fluiddicht mit dem Behälter verbunden und/oder verschließt diesen wenigstens oben oder an wenigstens der ersten Stirnseite, zumindest abschnittsweise, fluiddicht. Der Fluidbehälter kann optional eine Druckkammer aufweisen. Der Fluidbehälter weist optional wenigstens einen Auslass für das Fluid auf, welcher optional mit einem Ventil oder einem Sperrhahn zum Öffnen und Schließen des Auslasses versehen ist. Die Membran ist vorzugsweise sackartig (also mit einem toten Ende wie ein Sack, im Gegensatz zu einem oben und unten offenen Schlauch). Wenigstens ein, vorzugsweise zentraler oder mittlerer, vorzugsweise unterer oder distaler Abschnitt der Membran ist mittels wenigstens eines Verbinders an oder mit einem Abschnitt einer Wandung des Fluidbehälters, beispielsweise einer Bodenfläche hiervon, vorzugsweise lösbar, direkt oder indirekt, befestigt. Die Membran ist vorzugsweise aus einem Material gefertigt oder weist ein solches auf, welches wenigstens in einer ersten Richtung des Materials, vorzugsweise in einer Längseinrichtung des

Behälters oder in einer Einführrichtung des Körpergliedstumpfs, keine (oder eine nur geringe) Elastizität oder vorzugsweise keine (oder eine nur geringe) Dehnbarkeit aufweist. Anstatt die vorstehend genannte Membran und/oder den Verbinder aufzuweisen, kann die Vorrichtung ausgestaltet oder konfiguriert sein, um mit einer Membran und/oder einem Verbinder verbunden zu werden. Hierzu geeignete Verbindungseinrichtungen, etwa an einer Bodenfläche, können vorgesehen sein.

Die Erfindung betrifft ferner ein Verfahren zum Ermitteln eines Zusatzgewichts beim Anpassen eines Gipsabdrucks an einen Körpergliedstumpf oder beim Vermessen des Körpergliedstumpfs eines Patienten. Das Verfahren umfasst wenigstens ein Bereitstellen von patientenbezogenen Daten; ein Eingeben der patientenbezogenen Daten in eine Ermittlungsvorrichtung; ein Ermitteln eines Zusatzgewichts (kurz: Gewicht), mit welchem der Patient, dessen Körpergliedstumpf in die medizinische Vorrichtung eingeführt ist, beschwert werden muss, damit ein Druck, welcher auf der Membran, in der Druckkammer, auf einem Abschnitt der Oberfläche des von der Membran im Gebrauch der Vorrichtung bedeckten Körpergliedstumpfs und/oder zwischen Membran und Körpergliedstumpf herrscht, in einem vorbestimmten Zielwertebereich liegt.

Die vorliegende Erfindung betrifft ferner eine Berechnungsvorrichtung. Sie ist programmiert, konfiguriert und/oder eingerichtet, um das erfindungsgemäße Verfahren zum Ermitteln eines Zusatzgewichts beim Anpassen eines Gipsabdrucks durchzuführen.

Erfindungsgemäße Ausführungsformen jeder der vorgenannten Gegenstände können eines oder mehrere der im Folgenden genannten Merkmale in beliebiger Kombination aufweisen, sofern eine konkrete Kombination für den Fachmann nicht als offenkundig technisch unmöglich erkennbar ist. Auch die Gegenstände der Unteransprüche geben jeweils erfindungsgemäße Ausführungsformen an.

Bei allen obenstehenden und/oder folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wann immer hierin räumliche Bezüge wie "oben", "unten", "oberen" oder "unteren" genannt werden, so versteht der Fachmann diese im Zweifel als eine räumliche Angabe mit Bezug auf die Ausrichtung in den hier beigefügten Figuren und/oder die Anordnung der erfindungsgemäßen Vorrichtung(en) in ihrem bestimmungsgemäßen Gebrauch.

In gewissen erfindungsgemäßen, beispielhaften Ausführungsformen ist die Druckkammer ein abgeschlossener und/oder abschließbarer Raum, in welchem das Fluid einem Druck oberhalb des Atmosphärendrucks (kurz: Atmosphäre) ausgesetzt werden kann, ohne aus diesem Raum entweichen zu können.

In manchen erfindungsgemäßen, beispielhaften Ausführungsformen ist die Membran angeordnet, um die Druckkammer zu bilden oder, alternativ, um sie zu begrenzen, beispielsweise indem sie Teil, insbesondere elastischer oder in nur einer Richtung elastischer Teil, der Wand der Druckkammer ist.

In gewissen erfindungsgemäßen, beispielhaften Ausführungsformen dient die Membran dazu, um, für sich allein (z. B. in Form eines Ballons) oder alternativ gemeinsam mit Abschnitten der Wandung, eine zumindest teilweise oder vollständig im Inneren des Druckbehälters liegende, fluiddicht abgeschlossene Fluidkammer des Druckbehälters zu bilden. Da sie Fluid unter einem Druck aufnehmen und/oder halten kann, welcher oberhalb Atmosphärendruck liegt, wird diese Fluidkammer hierin als Druckkammer bezeichnet.

Die Begriffe "Fluidkammer" und "Druckkammer" sind in gewissen erfindungsgemäßen, beispielhaften Ausführungsformen und/oder in solchen Ausführungsformen, in denen für den Fachmann aus technischer Sicht nichts dagegen spricht, austauschbar. Was hierin zur "Druckkammer" gesagt ist, kann auch für eine "Fluidkammer" zutreffen.

In manchen erfindungsgemäßen, beispielhaften Ausführungsformen liegt die Druckkammer dann innerhalb des Inneren des Druckbehälters, wenn der in der Druckkammer herrschende Druck einen gewissen Druck nicht überschreitet. Die Druckkammer erstreckt sich hiervon abweichend in gewissen erfindungsgemäßen, beispielhaften Ausführungsformen auch auf das Äußere des Druckbehälters, wenn der in der Druckkammer herrschende Druck die Membran derart beaufschlagt, dass sich letztere beispielsweise durch die Einführöffnung hindurch nach außen, also in das Äußere des Druckbehälters, vorwölbt. Die Druckkammer kann somit ein variables Volumen aufweisen, welches von dem in der Druckkammer herrschenden Druck abhängt. Letzteres trifft nicht auch auf das konstante Innere des Druckbehälters zu.

In manchen erfindungsgemäßen, beispielhaften Ausführungsformen kann in der Druckkammer ungeachtet der die Wandung durchbrechenden Einführöffnung ein Fluid unter Druck gehalten werden, welcher oberhalb der Atmosphäre liegt.

In gewissen erfindungsgemäßen, beispielhaften Ausführungsformen dient die Druckkammer dem Aufnehmen oder dem Abstützen des in das Innere des Druckbehälters eingeführten distalen Endes des Körpergliedstumpfs des Patienten. Die Membran schmiegt sich aufgrund des in der Druckkammer enthaltenen Fluids lateral oder umfangsseitig an das distale Ende des Körpergliedstumpfs oder an den gesamten Körpergliedstumpf an. Auf diese Weise kann es möglich sein, den Körpergliedstumpf im Bereich der gesamten Gipsbinde durch die Membran hindurch mit - bezogen auf eine Flächeneinheit - vorzugsweise unverändertem oder gleichem Druck zu beaufschlagen. Letzteres kann einen vorteilhaften Beitrag der vorliegenden Erfindung darstellen, da bereits die gleichmäßige Druckbeaufschlagung zu einer gleichmäßigen Modellierung der feuchten Gipsbinde führen kann. Das Anliegen der Membran unter dem in der Druckkammer gespeicherten Fluid kann eine Modellierung von Hand vorteilhaft entbehrlich machen oder den Aufwand hierfür deutlich reduzieren. Die Gipsbinde kann eine handelsübliche sein.

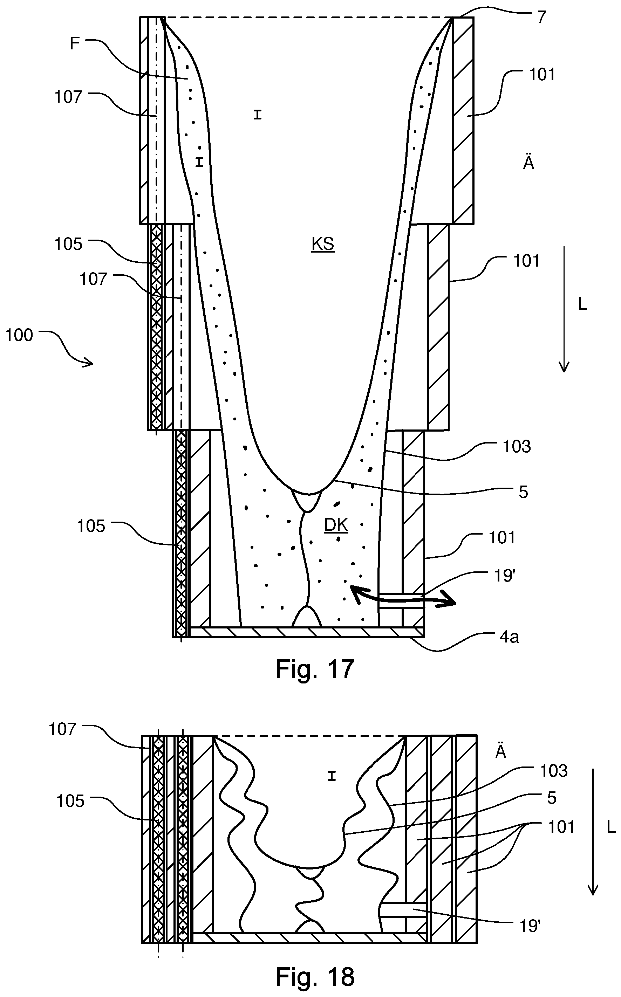

In manchen erfindungsgemäßen, beispielhaften Ausführungsformen ist die Druckkammer ein fluiddicht geschlossener Raum, welcher vollständig, oder unter anderem, durch die Wandung des Druckbehälters und die Membran gebildet oder begrenzt wird. In einigen erfindungsgemäßen, beispielhaften Ausführungsformen kann der Begriff "Druckkammer" durch die vorstehende Definition ersetzt werden. In manchen erfindungsgemäßen, beispielhaften Ausführungsformen liegt Wandung dabei ein unten beschriebener Schlauchabschnitt, siehe auch Fig. 17, auf oder bedeckt diese zu Inneren hin vollständig oder zumindest teilweise. Dieser kann zur Wandung gezählt werden.

In manchen erfindungsgemäßen, beispielhaften Ausführungsformen ist der Druckbehälter jener Behälter oder Raum, in welchem die Druckammer angeordnet ist.

In einigen erfindungsgemäßen, beispielhaften Ausführungsformen ist der Druckbehälter ein Wasserbehälter.

In manchen erfindungsgemäßen, beispielhaften Ausführungsformen kann der Begriff "Druckbehälter" durch den Begriff "Fluidbehälter" oder "Wasserbehälter" ersetzt werden.

In gewissen erfindungsgemäßen, beispielhaften Ausführungsformen hat der Druckbehälter eine zylindrische Form.

In manchen erfindungsgemäßen, beispielhaften Ausführungsformen ist die Druckkammer derart ausgestaltet und/oder angeordnet, dass der in ihr herrschende Druck, unter anderem oder ausschließlich, von der Einführtiefe des Körpergliedstumpfs in das Innere des Druckbehälters abhängt, jedenfalls im bestimmungsgemäßen Gebrauch der Vorrichtung und bei geschlossenen Ein- und Auslässen, soweit vorhanden.

In gewissen erfindungsgemäßen, beispielhaften Ausführungsformen ist die Membran im Bereich der Einführöffnung angeordnet und optional dort direkt oder indirekt mit der Wandung lösbar oder unlösbar verbunden, vorzugsweise fluiddicht. Sie verschließt die Einführöffnung vorzugsweise ähnlich eines Deckels, soweit der in der Druckkammer liegende Druck nicht wesentlich vom Atmosphärendruck abweicht.

In einigen erfindungsgemäßen, beispielhaften Ausführungsformen ist die Membran angeordnet, um einen Fluid- oder Stoffaustausch im Inneren des Druckbehälters in dessen axialer Richtung zu unterbinden.

In gewissen erfindungsgemäßen, beispielhaften Ausführungsformen ist die Membran in axialer und/oder radialer Richtung stets einlagig angeordnet.

In einigen erfindungsgemäßen, beispielhaften Ausführungsformen ist die Membran an einer ersten Stirnseite des Druckbehälters mit diesem direkt oder indirekt verbunden, nicht aber auch an einer zweiten, der ersten gegenüberliegenden Stirnseite.

In gewissen erfindungsgemäßen, beispielhaften Ausführungsformen ist die Membran flach oder ballonartig (d. h. an einem Ende offen), nicht aber schlauchartig (d. h. an beiden Enden offen).

In einigen erfindungsgemäßen, beispielhaften Ausführungsformen ist die Membran, wenigstens teilweise, vorzugsweise an ihrem Rand, als ein Dichtelement ausgestaltet, etwa als ein Dichtring. Sie wird in diesen Ausführungsformen, beispielsweise im Bereich der Einführöffnung, um den Körpergliedstumpf herum angeordnet, Sie verhindert optional, dass das Fluid, welches in der Druckkammer herrscht, an Druck entlang des Körpergliedstumpfs verliert. Sie mag vorteilhafter Weise einen Austritt von Fluid in das Äußere der Druckkammer und damit einen Druckabfall im Inneren der Druckkammer verhindern.

In manchen erfindungsgemäßen, beispielhaften Ausführungsformen ist die Membran, zumindest beim Gebrauch der Vorrichtung (also bei in das Innere des Druckbehälters eingeführtem Körpergliedstumpf des Patienten) und zumindest in Abschnitten hiervon, im Inneren des Druckbehälters angeordnet. Vorzugsweise liegt sie nur und/oder stets im Inneren des Druckbehälters vor. Alternativ oder ergänzend ist sie direkt oder indirekt in fluiddichter Verbindung mit Abschnitten der Wandung des Druckbehälters verbunden.

In einigen erfindungsgemäßen, beispielhaften Ausführungsformen liegt die Membran auch beim Gebrauch der Vorrichtung (also bei in das Innere des Druckbehälters eingeführtem Körpergliedstumpf des Patienten) ausschließlich im Inneren des Druckbehälters vor.

In manchen erfindungsgemäßen, beispielhaften Ausführungsformen steht die Membran nicht aus dem Inneren des Druckbehälters hervor, insbesondere nicht im Bereich einer zweiten Stirnseite oder im Bereich der Bodenfläche.

In manchen erfindungsgemäßen, beispielhaften Ausführungsformen ist die Membran dauerhaft mit der Vorrichtung verbunden. Dauerhaft bedeutet in gewissen erfindungsgemäßen Ausführungsformen, dass die Membran nicht ohne Einsatz von Werkzeug oder nur zerstörend vom Druckbehälter gelöst werden kann; sie kann beispielsweise mittels Klemmrings oder Klemmringe und einer oder mehrerer Schrauben mit der Wandung dauerhaft und doch lösbar verbunden sein. Eine Lösbarkeit mittels Werkzeugs kann vorteilhaft vorgesehen sein, um ein Austauschen der Membran, etwa aufgrund von Verschleiß nach einer Vielzahl von Verwendungen, zu ermöglichen. Sie soll in diesen Ausführungsformen allerdings nicht durch einfaches Überstülpen, Herunterziehen oder dergleichen vom Druckbehälter lösbar sein. Zugleich kann die dauerhafte Befestigung vorteilhaft sicherstellen, dass die bei der Benutzung der Vorrichtung in der Druckkammer über das Fluid auf die Membran übertragenen Kräfte die Membran nicht vom Druckbehälter oder von dessen Wandung lösen können.

In einigen erfindungsgemäßen, beispielhaften Ausführungsformen ist die Membran an wenigstens einer Oberfläche hiervon, zumindest abschnittsweise, mittels eines reibungsvermindernden Materials beschichtet, oder besteht hieraus oder trägt ein solches (z. B. aufgebracht durch Einschmieren, Einsprühen, oder dergleichen, der Membran). Dies erlaubt es dem Patienten den Körpergliedstumpf ausreichend tief durch die Einführöffnung in das Innere des Druckbehälters einzuführen. Auf dieses Weise ist vorteilhaft sichergestellt, dass sich die Membran nicht oder nicht übermäßig stark seitlich entlang des Körperglieds nach proximal verlagert. Dies stellt wiederum vorteilhaft sicher, dass ein Ausstülpen der Membran nach proximal nicht auftritt.

In einigen erfindungsgemäßen, beispielhaften Ausführungsformen weist die Vorrichtung keine andere axiale Aufnahme für das freie Stumpfende als die Membran und/oder keine *"axial reference compliant means"* auf. Das Stumpfende kommt vorzugsweise nur mit der Membran in Kontakt. In anderen erfindungsgemäßen Ausführungsformen kann dies anders sein.

In manchen erfindungsgemäßen, beispielhaften Ausführungsformen weist die erfindungsgemäße Vorrichtung keine, insbesondere kreisförmige, scheibenförmige Abdeckung der Einführöffnung auf, welche beispielsweise aus Gummi gefertigt ist und/oder welche optional einstückig mit einem integralen zentralen Loch ist.

In einigen erfindungsgemäßen, beispielhaften Ausführungsformen weist die erfindungsgemäße Vorrichtung, insbesondere in ihrem Gebrauch, keinen Sand, kein Gipsmaterial, kein aushärtendes Material auf, insbesondere nicht in der Druckkammer oder zwischen Wandung und Membran.

In gewissen beispielhaften Ausführungsformen ist das Fluid nicht Sand, nicht feste Partikel und nicht Kugeln, insbesondere keine Polystyrenkugeln, oder weist derartiges auf.

In gewissen beispielhaften Ausführungsformen ist die Membran nicht aus Polyethylen oder weist kein Polyethylen auf.

In manchen beispielhaften Ausführungsformen weist die erfindungsgemäße Vorrichtung keine Einrichtung wie einen elastischen Ring oder einen Gummiring und insbesondere keinen Gummiring auf, welcher zur Befestigung der Membran an einer äußeren Wandung des Druckbehälters vorgesehen ist. In anderen erfindungsgemäßen Ausführungsformen kann dies anders sein.

In einigen beispielhaften Ausführungsformen weist die erfindungsgemäße Vorrichtung keine Vakuumquelle auf (insbesondere keine Vakuumquelle, welche elektrisch oder hydraulisch betrieben wird) oder ist mit einer solchen nicht in Fluidverbindung verbunden.

In bestimmten beispielhaften Ausführungsformen weist die erfindungsgemäße Vorrichtung keine Druckquelle und/oder keine, insbesondere aufblasbaren, Aufweitevorrichtungen oder anderweitige *"expander means"* auf oder ist mit einer solchen nicht verbunden.

In manchen beispielhaften Ausführungsformen weist die erfindungsgemäße Vorrichtung keinen Unterdruckanschluss auf.

In gewissen beispielhaften Ausführungsformen weist die erfindungsgemäße Vorrichtung keine Luftkammer auf.

In manchen beispielhaften Ausführungsformen weist die erfindungsgemäße Vorrichtung keine Förderschrauben auf.

In einigen beispielhaften Ausführungsformen ist die Vorrichtung ausgestaltet, um mit ihr einen negativen Abdruck des Körpergliedstumpfs anzufertigen. Der negative Abdruck hat eine Wandstärke von vorzugsweise 2 bis 8mm, besonders bevorzugt von 3 bis 6 mm.

In manchen beispielhaften Ausführungsformen ist die Vorrichtung nicht ausgestaltet, um mit ihr einen positiven Abdruck des Körpergliedstumpfs anzufertigen.

In gewissen beispielhaften Ausführungsformen weist eine Schwenkvorrichtung auf, mittels welcher die erfindungsgemäße Vorrichtung oder ihr Druckbehälter derart geschwenkt werden kann, dass sich der Winkel zwischen der Längsachse des Druckbehälters der auf dem Boden aufgestellten Vorrichtung gegenüber einer Horizontalen ändert. Der Schwenkwinkel kann einstellbar sein, die Vorrichtung in der erzielten Schwenkstellung feststellbar. Dies erlaubt es vorteilhaft, auch für Patienten, welche aufgrund von Kontrakturen nicht gerade in die Einführöffnung eindringen können, einen Gipsabdruck zu fertigen.

In gewissen beispielhaften Ausführungsformen weist der Druckbehälter die Einführöffnung sowie optional einen oder mehrere Ein- und/oder Auslässe auf, welche jedoch allesamt mit einer oder jeweils einer Verschlusseinrichtung zu deren fluiddichtem Verschließen versehen sind. Ansonsten ist die Wandung des Druckbehälters oder der Druckkammer in diesen Ausführungsformen fluiddicht.

In einigen beispielhaften Ausführungsformen sind die mit einer Verschließeinrichtung versehenen Ein- und/oder Auslässe Teil der Wandung.

In gewissen beispielhaften Ausführungsformen ist die Druckkammer ausschließlich durch die Membran und Teile oder Abschnitte der Wandung, oder ausschließlich durch Membran und Teile oder Abschnitte der Wandung und fluiddichte Verbindungen zwischen Membran und Wandung ausgestaltet.

In einigen beispielhaften Ausführungsformen zählt eine Stirnseite des Druckbehälters zur Wandung, insbesondere die hierin als zweite Stirnseite bezeichnete Stirnseite.

In manchen beispielhaften Ausführungsformen ist der Druckbehälter an seiner zweiten Stirnseite fluiddicht verschlossen oder verschließbar.

Der Druckbehälter kann eine erste und eine zweite Stirnseite aufweisen. Die Einführöffnung kann eine stirnseitige Öffnung sein. Sie liegt vorzugsweise in der ersten Stirnseite oder im Bereich der ersten Stirnseite.

Die Membran kann lösbar oder nicht lösbar mit der Wandung oder einem anderen Abschnitt des Druckbehälters verbunden sein.

Die Druckkammer kann ein Raum des Druckbehälters sein, welcher durch die Wandung und die Membran geschlossen ist. Die Druckkammer liegt teilweise oder vollständig im Inneren des Druckbehälters. Ein in der Druckkammer gespeichertes Fluid kann durch die Kombination allein zwischen Wandung (soweit darin ggf. vorgesehene Öffnungen mittels der erfindungsgemäß vorgesehenen Verschlüsse, Ventile, Sperrhähne, usw. verschlossen sind) und Membran gefangen sein.

In einigen beispielhaften Ausführungsformen weist die mehrteilige Wandung der medizinischen Vorrichtung eine Mehrzahl von Wandungsteilabschnitten auf oder besteht aus einer Mehrzahl von Wandungsteilabschnitten, welche relativ zueinander bewegbar angeordnet sind.

In gewissen beispielhaften Ausführungsformen sind Wandungsteilabschnitte lösbar miteinander verbunden.

In manchen beispielhaften Ausführungsformen sind die Wandungsteilabschnitte als zueinander konzentrische Zylinderabschnitte ausgestaltet oder weisen solche auf.

In bestimmten beispielhaften Ausführungsformen sind die Wandungsteilabschnitte als polyederförmige Abschnitte ausgestaltet oder weisen solche auf. Im Gegensatz zu runden Zylinderabschnitten weisen polyederförmige Abschnitte Ebenen auf, die im Querschnitt, senkrecht zur Längsachse, ein Vieleck sind, umschreiben oder aufweisen. Beispielsweise kann der Querschnitt ein Quadrat oder ein Rechteck mit vier Ecken sein, gleichfalls ein Vieleck, insbesondere ein regelmäßiges Vieleck mit gleichlangen Seiten auf dem Umfang, mit beispielsweise sechs, acht, zwölf, sechzehn oder mehr Ecken. Mittels polyederförmiger Wandungsteilabschnitte kann eine relative Verdrehung, senkrecht zur Längsachse, der mehrteiligen Wandungsteilabschnitte zueinander verhindert werden, insbesondere, wenn diese ganz oder teilweise entlang einer Längsrichtung der medizinischen Vorrichtung ineinander eingeschoben sind. Zudem sind Körper mit polyederförmigem Querschnitt formstabiler als beispielsweise Körper mit rundem Querschnitt.

In einigen beispielhaften Ausführungsformen sind die wenigstens zwei Wandungsteilabschnitte zumindest teilweise ineinander einschiebbar oder zusammenschiebbar angeordnet und insbesondere entsprechend aufeinander abgestimmt dimensioniert. Beispielsweise können konzentrische Zylinderabschnitte dafür verschiedene Durchmesser aufweisen, so dass die Wandungsabschnitte aneinander entlang oder vorbei gleiten und so ineinander geschoben werden können. Hierzu kann ein geringfügiger Unterschied im Durchmesser bereits ausreichen.

In gewissen beispielhaften Ausführungsformen sind die Wandungsteilabschnitte mittels oder entlang wenigstens eines an oder in den Wandungsteilabschnitten angeordneten Führungsstifts zumindest teilweise ineinander einschiebbar angeordnet.

Der wenigstens eine Führungsstift kann beispielsweise an oder in einem inneren oder äußeren Wandungsteilabschnitt angeordnet sein. Der Führungsstift kann in einem Langloch, welches beispielweise mit einem Wandungsteilabschnitt verbunden ist, angeordnet sein. Die Führung kann eine lösbare Feder-Nut-Verbindung sein.

In manchen beispielhaften Ausführungsformen weist die erfindungsgemäße medizinische Vorrichtung eine Arretiereinrichtung zum lösbaren Fixieren der relativ zueinander verschiebbaren Wandungsteilabschnitte auf. Eine Arretiereinrichtung kann einen Haken, einen Schnappverschluss, einen Sicherungssplint oder ähnliches umfassen. Ein Sicherungssplint kann beispielsweise in hierfür vorgesehene Öffnungen oder Bohrungen eingesteckt werden, wobei die Öffnungen senkrecht oder auf andere Weise nichtparallel zur Bewegungsrichtung der verschiebbaren Wandungsabschnitte und/oder in diesen Wandungsabschnitten angeordnet sein können. Weiterhin kann eine Arretiereinrichtung den zuvor beschriebenen Führungsstift umfassen. Beispielsweise kann der Führungsstift, insbesondere in vorbestimmte Positionen, drehbar oder kippbar angeordnet sein, z. B. so, dass der Führungsstift die Funktion eines Sicherungssplints aufweist. Der Führungsstift kann in Langlöcher, die in einem vorgegebenen Winkel zur Bewegungsrichtung der verschiebbaren Wandungsabschnitte angeordnet sind, eingeführt oder eingesteckt werden. Der Winkel kann senkrecht zur Bewegungsrichtung der verschiebbaren Wandungsabschnitte angeordnet sein. Eine Arretierung der Wandungsabschnitte kann im ausgezogenen Zustand erfolgen, so dass ein ungewolltes Zusammenschieben der Wandungsabschnitte verhindert werden kann. Eine Arretierung der Wandungsabschnitte kann jedoch auch im zusammengeschobenen Zustand, oder im teilzusammengeschobenen Zustand erfolgen.

In einigen beispielhaften Ausführungsformen weist die Arretierung eine oder mehrere Klemmschrauben auf, mittels welcher die Wandungsteilabschnitte im ausgezogenen, im zusammengeschobenen oder im teilzusammengeschobenen Zustand arretiert, fixiert oder festgestellt werden können. Die Klemmschraube kann eine Rändelschraube sein.

In bestimmten beispielhaften Ausführungsformen weist der Druckbehälter eine erste Stirnseite und eine zweite Stirnseite auf.

In manchen beispielhaften Ausführungsformen weist der Druckbehälter eine erste Stirnseite und eine zweite Stirnseite auf, wobei der Auslass des Druckbehälters im Bereich der zweiten Stirnseite oder eines Stirnendes des Druckbehälters angeordnet ist, insbesondere in dessen Wandung.

In gewissen beispielhaften Ausführungsformen weist die erfindungsgemäße Vorrichtung wenigstens einen Schlauchabschnitt zum Abdecken der Fluidkammer oder Druckkammer gegen die mehrteilige Wandung auf. Der Schlauchabschnitt kann eine schlauchförmige Membran sein. Die schlauchförmige Membran kann fluiddicht sein. Der Schlauchabschnitt kann die Fluidkammer oder Druckkammer gegen die mehrteilige Wandung begrenzen.

Der Schlauchabschnitt kann ein Ineinanderschieben der mehrteiligen Wandung zulassen, ohne dass die Fluidkammer oder Druckkammer oder das Fluid unmittelbar mit den sich relativ zueinander bewegenden Wandungsteilabschnitten in Kontakt treten. Somit kann vorteilhaft eine Beschädigung der Fluidkammer oder der Druckkammer oder der Membran durch die Wandungsteilabschnitte vermieden werden. Eine Beschädigung könnte andernfalls durch vorstehende Kanten erfolgen, durch Einzwicken der Membran zwischen benachbarten Wandungsteilabschnitten während sich diese relativ zueinander bewegen, usw.

In gewissen beispielhaften Ausführungsformen ist der Schlauchabschnitt ergänzend zur hierin beschriebenen Membran vorgesehen, welche die Druckkammer bildet oder hieran beteiligt ist.

In manchen beispielhaften Ausführungsformen ist der Schlauchabschnitt wenigstens an der ersten Stirnseite (oder einem obersten Wandungsteilabschnitt) und an der zweiten Stirnseite (oder einem untersten Wandungsteilabschnitt) des Druckbehälters fixiert. Eine Fixierung kann eine Klemmung, eine Klebung oder eine andere wieder lösbare oder nicht wieder lösbare Verbindung sein.

In gewissen beispielhaften Ausführungsformen deckt der Schlauchabschnitt die sich relativ zueinander bewegenden Wandungsteilabschnitten ab. Das Material des Schlauchabschnitts kann textiles Gewebe oder Fasern umfassen oder aus diesem hergestellt sein. Weiterhin kann das Material zusätzlich oder ausschließlich Silikon oder ein silikonähnliches Material umfassen oder aus diesem hergestellt sein.

In einigen beispielhaften Ausführungsformen weist die Länge der zumindest teilweise ineinander einschiebbaren, mehrteiligen Wandung im vollständig eingeschobenen oder zusammengeschobenen Zustand weniger als 50% der Ausgangslänge der vollständig ausgezogenen Wandung auf. Beispielsweise ist die Länge der vollständig zusammengeschobenen Wandung bei drei gleich langen Wandungsteilabschnitten etwa ein Drittel der Länge im vollständig auseinandergezogenen Zustand.

In gewissen beispielhaften Ausführungsformen sind der Schlauchabschnitt und/oder die Membran in ihrer Länge einstellbar und damit für verschieden hohe Vorrichtungen verwendbar.

In einigen beispielhaften Ausführungsformen weist die medizinische Vorrichtung wenigstens einen Auslass auf, welcher eine Fluidverbindung zwischen der Druckkammer und dem Äußeren des Druckbehälters ist oder ermöglicht. Ferner weist sie ein Ventil, einen Sperrhahn oder eine andere Verschlussvorrichtung zum reversiblen Schließen des Auslasses oder der Fluidverbindung auf.

In manchen beispielhaften Ausführungsformen weist die erfindungsgemäße medizinische Vorrichtung einen Auslass auf, welcher eine Fluidverbindung zwischen der Fluidkammer oder Druckkammer und dem Äußeren des Druckbehälters ist, und ferner ein Ventil, eine Sperreinrichtung oder einen Sperrhahn, zum Öffnen und Schließen des Auslasses oder der Fluidverbindung.

In einigen beispielhaften Ausführungsformen ist die erfindungsgemäße medizinische Vorrichtung mit einer Fluidquelle oder einem Versorgungsbehälter, mit wiederum einem Inneren und einem Äußeren, verbunden oder weist eine(n) solche(n) auf. Hierbei weist sie ferner eine Fluidverbindung auf oder ist mit einer solchen verbunden. Mittels der Fluidverbindung stehen das Innere des Versorgungsbehälters oder der Fluidquelle einerseits und der Auslass andererseits in Kontakt und/oder in Fluidverbindung.

Der Auslass kann vorteilhaft genutzt werden, um den in der Druckkammer vorliegenden Druck durch Ablassen von Fluid aus der Druckkammer zu senken. Dies kann zum Einführen des Körpergliedstumpfs erforderlich oder hilfreich sein, oder zum Einstellen einer Einführtiefe des Stumpfs.

In gewissen beispielhaften Ausführungsformen weist die medizinische Vorrichtung einen Versorgungsbehälter mit einem Inneren und einem Äußeren auf oder ist hiermit verbunden. Weiterhin weist sie eine Fluidverbindung auf, wobei das Innere des Versorgungsbehälters und der Auslass in Kontakt und/oder in Fluidverbindung stehen. Anstelle des Versorgungsbehälters kann eine Fluidquelle oder Fluiddruckquelle mit der medizinischen Vorrichtung und der Fluidverbindung verbunden oder hierzu vorgesehen sein.

Das in der Druckkammer vorliegende Fluid kann einem Vorratsbehälter entnommen werden. Dieser kann, beispielsweise bei Ablassen von Fluid aus der Druckkammer, wie vorstehend beschrieben, das Fluid aufnehmen. Der Orthopädietechniker kann somit den Fluidstand oder -druck innerhalb der Druckkammer vorteilhaft ändern, ohne sich mit der Frage der Versorgung mit Fluid befassen zu müssen. Ebenso kann das abgelassene und vom Vorratsbehälter aufgenommene Fluid in diesem bis zu seiner nächsten Verwendung gespeichert werden. Das Verwenden der Vorrichtung mit einem Vorratsbehälter macht den Orthopädietechniker vorteilhaft unabhängig von einer externen Fluidquelle.

In einigen beispielhaften Ausführungsformen stehen das Innere des Druckbehälters und das Innere des Versorgungsbehälters somit mittels des Auslasses in Fluidverbindung. Diese kann jedoch durch das Ventil unterbunden werden.

In gewissen beispielhaften Ausführungsformen ist der Vorratsbehälter ein Faltenbalg. In anderen ist er ein im leeren Zustand flacher Folienbeutel (ähnlich einer fluidisch abgedichteten Kunststoffeinkaufstüte) mit einem Ein- und/oder Auslass. Bei letzterer Ausgestaltung ist er im leeren Zustand einfach aufrollbar oder dünn faltbar und kann platzsparend transportiert werden. Eine Öffnung oder ein Griff können zum Halten, Heben oder Aufhängen vorgesehen sein.

In manchen beispielhaften Ausführungsformen ist der Druckbehälter zumindest teilweise in einem Inneren des Vorratsbehälters angeordnet. Dies erlaubt vorteilhaft ein Verschieben von Fluid zwischen Druckkammer und Vorratsbehälter, ohne dass es hierzu einer Leitung bedarf.

In einigen beispielhaften Ausführungsformen weist der Druckbehälter eine erste und eine zweite Stirnseite auf. Dabei ist der Auslass des Druckbehälters im Bereich der zweiten Stirnseite oder eines Stirnendes des Druckbehälters angeordnet. Es genügt somit, den stehend verwendeten Druckbehälter nur mit dessen unteren Bereich im Vorratsbehälter zu platzieren.

In gewissen beispielhaften Ausführungsformen ist der Vorratsbehälter flüssigkeitsdicht gegenüber einem Äußeren. Diese Ausgestaltung kann zu einem verlustfreien und kleckerfreien Verwenden der Vorrichtung selbst bei Austausch von Fluid zwischen Druckkammer und Vorratsbehälter führen.

In manchen beispielhaften Ausführungsformen weisen der Druckbehälter und/oder der Vorratsbehälter eine Druckquelle auf, die zum Beaufschlagen der Druckkammer des Druckbehälters und/oder des Inneren des Vorratsbehälters mit Druck dient, oder sind mit der Druckquelle in Fluidverbindung verbunden.

In einigen beispielhaften Ausführungsformen ist der Druckbehälter mit dem Auslass oder mit einem hiervon getrennt vorliegenden Einlass mit der Druckquelle verbunden. Es können somit zwei Durchlässe in der Wandung vorliegen, nämlich Auslass und Einlass.

In einigen beispielhaften Ausführungsformen weisen die Druckkammer des Druckbehälters und/oder der Vorratsbehälter in seinem/ihrem Inneren Wasser oder eine andere Flüssigkeit auf oder sind hiermit gefüllt. Eine Flüssigkeit führt aufgrund ihrer fehlenden Komprimierbarkeit zu reproduzierbareren Ergebnissen beim Fertigen des Gipsabdrucks. Wasser ist dabei die billigste und am einfachsten verfügbare Variante.

In gewissen beispielhaften Ausführungsformen sind die Wandung des Druckbehälters und/oder der Vorratsbehälter zumindest in Abschnitten hiervon transparent. Dies kann eine visuelle Überwachung der Einführtiefe und anderer Aspekte beim Fertigen des Gipsabdrucks durch den Orthopädietechniker auf einfache Weise erlauben.

In manchen beispielhaften Ausführungsformen weisen der Druckbehälter und/oder der Vorratsbehälter im Bereich wenigstens eines transparenten Abschnitts hiervon eine oder mehrere Markierungen auf. An ihnen kann die Einführtiefe des Körpergliedstumpfs abgelesen werden.

In einigen beispielhaften Ausführungsformen weist der Druckbehälter einen mehrteiligen Dichtring, insbesondere einen Oberschenkeldichtring auf, in anderen Ausführungsformen weist er keinen solchen Ring auf. Dieser ist, falls vorgesehen, am Druckbehälter im Bereich der Einführöffnung oder eines Klemmrings bzw. eines Befestigungsrings, welcher die Membran trägt, lösbar verbindbar, beispielsweise anschraubbar. Er kann ein Auftreiben der Membran in ein Äußeres des Druckbehälters aufgrund des jenseits der Membran herrschenden Drucks verhindern oder begrenzen. Dies kann zu korrekteren Druckverhältnissen und Ergebnissen des Abdrucks führen. Zudem kann die Membran vor einem Abscheren beispielsweise am Rand der Einführöffnung oder einem anderweitigen Verletzen der Membran, etwa im Spalt zwischen Körpergliedstumpf und Rand der Einführöffnung, bewahrt werden.

In gewissen beispielhaften Ausführungsformen weist der Druckbehälter eine erste und eine zweite Stirnseite auf.

Der Vorratsbehälter oder der Druckbehälter weisen in manchen erfindungsgemäßen, beispielhaften Ausführungsformen eine Aufstellvorrichtung auf, mittels welcher die Vorrichtung in einer Gebrauchsstellung gehalten wird. In dieser konkreten Gebrauchsstellung liegen die erste Stirnseite oben und die zweite Stirnseite unten.

Die Vorrichtung ist in gewissen erfindungsgemäßen, beispielhaften Ausführungsformen auch zum Versorgen von Patienten geeignet, welche eine Oberschenkelprothese benötigen. Die erfindungsgemäße Vorrichtung kann zur Versorgung solcher Patienten eine Aufsitzvorrichtung aufweisen. Diese kann dazu dienen, dass sich der Patient hinsetzt oder mittels knöcherner Beckenstrukturen zumindest in horizontaler Richtung abstützen kann.

Die Aufsitzvorrichtung kann mit einem Abschnitt der Vorrichtung oder des Fluidbehälters, beispielsweise mit dessen Wandung, lösbar oder dauerhaft verbunden sein, beispielsweise durch Aufstecken, Aufklemmen, Verschrauben oder dergleichen.

Die Aufsitzvorrichtung kann die Form eines Sattels oder eines Abschnitts eines Sattels haben.

Die Aufsitzvorrichtung kann der Aufsitzvorrichtung, wie sie vom vorliegenden Anmelder unter der DE 10 2015 102 185.4 mit dem Titel "Aufsitzvorrichtung und Sitzmöbel zum Erstellen eines Gipsabdrucks am sitzenden Patienten zum Fertigen eines Prothesenschafts für die untere Extremität" am 16.02.2015 beim DPMA eingereicht wurde, oder eine, insbesondere vorderen, Abschnitts hiervon, entsprechen. Die diesbezügliche Offenbarung jener Anmeldung wird hiermit mittels Verweises vollumfänglich aufgenommen.

In einer bestimmten beispielhaften Ausführungsform erstreckt sich die Aufsitzvorrichtung in eine Längsrichtung und in eine Querrichtung hiervon. Sie weist wenigstens einen sich in der Längsrichtung erstreckenden Sitzabschnitt auf, auf welchem der Patient rittlings sitzen kann, also wie auf einem Sattel, so dass sich der Sitzabschnitt von vorne nach hinten zwischen den Oberschenkeln hindurch erstreckt. Sie weist ferner optional wenigstens ein sich in der Querrichtung erstreckendes Anrutsch-, Ansitz- oder Anschlagelement auf (im Folgenden: Anschlagelement), welches geeignet ist, ein Rutschen des rittlings auf dem Sitzabschnitt sitzenden Patienten entlang der Aufsitzvorrichtung oder entlang des Sitzabschnitts zu begrenzen. Diese optionale Begrenzung ist derart, dass der Patient möglicherweise entlang des Sitzabschnitts zwar in einer Richtung (nach dorsal, bezogen auf den bestimmungsgemäß sitzenden Patienten) entlang der Längsrichtung oder der Längsachse des Sitzabschnitts ungehindert rutschen kann, in der entgegengesetzten (nach ventral, bezogen auf den bestimmungsgemäß sitzenden Patienten) allerdings nur bis gegen das Anschlagelement. Das Anschlagelement ist optional.

Es muss erfindungsgemäß also nicht vorgesehen sein. Seine Funktion könnte beispielsweise durch die Hand des Orthopädietechnikers erbracht oder ersetzt werden. Da das Anschlagelement jedoch das Fertigen des Gipsabdrucks maßgeblich vereinfachen kann, ist ein Anschlagelement vorzugsweise von der Aufsitzvorrichtung umfasst. Die Längsrichtung kann jene Richtung sein, welche, wenn der Patient bestimmungsgemäß auf der Aufsitzvorrichtung Platz genommen oder auf diese gesetzt wurde, bezogen auf diesen von ventral nach dorsal oder von vorne nach hinten oder durch die Oberschenkel hindurch verläuft. Die Querrichtung kann jene Richtung sein, welche, wenn der Patient bestimmungsgemäß auf der Aufsitzvorrichtung Platz genommen oder auf diese gesetzt wurde, bezogen auf diesen, von caudal nach cranial oder vom Becken zum Kopf verläuft.

In manchen erfindungsgemäßen, beispielhaften Ausführungsformen ist die Druckkammer durch wenigstens einen Abschnitt der Wandung und durch die Membran gebildet oder begrenzt. Die Membran ist mit dem Abschnitt der Wandung, vorzugsweise im Bereich der zweiten Stirnseite des Druckbehälters, stoffschlüssig, kraftschlüssig und/oder formschlüssig verbunden. Hierzu kann ein Verbinder, oder eine Verbindungseinrichtung, vorgesehen sein. Mittels des Verbinders kann in einigen erfindungsgemäßen Ausführungsformen ein ungewünschtes Auswölben, Wandern, Auftreiben oder Erstrecken der Membran noch oben oder in ein Äußeres des Druckbehälters, in welchem die Membran - anders als im Inneren des Druckbehälters - nicht durch die Wandung seitlich gestützt wird, verhindert oder auf ein als zulässig erachtetes Maß beschränkt werden. Der Verbinder hält die Membran somit, zumindest im Wesentlichen, optional im Inneren des Druckbehälters. Dies, oder der Verbinder, kann einem unerwünschten Auftreiben des Körpergliedstumpfs entgegenwirken. Das Auftreiben kann zu einer ungünstigen Beeinflussung des in der Druckkammer und mittels der Membran auf den Körpergliedstumpf herrschenden Druck haben, indem die Membran nicht mehr in allen Abschnitten, in welchen sie den Stumpf umgibt, diesen mit einheitlichem Druck anliegt. Damit aber liegen beim Fertigen des Gipsabdrucks nicht die optimalen Drücke am Stumpf an mit der Folge, dass der gefertigte Gipsabdruck nicht unter späteren Belastungen, die beim Gehen mit der zu fertigenden Prothese auftreten, gefertigt wurde. Ferner kann ein vermindertes oder verhindertes Auftreiben einen Beitrag zum Schutz der Membran leisten, welche im Inneren des Druckbehälters durch dessen Wandung vor Beschädigung geschützt ist. Es bedarf beim Vorsehen des Verbinders erfindungsgemäß vorteilhaft keiner anderweitigen Begrenzung eines Auftreibens der Membran, etwa durch einen eng am Oberschenkel anliegenden Ring. Da ein solcher Ring in einer Vielzahl von Größen vorgehalten werden müsste, um mit der erfindungsgemäßen Vorrichtung Gipsmodelle für eine Vielzahl unterschiedlich dicker Körpergliedstümpfe fertigen zu können, liegt eine für den Fachmann leicht nachzuvollziehende Vereinfachung vor. Sie bedeutet neben einer Vereinfachung des Gebrauchs der Vorrichtung auch eine Einsparung an Material für Ringe, Kosten und dergleichen.

In manchen erfindungsgemäßen, beispielhaften Ausführungsformen ist der Verbinder mit der Membran in einem Abschnitt der Membran verbunden, welcher nicht im Bereich der Einführöffnung liegt.

In bestimmten beispielhaften Ausführungsformen ist die Membran im Bereich der Einführöffnung oder der ersten Stirnseite des Druckbehälters mit diesem verbunden, und zusätzlich in einem zweiten, hiervon verschiedenen Bereich oder Abschnitt des Druckbehälters. Der zweite Bereich kann vorzugsweise im Inneren des Druckbehälters und/oder in der Druckkammer liegen. Die Membran kann vorzugsweise mittels des Verbinders mit dem zweiten Bereich verbunden sein. Die Membran kann vorzugsweise ausschließlich indirekt mit dem zweiten Bereich in Kontakt stehen, nämlich optional mittels des Verbinders, also vorzugsweise selber keinen direkten Kontakt mit dem zweiten Bereich haben, diesen vorzugsweise also nicht berühren.

In einigen beispielhaften Ausführungsformen ist der zweite Bereich ein zentraler Abschnitt oder die Mitte der Bodenfläche, der zweiten Stirnfläche oder -seite.

In manchen beispielhaften Ausführungsformen ist der Verbinder eine elastische Feder oder weist ein elastisches Element auf.

In einigen beispielhaften Ausführungsformen ist der Verbinder nicht elastisch oder nicht dehnbar.

In einigen beispielhaften Ausführungsformen ist der Verbinder längenverstellbar. Mittels einer entsprechenden Verstelleinrichtung, welche vorzugsweise vom Äußeren des Druckbehälters aus verstellbar ist, kann seine Länge verstellt werden. Hierdurch wird der Abstand zwischen distalem Ende der Membran und beispielsweise der Bodenfläche, unteren Stirnseite oder Stirnfläche des Druckbehälters verändert. Dies erlaubt es, die Membran innerhalb des Druckbehälters veränderlich auszurichten, was ein optimales Einstellen der erfindungsgemäßen Vorrichtung auf den konkreten Körpergliedstumpf ungeachtet seiner Länge erlauben kann.

In manchen beispielhaften Ausführungsformen verbindet der Verbinder die Membran, direkt oder indirekt, mit einer Stirnseite des Druckbehälters, welche dem Ende des Druckbehälters, welches die Einführöffnung aufweist, gegenüberliegt, also insbesondere mit einer unteren Stirnseite, Stirnfläche oder Bodenfläche.

In manchen beispielhaften Ausführungsformen verbindet der Verbinder die Membran mit einem mittigen oder zentralen Bereich der Stirnfläche, Stirnseite oder Bodenfläche. Dies erlaubt oder begünstigt eine vergleichsweise gerade Anordnung des in die Membran eingeführten Körpergliedstumpfs innerhalb des Druckbehälters, was das Aufwerfen von Falten der Membran verhindern und eine gleichmäßige Druckbeaufschlagung begünstigen kann. Das Vermeiden von Falten kann ferner von Vorteil sein, da Falten eine - insbesondere digitale und/oder automatische - Vermessung des in den Druckbehälter oder Behälter eingeführten Körpergliedstumpfs erschweren können.

In manchen beispielhaften Ausführungsformen wird unter einem Verbinden ein form- und/oder kraftschlüssiges und/oder stoffschlüssiges Verbinden verstanden.

In manchen beispielhaften Ausführungsformen ist der Verbinder angeordnet, um einen im Gebrauch untersten Abschnitt der Membran, oder einen zentralen Abschnitt der Membran, zu verbinden.

In manchen beispielhaften Ausführungsformen ist der Verbinder angeordnet, um einen Abstand zwischen dem mit dem Verbinder verbundenen Abschnitt der Membran einerseits und dem mit dem Verbinder ebenfalls verbundenen Abschnitts der Bodenfläche, unteren Stirnseite oder Stirnfläche andererseits innerhalb vorgegebener Grenzen zu halten. Der Abstand kann bei nicht elastischem Verbinder beispielsweise konstant sein.

In manchen beispielhaften Ausführungsformen ist die Membran direkt mit einem zentralen Bereich der Bodenfläche, Bodenseite oder unteren Stirnseite verbunden. Der Verbinder kann hierbei das Ergebnis eines Fügeverfahrens sein, etwa ein Klebeabschnitt, ein Niet oder dergleichen.

In manchen beispielhaften Ausführungsformen berührt die Membran im Bereich ihrer Verbindung die Bodenfläche, Bodenseite oder unteren Stirnseite, in anderen Ausführungsformen berührt die Membran oder Material der Membran sie nicht.

In einigen beispielhaften Ausführungsformen weist die Membran eine Verstärkungseinrichtung auf oder ist hiermit stoffschlüssig, kraftschlüssig und/oder formschlüssig verbunden. Die Verstärkungseinrichtung ist ferner auch mit dem Druckbehälter, dessen Wandung oder einem anderen Abschnitt der erfindungsgemäßen Vorrichtung stoffschlüssig, kraftschlüssig und/oder formschlüssig verbunden. Sie kann die Membran ähnlich wie der vorstehend beschriebene Verbinder vorzugsweise daran hindern, aufzuschwimmen oder aufzutreiben. Die Verstärkungsvorrichtung kann damit als Ergänzung als Alternative zum Verbinder verstanden werden. Sie kann eine Stange sein. Sie kann angeordnet sein, um mittels Schubs oder Drucks beansprucht zu werden, möglicherweise aber nicht mittels Zugs.

In gewissen beispielhaften Ausführungsformen ist die Membran aus einem Material gefertigt oder weist ein solches auf, welches in einer ersten Richtung des Materials eine andere Elastizität oder Dehnbarkeit des Materials aufweist als in einer von der ersten Richtung verschiedenen zweiten Richtung, wobei die zweite Richtung zur ersten Richtung optional senkrecht stehen kann. In diesen oder anderen erfindungsgemäßen, beispielhaften Ausführungsformen wird optional die in der ersten und/oder zweiten der beiden Richtungen jeweils spezifische Elastizität oder Dehnbarkeit durch Fasern erreicht, welche die Membran aufweist, welche beispielsweise in Silikon, eine Silikonmatrix oder ein anderes Material, vorzugsweise ein fluiddichtes Material, eingebettet sind. Solche Fasern können in der ersten und/oder zweiten Richtung der Membran verlaufen, im Wesentlichen in der ersten und/oder zweiten Richtung verlaufen und/oder im Wesentlichen in der ersten und/oder zweiten Richtung wirken. In diesen oder anderen erfindungsgemäßen Ausführungsformen werden die unterschiedlichen Elastizitäten oder Dehnbarkeiten ergänzend oder alternativ durch andere Ausgestaltungen erreicht.

Die erste Richtung kann, wenn die Membran gebrauchsfertig oder bestimmungsgemäß am Druckbehälter befestigt ist, eine Einführrichtung des Körpergliedstumpfs oder eine Längsrichtung des Druckbehälters sein. Die zweite Richtung kann im Winkel, z. B. im rechten Winkel, zur ersten Richtung stehen.

Fasern, welche sich in der ersten Richtung erstrecken oder verlaufen oder wirken, sind optional nicht oder in nur geringem Umfang dehnbar. Optional sind sie weniger dehnbar als optional vorgesehene Fasern, welche in der zweiten Richtung verlaufen, sich erstrecken oder wirken. Fasern, welche in der zweiten Richtung verlaufen, wirken oder sich erstrecken, können, sofern vorgesehen, dehnbar sein, sie können dehnbarer oder elastischer sein als jene Fasern der ersten Richtung; ihre Dehnbarkeit kann optional jener der Fasern der in erster Richtung verlaufenden Fasern entsprechen. Die in der ersten Richtung verlaufenden Fasern sind optional, ebenso die in der zweiten Richtung verlaufenden Fasern. Die Dehnbarkeit oder Elastizität der Fasern kann der Dehnbarkeit oder Elastizität der Membran entsprechen oder hierzu korrelieren. Die Fasern können aus Nylon sein oder Nylon aufweisen. Sie können aus zugfestem Material sein, insbesondere aus Material, welches zugfester ist als das sie einbettende Material. Eine solche Ausgestaltung, welche beispielsweise eine Längsdehnung (welche optional die erste Richtung sein kann) verhindert oder merklich beschränkt, zugleich aber eine Dehnung der Membran in Umfangs- oder Radialrichtung zu ihrem Anpassen an den Körpergliedstumpf erlaubt, kann ebenfalls einem unerwünschten Auftreiben des Körpergliedstumpfs vorteilhaft entgegenwirken. Zudem kann das Verhältnis zwischen der Tiefe der Druckkammer und der Einführtiefe innerhalb der Membran vorteilhaft gleich bleiben. Zudem kann optional auf den o. g. mehrteiligen Dichtring oder Oberschenkeldichtring verzichtet werden. Ferner kann es möglich sein, mit nur einer Membran erfindungsgemäß Gipsabdrücke an einer ganzen Reihe von im Querschnitt unterschiedlich dicken Körpergliedstümpfen zu fertigen. Die Membran legt sich dabei vorteilhaft sowohl bei dicken als auch bei dünnen Stümpfen ausreichend eng an, um das hierin beschrieben Faltenwerfen der Membran zu vermeiden.

Die Quer- oder Umfangsdehnbarkeit, welche die Membran in einigen erfindungsgemäßen Ausführungsformen selbst dann aufweisen kann, wenn die Längsdehnbarkeit verhindert oder beschränkt ist, trägt hierzu vorteilhaft bei.

Optional ist die Membran vorteilhaft ausgewählt und angeordnet, dass die Membran aufgrund ihrer Dehnbarkeit eine Eintrittsöffnung zum Einführen des Stumpfs in das Innere des von der Membran in einigen Ausführungsformen gebildeten Schlauchs in einem Bereich von 3 cm Durchmesser bis annähernd den Innendurchmesser der Wandung oder des Druckbehälters erlaubt.

In einigen beispielhaften Ausführungsformen weist die medizinische Vorrichtung eine Heizeinrichtung auf. Sie kann angeordnet sein, um das in der Druckkammer vorliegende Fluid zu erwärmen oder geregelt zu erwärmen. Wird beispielsweise Wasser als Fluid verwendet, so kann das Einführen des Körpergliedstumpfs in die Membran für den Patienten angenehmer sein, wenn deren Temperatur, welche in etwa der Wassertemperatur entsprechen wird, bei beispielsweise 30° C oder bei Körpertemperatur liegt, als wenn das Wasser beispielsweise Zimmertemperatur oder die Temperatur hat, mit welcher es einer Wasserleitung entnommen wurde. Zudem kann ein warmes Fluid zu einem rascheren Trocknen der Gipsbinde beitragen als kaltes Fluid.

In manchen beispielhaften Ausführungsformen weist die medizinische Vorrichtung eine Rolleneinrichtung zum Rollen der Vorrichtung auf einen Untergrund, also zum Fortbewegen auf rollende Weise, mit den bekannten Vorteilen auf. Die Rolleneinrichtung kann lösbar vorgesehen sein.

In einigen beispielhaften Ausführungsformen weist die medizinische Vorrichtung eine Stütze auf, an welcher sich der stehende Patient bei durch die Einführöffnung hindurch in das Innere eingeführtem Körpergliedstumpf abstützen kann.

In manchen beispielhaften Ausführungsformen weist die medizinische Vorrichtung eine Aufhängvorrichtung auf. Sie erlaubt es, die Vorrichtung beispielsweise zum Anfertigen eines Gipsabdrucks für eine Unterarmprothese beispielsweise an der Wand aufzuhängen. Der Patient kann auf vorbestimmte Weise den Arm gegen die Membran drücken.

In gewissen beispielhaften Ausführungsformen ist die Stütze einklappbar, teleskopierbar und/oder verschwenkbar ausgestaltet. Sie kann ferner derart ausgestaltet sein, dass sie höhenverstellbar und/oder von einer Gebrauchsstellung in eine Nichtgebrauchsstellung überführbar ist. Die Vorrichtung lässt sich somit auf einfache Weise an den konkreten Patienten anpassen, sich einfacher transportieren, und kann bei vergleichsweise geringem Raumbedarf bis zu ihrer nächsten Verwendung verstaut werden.

In manchen beispielhaften Ausführungsformen begrenzt die Wandung des Druckbehälters die Druckkammer. Ferner weist sie einen Einlass auf, welcher zum Einbringen von Fluid zum Erhöhen des in der Druckkammer des Druckbehälters herrschenden Drucks dient. Der Einlass kann der o. g. Auslass sein oder eine eigenständige Vorrichtung.

In einigen beispielhaften Ausführungsformen ist die Membran teilweise, in wenigstens einem Abschnitt hiervon oder vollständig bzw. insgesamt weniger als 2 mm, vorzugsweise weniger als 1 mm dick. Bei dieser Dicke verfälscht sie den Unterschied zwischen der tatsächlichen Geometrie des Körpergliedstumpfs und dem Gips in einem allenfalls vernachlässigbaren Maß.

In gewissen beispielhaften Ausführungsformen weisen der Druckbehälter und/oder der Vorratsbehälter eine verschließbare Luftablassöffnung mit entsprechender Verschließeinrichtung auf. Aus dieser kann Luft in deren nicht verschlossenem Zustand aus der Druckkammer und/oder dem Inneren des Vorratsbehälters entweichen. Dies ist beim erstmaligen Befüllen der Druckkammer mit einer Flüssigkeit von Vorteil; die Vorrichtung, sofern sie mit Flüssigkeit befüllt verwendet werden soll, muss nicht mit dieser befüllt erworben und ausgeliefert werden. Sie kann luftgefüllt und damit ungleich leichter transportiert werden. Die vorhandene Luft kann beim Befüllen mit Flüssigkeit aus der verschließbaren Luftablassöffnung ausgelassen werden.

In manchen beispielhaften Ausführungsformen weist der Vorratsbehälter eine Federeinrichtung und einen Raum veränderlicher Größe zur Aufnahme des Fluids auf. Die Federeinrichtung kann angeordnet sein, um den Raum mittels Federkraft zu verringern (etwa, indem eine bewegbare Wand des Raums dem Federdruck ausgesetzt ist). Die Federkraft kann dabei eingestellt oder mittels eines vorgesehenen Mechanismus einstellbar sein, um im Raum einen Fluiddruck aufrecht zu erhalten, welcher eine gewünschte Einführtiefe des Körpergliedstumpfs erlaubt, begrenzt oder sicherstellt. Ferner oder alternativ kann die Federkraft bewirken, dass sich die Druckkammer nach der Behandlung eines Patienten, zu deren Zweck etwas Fluid aus der Druckkammer abgelassen und in den Vorratsbehälter eingelassen oder übergeführt wurde, automatisch wieder befüllt. Damit wird die Druckkammer automatisch für die Behandlung des nächsten Patienten befüllt. Ferner mag man sich bei einer solchen Ausgestaltung eine Schlauchverbindung zwischen Druckkammer und Vorratsbehälter einsparen.

In gewissen beispielhaften Ausführungsformen sind Druckbehälter und Vorratsbehälter einstückig oder integral ausgestaltet. Dies kann vorteilhaft eine zuverlässige Dichtheit zwischen Druckbehälter und Vorratsbehälter bewirken.

In manchen beispielhaften Ausführungsformen weist die medizinische Vorrichtung eine Einrichtung auf, mittels welcher wenigstens der Druckbehälter bezogen auf einen Untergrund, auf welchem die Vorrichtung ruht oder der Patient mit seinem gesunden Bein steht, höhenverstellbar ist.

In gewissen beispielhaften Ausführungsformen können das Innere und das Äußere des Druckbehälters oder des Vorratsbehälters durch jeweils eine Wandung voneinander getrennt sein.

In gewissen beispielhaften Ausführungsformen ist die Wandung des Druckbehälters und/oder die Wandung des Vorratsbehälters aus Kunststoff, beispielsweise Plexiglas oder Polycarbonat oder weist eines oder mehrere dieser Materialien auf.

In einigen beispielhaften Ausführungsformen ist die Wandung des Vorratsbehälters aus Aluminium, einer Legierung, Edelstahl oder weist eines oder mehrere dieser Materialien auf.

In manchen beispielhaften Ausführungsformen ist die Wandung des Vorratsbehälters aus PVC (Polyvinylchloride), in anderen hingegen nicht.

In gewissen beispielhaften Ausführungsformen weist die Vorrichtung keinen Rand oder Ring mit einer Öffnung für den Oberschenkel des Patienten auf, welcher ausgestaltet ist, um mit dem Druckbehälter und/oder der Wandung lösbar verbunden zu werden.

In gewissen beispielhaften Ausführungsformen weist der Druckbehälter und/oder die Wandung keine Luftöffnung auf.

In einigen beispielhaften Ausführungsformen weist der Druckbehälter in seinem Inneren keine zur Wandung beabstandete Streben auf, insbesondere keine solchen, welche Verbindung zur Membran haben und/oder insbesondere keine solchen, welche sich in Längsrichtung des Druckbehälters erstrecken.

In bestimmten beispielhaften Ausführungsformen ist wenigstens ein, vorzugsweise zentraler oder mittlerer, Abschnitt der Membran mittels wenigstens eines Verbinders an einem Abschnitt der Wandung, vorzugsweise lösbar, befestigt.

In bestimmten beispielhaften Ausführungsformen ist wenigstens ein, vorzugsweise zentraler oder mittlerer, Abschnitt der Membran mittels wenigstens eines Verbinders, vorzugsweise lösbar, vorzugsweise direkt, an einer Bodenfläche oder an der zweiten Stirnseite des Druckbehälters, und vorzugsweise in einem mittleren oder zentralen Bereich oder Abschnitt hiervon, befestigt.

In bestimmten beispielhaften Ausführungsformen weist der Verbinder, die Membran und/oder die Bodenfläche wenigstens ein Gewinde zum Verschrauben der Membran oder eines hiermit verbundenen Elements, direkt oder indirekt, mit dem Druckbehälter auf.

In bestimmten beispielhaften Ausführungsformen begrenzt der Verbinder ein durch Fluid in der Druckkammer erzeugtes Auftreiben der Membran derart, dass eine Kontaktpunktfläche zwischen Körpergliedstumpf und Membran auf selber Höhe liegt wie ein Übergangsabschnitt, in welchem die Druckkammer gerade nicht mehr von der Wandung (oder einem hiermit verbundenen Element wie einem Klemmring) sondern von der Membran gebildet wird und/oder die Membran nicht mehr von der Wandung (oder einem hiermit verbundenen Element wie einem Klemmring) - insbesondere, radial, lateral oder seitlich - gestützt wird.

In bestimmten beispielhaften Ausführungsformen ist die Membran aus einem Material gefertigt oder weist ein solches auf, welches in einer ersten Richtung und/oder in einer zweiten Richtung des Materials Fasern aufweist, welche in eine Matrix eingebettet oder mit dieser auf andere Weise verbunden sind.

In bestimmten beispielhaften Ausführungsformen sind die Membran, welche optional eine Matrix aufweist, oder ihre Matrix, aus Silikon oder weisen Silikon auf.

In bestimmten beispielhaften Ausführungsformen haben manche oder alle der Fasern einen gewellten, kurvigen oder zig-zag-Verlauf.

In bestimmten beispielhaften Ausführungsformen ist die Membran in einer ersten Richtung hiervon und/oder in einer zweiten Richtung hiervon nicht dehnbar oder nicht elastisch.

In bestimmten beispielhaften Ausführungsformen umfasst das Verfahren ein Anpassen einer Länge des Verbinders derart, dass eine durch Fluid in der Druckkammer erzeugtes Auftreiben der Membran derart ist, dass eine Kontaktpunktfläche zwischen Körpergliedstumpf und Membran auf selber Höhe liegt wie ein Übergangsabschnitt, in welchem die Druckkammer gerade nicht mehr von der Wandung sondern von der Membran gebildet wird.

In bestimmten beispielhaften Ausführungsformen bedeutet "nicht-dehnbar" oder "nichtelastisch", dass der E-Modul des betreffenden Bauteils (Verbinder, Membran, Fasern, usw.) wenigstens oberhalb 700 N/mm², vorzugsweise oberhalb 1000 N/mm², besonders bevorzugt oberhalb 2000 N/mm², liegt.

In bestimmten beispielhaften Ausführungsformen bedeutet "nicht-dehnbar" oder "nichtelastisch", dass eine Dehnung des betreffenden Bauteils (Verbinder, Membran, Fasern, usw.) nicht mehr als 20%, bevorzugt nicht mehr als 10%, vorzugsweise nicht mehr als 5%, besonders bevorzugt nicht mehr als 2% seiner Länge betragen darf, bevor das Bauteil reißt oder bricht.

In bestimmten beispielhaften Ausführungsformen hat die Membran, Fasern hiervon und/oder der Verbinder einen E-Modul wie Nylon.

In manchen beispielhaften Ausführungsformen umfasst das Verfahren das Einführen des mit einer feuchten Gipsbinde umwickelten Körpergliedstumpfs in die Membran derart, dass der Körpergliedstumpf zumindest in Abschnitten hiervon um seinen gesamten Umfang herum von der Membran umgeben ist.

In gewissen beispielhaften Ausführungsformen umfasst das Verfahren das Vorsehen eines Haftstrumpfs über der feuchten Gipsbinde, bevor der Körpergliedstumpf in das Innere des Druckbehälters eingeführt wird. Das Haftstrumpfmaterial ist luftdurchlässig und erlaubt ein Entweichen von Luft, welche sich vor dem Einführen in der Membran befinden kann, entlang des Körpergliedstumpfs zum Äußeren des Druckbehälters hin. Hierdurch kann vorteilhaft vermieden werden, dass das distale Ende des Körpergliedstumpfs während des Anfertigens des Gipsabdrucks auf einem Luftnest oder -polster ruht, welches die Form des Gipsabdrucks im Bereich des distalen Stumpfendes als Artefakt verzerren könnte. Anstelle oder ergänzend zum Haftstrumpf kann ein Schlauch oder dergleichen zum Ableiten von o. g. Luft vorgesehen sein. Das freie Ende des Schlauchs kann entlang des Körpergliedstumpfs zur Einführöffnung zurückgeführt werden (auf beliebiger Seite der Membran, d. h. durch das Fluid oder auf der Gipsbinde). Dieses Ende kann aber auch das Ende eines blind endenden Schlauchs sein, welcher z. B. im Fluid liegt.

In einigen erfindungsgemäßen, beispielhaften Ausführungsformen erfolgt das Einführen bei stehendem Patienten.

In gewissen beispielhaften Ausführungsformen liegt die feuchte Gipsbinde auf der nackten Haut des Körpergliedstumpfs auf. Auch dies trägt vorteilhaft dazu bei, dass bei erfindungsgemäßem Vorgehen der Unterschied zwischen der tatsächlichen Geometrie des Körpergliedstumpfs und dem Gips auf ein vernachlässigbares Maß verringert wird.

In manchen beispielhaften Ausführungsformen umfasst das Verfahren das Regulieren oder Steuern der Einführtiefe, mit welcher der Körpergliedstumpf in den Druckbehälter eingeführt ist. Diese wird durch Öffnen und/oder Schließen des Auslasses oder des Ventils oder Sperrhahns reguliert. Idealerweise wird das Ventil oder der Sperrhahn oder eine andere Absperreinrichtung geschlossen, bevor das distale Ende des Körpergliedstumpfs in Kontakt mit der zweiten Stirnseite gekommen ist. Es wird bei diesen Ausführungsformen also darauf geachtet, dass unter dem distalen Stumpfende stets noch Fluid vorliegt. Hierdurch kann der Körpergliedstumpf wie gewünscht in dem Fluid und auf dem Fluid ruhen. Dieses drückt dabei über oder auf die gesamte Fläche der Gipsbinde.

In einigen beispielhaften Ausführungsformen umfasst das Verfahren das Regulieren oder Steuern der Einführtiefe, mit welcher der Körpergliedstumpf in den Druckbehälter eingeführt ist als weiteren Schritt, wobei dies durch Öffnen und/oder Schließen des Auslasses oder des Ventils oder des Sperrhahns und/oder durch Anpassen einer Länge des Verbinders erfolgt. Und zwar derart, dass eine durch Fluid in der Druckkammer erzeugte Auftreibung der Membran derart ist, dass eine Kontaktpunktfläche zwischen Körpergliedstumpf und Membran auf selber Höhe liegt wie ein Übergangsabschnitt, in welchem die Druckkammer gerade nicht mehr von der Wandung, sondern von der Membran gebildet wird.

In manchen beispielhaften Ausführungsformen umfasst das Verfahren ein Erhöhen des in der Fluidkammer oder Druckkammer des Druckbehälters herrschenden Drucks als weiteren Schritt, beispielsweise um 0,2 bis 0,3 bar.

In einigen beispielhaften Ausführungsformen umfasst das Verfahren ein Erhöhen des in der Druckkammer des Druckbehälters herrschenden Drucks, insbesondere um 0,05 und 0,5 bar, ganz insbesondere um 0,2 bis 0,3 bar, insbesondere beim Patienten, welcher mit seinem Körpergliedstumpf im Druckbehälter steht. Das Erhöhen des Drucks erlaubt es, einen unter höherem Druck als üblich geformten Gipsabdruck zu erhalten. Ein hierauf basierend gefertigter Schaft kann Patienten, welche den Schaft etwa beim Sport überdurchschnittlich belasten, entgegenkommen, da der Schaft auch zum Übertragen eines entsprechend höheren Drucks vorbereitet sein wird.

In manchen beispielhaften Ausführungsformen weisen der Druckbehälter und/oder eine hiermit kommunizierende Leitung ein Manometer auf, welches den in der Druckkammer herrschenden Druck bestimmt oder misst. Der Orthopädietechniker kann sich beispielsweise für die vorstehend genannte Druckerhöhung am gemessenen Druck orientieren.

In gewissen beispielhaften Ausführungsformen erfolgt die Druckerhöhung durch Einbringen weiterer Flüssigkeit oder weiteren Gases in die Druckkammer des Druckbehälters.

In manchen beispielhaften Ausführungsformen erfolgt die Druckerhöhung durch ein Reduzieren des Volumens des Inneren oder der Druckkammer des Druckbehälters.

In gewissen beispielhaften Ausführungsformen umfasst das Verfahren das Abwarten, während der Körpergliedstumpf des Patienten weiterhin im Inneren des Druckbehälters ruht, bis die Gipsbinde zumindest teilweise getrocknet ist, alternativ ein Abwarten von 2 bis 10 min (Minuten), besonders bevorzugt zwischen 3 und 6 min, ganz besonders bevorzugt 5 min.

In einigen beispielhaften Ausführungsformen erfolgt dieses Verfahren am stehenden Patienten. Dies führt beim Fertigen des Abdrucks für einen Körpergliedstumpf der unteren Extremität zu besonders guten Ergebnissen.

In manchen beispielhaften Ausführungsformen ist das Fluid ein nicht komprimierbares Fluid, in manchen erfindungsgemäßen Ausführungsformen ist das Fluid eine Flüssigkeit, beispielsweise Wasser.

In einigen beispielhaften Ausführungsformen ist die Membran ein- und/oder ausstülpbar, vorzugsweise elastisch dehnbar. In anderen ist sie in einer Richtung elastisch, in einer hierzu insbesondere senkrechten Richtung hingegen nicht elastisch oder vergleichsweise oder maßgeblich weniger elastisch.

In manchen beispielhaften Ausführungsformen ist die Membran angeordnet, um bei Befüllen der Druckkammer mit einem Fluid durch dieses Fluid durch die Einführöffnung hindurch in ein Äußeres des Druckbehälters stülpbar zu sein.

In gewissen beispielhaften Ausführungsformen ist die Membran dehnbar, bleibt aber ähnlich einem Finger eines Gummihandschuhs oder einem Luftballon im gedehnten Zustand bis auf eine Öffnung geschlossen.

In einigen beispielhaften Ausführungsformen ist die Membran am Druckbehälter angeordnet, um diesen stirnseitig abzudichten.

In manchen beispielhaften Ausführungsformen weist die Vorrichtung in einem Ringraum oder zylindrischen Raum zwischen der, vorzugsweise elastisch dehnbaren, Membran und der Wandung des Druckbehälters keine Mündung eines Anschlussschlauchs auf.

In einigen beispielhaften Ausführungsformen weist die Vorrichtung einen Befestigungsring oder Stumpfdichtring auf, welcher beispielsweise wenigstens zwei Halb- oder Teilschalen (kurz: Schalen) aufweist oder hieraus besteht. Der Befestigungsring ist dem Druckbehälter stirnseitig auflegbar (und beispielsweise mittels geeigneter Verbindungselemente, wie rein exemplarisch Schrauben, mit diesem verbindbar), unter Begrenzung der Einführöffnung für den Stumpf. Der Befestigungsring verhindert ein Auftreiben der Membran beim Gebrauch der Vorrichtung.

In einigen beispielhaften Ausführungsformen weist die Vorrichtung wenigstens eine, vorzugsweise zwei oder mehr Kameras, Bildaufnahmesysteme, Oberflächenscanner, Magnet- oder Laserscanner, 3D-Scanner, Infrarotscanner oder andersartige Scanner, Ultraschalleinrichtungen oder andere Einrichtungen auf, welche geeignet und/oder konfiguriert sind, den Körpergliedstumpf, welcher in der Membran steckt und mit Druck beaufschlagt wird, zu erfassen oder zu vermessen und/oder um dessen Volumen und/oder Geometrie (z. B. Länge, Breite, Oberfläche, Außenkontur, Radien, Krümmungen, Dellen, Kanten, Winkel und dergleichen) zu bestimmen und/oder zum Abtasten der Oberfläche des Körpergliedstumpfs. Basierend auf diesen Messungen, Erfassungen, Abtastungen und dergleichen kann es möglich sein, einen Schaft für den Körpergliedstumpfs zu fertigen, ohne hierzu mittels der erfindungsgemäßen Vorrichtung einen Gipsabdruck gefertigt zu haben. Der Patient muss bei der Erfassung der Maße seines Körpergliedstumpfs noch nicht einmal eine Gipsbinde tragen. Es bedarf bei dieser Vorgehensweise vorteilhafterweise keines Fertigens eines Gipsabdrucks. Das Erfassen oder Vermessen ist in gewissen erfindungsgemäßen Ausführungsformen ein Abtasten und/oder ein Oberflächen-Abtasten des Stumpfs.

Dabei ist es in bestimmten Ausführungsformen vorgesehen, Vorrichtungen für eine rein äußerliche Abtastung oder Vermessung oder Bildgebung vorzusehen. Diese können optional keine 3-dimensionale Bildgebung erlauben und sind daher wesentlich weniger aufwendiger in der Handhabung und günstiger in der Anschaffung.

In anderen beispielhaften Ausführungsformen ist die Einrichtung zum Messen, Erfassen oder Abtasten des Körpergliedstumpfs ausgewählt aus der Gruppe, welche aus einem Computertomographen, einem Magnet-Resonanz-Tomographen und einer Ultraschallabtasteinrichtung besteht.

Die Einrichtung zum Messen, Erfassen oder Abtasten des Körpergliedstumpfs kann konfiguriert sein zur Gewinnung einer dreidimensionalen Darstellung des Körpergliedstumpfs oder eines Abschnitts hiervon.

Die vorgenannten Einrichtungen zum Messen, Erfassen oder Abtasten des Körpergliedstumpfs wie Kameras, Bildaufnahmesysteme, Scanner, Ultraschalleinrichtungen oder andere Einrichtungen können über den Umfang des Druckbehälters oder der Druckkammer verteilt vorgesehen sein, in identischen oder voneinander verschiedenen Abständen am Druckbehälter oder an seinem Umfang verteilt sein. Sie können in die Wandung des Druckbehälters integriert sein. Sie können relativ zur Wandung des Druckbehälters bewegbar vorgesehen sein, etwa indem sie sich um den Druckbehälter oder in dessen Inneren drehen können, entlang eines Umfangs und/oder entlang einer Längsrichtung des Druckbehälters.

In bestimmten beispielhaften Ausführungsformen weist die Vorrichtung eine rotierbare Vorrichtung auf, welche vorgenannte Einrichtungen zum Messen, Erfassen oder Abtasten des Körpergliedstumpfs tragen und rotierend bewegen kann.

Zu den vorgenannten Einrichtungen zum Messen, Erfassen oder Abtasten des Körpergliedstumpfs kann auch eine Lichtquelle zählen. So können beispielsweise eine Lichtquelle, welche auf den Körpergliedstumpf gerichtet ist und diesen beleuchtet, und eine Kamera, in vorzugsweise zueinander festem Abstand (z. B. 10 bis 20 cm, beispielsweise 15 cm), gemeinsam um dem Körpergliedstumpf rotiert werden.

In einigen beispielhaften Ausführungsformen weist die Vorrichtung wenigstens eine Einrichtung auf, welche konfiguriert ist zum Erstellen oder Errechnen eines Datenmodells des Körpergliedstumpfs oder eines Datenmodells des zu fertigenden Schafts für den vermessenen oder abgetasteten Körpergliedstumpf.

Das Datenmodell, hierin auch als Modell bezeichnet, ist vorzugsweise dreidimensional. Es ist vorzugsweise stetig.

In einigen beispielhaften Ausführungsformen weist die Vorrichtung eine Formgebungs-Vorrichtung auf oder ist hiermit in Signalverbindung verbunden. Die Formgebungs-Vorrichtung ist eingerichtet und/oder konfiguriert zum Herstellen des Prothesenschafts auf der Basis des Datenmodells des Körpergliedstumpfs oder des Datenmodells des zu fertigenden Schafts für den Körpergliedstumpf.

In einigen beispielhaften Ausführungsformen ist die Formgebungs-Vorrichtung eine CNC-Fräsvorrichtung, eine Rapid-Prototyping-Vorrichtung oder ein 3D-Drucker (kurz für: dreidimensionaler Drucker).

Die Membran hat eine Oberseite und eine Unterseite. Im Gebrauch der erfindungsgemäßen Vorrichtung ist die Oberseite dem Körpergliedstumpf zugewandt, die Unterseite begrenzt die Druckkammer. In bestimmten erfindungsgemäßen, beispielhaften Ausführungsformen weist die Membran wenigstens auf ihrer Unterseite eine Markierung auf, welche durch die Einrichtung zum Messen, Erfassen oder Abtasten des Körpergliedstumpfs als solche erkennbar oder identifizierbar ist. Die Markierung kann bei der Berechnung des Volumens oder der Oberfläche des Körpergliedstumpf oder des Gipsabdrucks als Orientierung dienen.

Diese Markierung kann eine optische und/oder haptische Markierung sein. Sie kann eine Erhabenheit, ein Kontrast, eine Farbmarkierung, eine Codierung, ein Farbmuster, ein Barcode oder dergleichen sein. Sie kann symmetrisch oder unsymmetrisch sein. Sie ist vorzugsweise vom Hersteller auf die Membran aufgebracht oder in diese integriert. Sie dient dazu, von einer der Einrichtungen der Vorrichtung erkannt und/oder ausgewertet zu sein. Die hierzu verwendete Software kann programmiert sein, die Markierung zu identifizieren und/oder ihre Lage im Raum auszuwerten.

In manchen beispielhaften Ausführungsformen weist die Vorrichtung keine aus dem Druckbehälter herausragende Schubstange auf, die am inneren Ende eine koaxiale Antikompressionstasse mit Ringwand aufweist.

In einigen beispielhaften Ausführungsformen weist die Vorrichtung keinen Schlitten auf, auf welchem der Druckbehälter montiert wäre.

In manchen beispielhaften Ausführungsformen weist die Vorrichtung keine elektrisch, hydraulisch und/oder hydraulisch betriebene oder betreibbare Einrichtung zum Erzeugen von Druck, insbesondere angeordnet zum Erzeugen von Druck auf die Membran, auf oder ist ein einer solchen nicht verbunden.

In einigen beispielhaften Ausführungsformen umfasst das Verfahren ein Platzieren des Patienten, welcher optional eine feuchte Gipsbinde um seinen Körpergliedstumpf trägt, an der Vorrichtung derart, dass dessen Körpergliedstumpf durch die Einführöffnung in das Innere des Druckbehälters eingeführt ist oder wird.

In einigen beispielhaften Ausführungsformen umfasst das Verfahren das Erstellen eines Modells basierend auf mittels der Einrichtung zum Erfassen, Abtasten oder Vermessen des Körpergliedstumpfs erfassten Daten.

Das Modell ist vorzugsweise ein stetiges Modell. Es ist vorzugsweise ein dreidimensionales Modell.

In einigen beispielhaften Ausführungsformen erfolgt das Abtasten mittels Ultraschall.

In manchen beispielhaften Ausführungsformen umfasst das Abtasten ein 3D-Scannen.

In einigen beispielhaften Ausführungsformen umfasst das Verfahren das Herstellen des Prothesenschafts auf der Basis des Modells. Es bedient sich hierzu eines Formgebungsverfahrens.

In manchen beispielhaften Ausführungsformen umfasst das Verfahren kein Bestimmen und/oder kein Verwenden eines Wertes des Gewichts des Patienten.

In gewissen beispielhaften Ausführungsformen umfasst das Verfahren kein Erstellen und/oder kein Berechnen von Querschnitten durch den Körpergliedstumpf oder von Schnittbilddaten.

In manchen beispielhaften Ausführungsformen umfasst das Verfahren kein Vorgeben oder Berücksichtigen einer Ziel-Kompression.

In einigen beispielhaften Ausführungsformen sieht das Verfahren nicht vor, den distalen Stumpf mit einer Kappe zu bedecken.

In manchen beispielhaften Ausführungsformen weist das Verfahren die folgenden Schritte des Einführens und des Erhöhens des Drucks auf, wobei das Erhöhen nach dem Einführen erfolgt. Das Einführen entspricht dem Einführen des optional mit einer feuchten Gipsbinde umwickelten Körpergliedstumpfs (oder des distalen Teils hiervon) in die Membran oder die Fluidkammer oder Druckkammer, beispielsweise derart, dass der Körpergliedstumpf zumindest in Abschnitten hiervon um seinen gesamten Umfang herum von der Membran umgeben ist. Das anschließend erfolgende Erhöhen des in der Fluidkammer oder Druckkammer herrschenden Drucks durch Einbringen weiterer Flüssigkeit oder weiteren Gases in die Fluidkammer oder Druckkammer dient dazu, den zum Anfertigen des Gipsabdrucks, wie dies hierin beschrieben ist, erforderlichen oder vorbestimmten Fluiddruck zu erzielen oder zu erzeugen. Da der endgültige Druck erst erzeugt wird, nachdem der Patient seinen Körpergliedstumpf bei vergleichsweise geringem Druck in der Fluidkammer oder Druckkammer ausreichend oder wie gewünscht tief in die Membran eingeführt hat, erfolgt vorteilhafter Weise kein Richtung cranial oder proximal erfolgendes Massieren oder Verschieben von Weichteilgewebe des Körpergliedstumpfs. Letzteres tritt nach Beobachtung des Erfinders dann auf, wenn der Körpergliedstumpf bei endgültigem Druck in die Membran eingeführt wird und kann nachteilig sein, da durch Reibungskräfte, welche beim Einsteigen in die Membran der Bewegungsrichtung entgegen wirken können, das Verschieben von Weichteilen beim Vorgang des Erstellen des Gipsabdrucks oder des Vermessens des Körpergliedstumpf zu Ergebnissen, Maßen oder Erkenntnissen führen kann, welche nicht mit den Gegebenheiten oder Zuständen des Körpergliedstumpfs später im - nach dem Muster des Gipsabdrucks erstellten - Schaft übereinstimmen. Der Schaft kann, anders ausgedrückt, nicht-optimal ausfallen. Das Einführen bei niedrigem Druck oder Atmosphärendruck führt nicht zu einem Verschieben von Weichteilen. Dies ändert sich auch dann nicht mehr, wenn der Fluiddruck später um die Membran und den Körpergliedstumpf durch Einbringen von Fluid aufgebracht wird. Die Reihenfolge "erst Einführen, dann Druckaufbauen" kann daher vorteilhaft zu korrekteren Gipsabdrücken führen.

In gewissen beispielhaften Ausführungsformen sieht das Verfahren ferner das Belasten oder maximale Belasten des Körpergliedstumpfs durch den Patienten und/oder das Beschweren des Patienten mit Gewicht vor. Dabei schließt sich dieser Schritt vorzugsweise an die Schritte des Einführens und des Erhöhens des Drucks an. Das Belasten des Körpergliedstumpfs erst nach Aufbauen des erforderlichen oder gewünschten Drucks oder Zieldrucks kann weiter ein Verbleiben des Weichteilgewebes an Ort und Stelle begünstigen, mit den vorstehend genannten Vorteilen.

In einigen beispielhaften Ausführungsformen ist die Membran nicht schlauchförmig, d. h. nicht an beiden Enden hiervon offen.

In gewissen beispielhaften Ausführungsformen ist die Membran aus fluiddichtem, insbesondere wasserdichtem Material. Sie kann aus Silikon sein oder Silikon aufweisen. Sie kann aus faserverstärktem Silikon sein oder solches aufweisen. Statt Silikon kann ein Co-Polymer oder ein Gummi vorgesehen sein.

In einigen beispielhaften Ausführungsformen wird keine Druckluft auf die Gipsbinde aufgebracht.

In manchen beispielhaften Ausführungsformen weist die medizinische Vorrichtung wenigstens einen Drucksensor, eine Druckmessvorrichtung oder eine andere Vorrichtung zum Ermitteln oder Feststellen von Druck auf oder steht hiermit in Signalverbindung. Der Drucksensor oder dergleichen kann dabei angeordnet, konfiguriert und/oder eingebunden sein, um mit seiner Hilfe einen Druck zu messen, welcher auf die Membran, in der Druckkammer, auf einem Abschnitt der Oberfläche des von der Membran im Gebrauch der medizinischen Vorrichtung bedeckten Körpergliedstumpfs und/oder zwischen Membran und Körpergliedstumpf herrscht.

In einigen beispielhaften Ausführungsformen ist der Sensor ein Drucksensor, ein Array aus mehreren Drucksensoren (wobei die von ihnen ermittelten Druckwerte z. B. gemittelt werden können), oder dergleichen. Der Sensor kann kabellos, kabelgebunden, usw. sein. Er kann als RFID oder Sender ausgestaltet sein.

In manchen beispielhaften Ausführungsformen weist die medizinische Vorrichtung eine Anzeigevorrichtung auf, ist hiermit verbunden und/oder wird hiermit verwendet, welche konfiguriert ist, um vom Drucksensor oder von der Druckmessvorrichtung oder von einer Auswertevorrichtung ein Signal zu empfangen und anzuzeigen, welches dem ermittelten Druck oder einem Drucksignal entspricht.

In einigen beispielhaften Ausführungsformen weist die medizinische Vorrichtung eine Ermittlungsvorrichtung auf oder ist hiermit verbunden und/oder wird hiermit verwendet. Die Ermittlungsvorrichtung ist konfiguriert, um basierend oder unter Einbezug eines von einer Druckmessvorrichtung, z. B. dem Drucksensor, ermittelten Druckwerts das Gewicht zu ermitteln, mit welchem der Patienten, dessen Körpergliedstumpf in die medizinische Vorrichtung eingeführt ist, beschwert werden muss, damit der mittels der Druckmessevorrichtung gemessene Druck in einem vorbestimmten Zielwertebereich liegt oder sich in diesen bewegt.

Ein vorbestimmter Zielwertebereich für den Fluiddruck oder Druck kann zwischen 250 mbar und 550 mbar, bevorzugt zwischen 350 mbar und 450 mbar und ganz bevorzugt zwischen 380 und 420 mbar liegen. Diese Werte haben zu besonders passenden Schäften geführt, welche ausgehend von dem wie hierin beschrieben erzeugten Gipsmodell erzeugt wurden.

In einigen beispielhaften Ausführungsformen weist die medizinische Vorrichtung eine Anzeigevorrichtung auf, welche konfiguriert ist, das Gewicht anzuzeigen, mit welchem der Patient, dessen Körpergliedstumpf in die medizinische Vorrichtung eingeführt ist, beschwert werden muss, damit der mittels der Druckmessvorrichtung gemessene Druck in einem vorbestimmten Zielwertebereich liegt.

In einigen beispielhaften Ausführungsformen wird das Gewicht, um welches der Patient beschwert werden muss, beispielsweise basierend auf zumindest dem gemessenen Druck und/oder beispielsweise anhand einer Formel (in welche beispielsweise die Größe des Umfangs des Körpergliedstumpfs, das Gewicht des Patienten, die Eindringtiefe, um welche der Körpergliedstumpf durch die Einführöffnung hindurch in die medizinische Vorrichtung geführt ist), anhand einer Nachschlagetabelle ermittelt wird. Eine solche Formel könnte $P = \left(m*9,81*4*Π\right) / {U}^{2}$ lauten, mit
P als Zieldruck, m als Masse des Patienten, Π als Kreiszahl und U als Umfang des Körpergliedstumpfs im Bereich der Eintrittsöffnung. Diese Formel führt in erster Näherung zu hinreichend guten Ergebnissen. Sie kann jedoch modifiziert werden. Dabei können auch andere der hierin genannten Größen verwendet werden.

In einigen beispielhaften Ausführungsformen umfasst das Verfahren zum Anpassen eines Gipsabdrucks an einen Körpergliedstumpf oder zum Vermessen des Körpergliedstumpfs eines Patienten die Schritte:
- Bereitstellen einer medizinischen Vorrichtung, mit einer Membran und einer Druckkammer, vorzugsweise wie hierin beschrieben;
- optionales Überziehen eines Verbundstrumpfes über den Körpergliedstumpf, wobei der Verbundstrumpf auf der zum Körpergliedstumpf hin orientierten Seite glatt (oder glatter als auch der nach außen hin orientierten Seite) und auf der nach außen hin orientierten Seite rau oder oberflächenstrukturiert (oder rauer oder oberflächenstrukturierter als auch der nach zum Körpergliedstumpf hin orientierten Seite) ist, wobei die nach außen hin orientierte Seite kraftschlüssig und/oder formschlüssig mit einer Gipsbinde verbunden werden kann;
- Einführen des optional mit einer feuchten Gipsbinde umwickelten Körpergliedstumpfs in die Membran;
- Ermitteln des Drucks, welcher auf die Membran, in der Druckkammer, auf einem Abschnitt der Oberfläche des von der Membran bedeckten Körpergliedstumpfs und/oder zwischen Membran und Körpergliedstumpf herrscht;
- Ermitteln eines Gewichts, um welches der Patient beschwert werden oder entlastet (etwa durch Aufstützen) muss, damit der mittels der Druckmessvorrichtung gemessene oder messbare Druck in einem vorbestimmten Zielwertebereich liegt oder in diesen wandert.

In manchen beispielhaften Ausführungsformen kann der Verbundstrumpf ein Liner sein. Ein Liner kann ein Textilstrumpf sein, der in der Praxis regelmäßig über den Körpergliedstumpf gezogen wird, wobei dies in der Praxis bislang jedoch so erfolgt, dass die zum Körpergliedstumpf hin orientierte Seite weniger glatt als die nach außen hin orientierte Seite ist. Über den Liner kann der Prothesenschaft gezogen werden.

In gewissen beispielhaften Ausführungsformen wird der Verbundstrumpf über einen Liner, der vorzugsweise direkt über den Körpergliedstumpf übergezogen wird und diesem anliegt, zusätzlich übergezogen. Der Liner weist regelmäßig auf seiner dem Körpergliedstumpf zugewandten Seite eine weiche und strukturierte Oberfläche auf, um den Tragekomfort weiterer Komponenten, die über den Liner gezogen werden (insbesondere den Prothesenschaft) zu erhöhen. Weiterhin kann der Liner auf seiner Außenseite eine glatte Oberfläche aufweisen, um eine hohe Haftkraft oder Reibkraft zu dem Prothesenschaft zu erreichen. Der Verbundstrumpf kann, insbesondere in Form eines zusätzlichen Liners, auf den ersten Liner aufgezogen werden. Die rauere oder strukturiertere Oberfläche der Außenseite des Verbundstrumpfs kann vorteilhaft einen Verbund und Kraftschluss mit einer feuchten Gipsbinde ermöglichen. Zudem kann sie vorteilhaft beim Trocknen der Gipsbinde mit dieser "verbacken" und so Teil des Gipsabdrucks werden. Der so mit dem Verbundstrumpf verbundene Gipsabdruck erhält auf diese Weise eine vergleichsweise glatte Innenfläche, welche den Aufwand für ein Nachbearbeiten der Oberflächenbeschaffenheit der Innenfläche des Gipsabdrucks verringern oder vermeiden helfen kann.

In einigen beispielhaften Ausführungsformen umfasst das Verfahren, dass der Patient mittels des ermittelten Gewichts beschwert wird.

In manchen beispielhaften Ausführungsformen umfassen die patientenbezogenen Daten wenigstens ein Element aus der Gruppe, welche besteht aus:
- Gewicht des Patienten;
- Umfang des Körpergliedstumpfs des Patienten;
- Umfang des Körpergliedstumpfs des Patienten im Bereich der Eintrittsöffnung;
- Gemittelter Umfang des Körpergliedstumpfs des Patienten, soweit in die Vorrichtung eingeführt oder einzuführen;
- Volumen des eingeführten oder einzuführenden Körpergliedstumpfs;
- Oberfläche des eingeführten oder einzuführenden Körpergliedstumpfs;
- Querschnittsfläche des Körpergliedstumpfs, insbesondere im Bereich der Einführöffnung;
- Kompressibilität des Körpergliedstumpfs;
- Länge, über welche der Körpergliedstumpf in die Vorrichtung eingeführt ist oder einzuführen ist.

Die Kompressibilität kann durch Messen einer Kraft oder eines Drucks bestimmt werden, welche aufgebracht oder welcher angelegt werden muss, um einen vorbestimmten Eindruck (Impression) in den Körpergliedstumpf zu bewirken.

In einigen beispielhaften Ausführungsformen weist die Bodenfläche der erfindungsgemäßen, medizinische Vorrichtung wenigstens abschnittsweise eine gekrümmte Form zum kippbaren, verdrehbaren und/oder verschiebbaren Bewegen der Vorrichtung auf einer Auflagefläche auf.

Eine gekrümmte Form kann beispielsweise eine konkave, nach innen zur Druckkammer hin, gekrümmte Form aufweisen. Eine Vorrichtung mit einer konkav gekrümmten Bodenfläche kann auf einem kugelförmigen Untergrund oder auf einem kugelförmigen oder zylinderförmigen Wälzkörper gedreht oder gekippt werden. Die Form der Unterlage kann gleichfalls parabolidförmig sein oder eine andere Form aufweisen. Die Bewegung der Vorrichtung auf der Unterlage kann eine Abrollbewegung oder Gleitbewegung sein.

Die gekrümmte Form kann alternativ eine konvexe, nach außen, weg von der Druckkammer gerichtete, gekrümmte Form aufweisen. Eine Vorrichtung mit einer konvex geformten Bodenfläche kann vorzugsweise auf einem ebenen Untergrund bewegt oder abgerollt werden. Die konvexe Form kann beispielsweise eine Halbkugelform, eine Kugelsegmentform, eine Paraboloidform eine Teilzylinderform oder eine andere gekrümmte, konvexe Form aufweisen.

Das System mit einer medizinischen Vorrichtung und wenigstens einer Auflagefläche kann in einigen erfindungsgemäßen Ausführungsformen während der Anfertigung eines Gipsabdrucks eines Körpergliedstumpfs relativ zur Auflagefläche gekippt, verdreht und/oder verschoben werden. Die Bewegung der Vorrichtung relativ zur Auflagefläche kann eine Abrollbewegung, eine Gleitbewegung oder eine kombinierte Bewegung aus einem Abrollen und einem Gleiten sein. Die Bewegung kann von dem Prothesenträger selbst veranlasst und durchgeführt, oder von ihm unterstützt werden. Mittels dieser Bewegung kann vorteilhaft während des Gipsabdrucks nicht nur eine statische Belastung, sondern auch eine muskuläre und/oder knöcherne Verschiebung und Belastung, wie sie später beim Tragen der Prothese in der Bewegung auftritt, abgeformt werden. Diese zusätzliche, dynamische Belastung kann die Passgenauigkeit, den Tragekomfort, das Vermeiden möglicher Druckstellen zwischen dem Körpergliedstumpf und der Prothese, verbessern oder erhöhen. Das Abdruckverfahren, wie es zuvor beschrieben ist, kann bei der Anwendung des erfindungsgemäßen Systems grundsätzlich gleich sein.

In manchen beispielhaften Ausführungsformen weist die Auflagefläche eine ebene oder gekrümmte Oberflächenform auf. Eine gekrümmte Oberflächenform kann eine gewölbte, eine halbkugelförmige, eine kugelsegmentförmige (oder kugelabschnittförmige), eine paraboloidförmige oder eine andere Form sein oder aufweisen.

In gewissen beispielhaften Ausführungsformen bildet die Auflagefläche einen Wälzkörper aus. Anders ausgedrückt, kann die Auflagefläche auf der Oberfläche eines Wälzkörpers abgebildet werden. Ein Wälzkörper kann beispielsweise eine Kugel, eine Walze, ein Ellipsoid sein.

In bestimmten beispielhaften Ausführungsformen ist die Auflagefläche mittels einer Führungsvorrichtung und/oder mittels Wälzkörpern relativ zur Vorrichtung und/oder relativ zu einem festen Untergrund bewegbar. Eine Führungsvorrichtung kann ein Schlitten sein. Der Schlitten kann Rollen oder Räder zu seiner Bewegung auf einem Untergrund haben. Er kann Rollen haben, die eine Bewegung in nur einer Richtung erlauben (ähnlich einer Ratsche).

Der Schlitten kann optional mittels Schienen entlang eines vorgegebenen Wegs oder Pfads geführt werden kann. Ein vorgegebener Weg kann daraufhin optimiert oder vorteilhaft sein, dass eine dynamische Belastung des Körpergliedstumpfs während eines Gipsabdruckes hinsichtlich einer muskulären und/oder knöchernen Verschiebung beim Gehen mittels des vorgegebenen Wegs der Führungsvorrichtung simuliert wird.

In manchen beispielhaften Ausführungsformen ist die Auflagefläche mit der zweiten Stirnseite und/oder mit der Bodenfläche der Vorrichtung, vorzugsweise wieder lösbar, verbunden und/oder verbindbar. Die mit der Vorrichtung verbundene Auflagefläche kann als Stützeinrichtung bezeichnet werden.

In gewissen , beispielhaften Ausführungsformen weist das erfindungsgemäße System wenigstes eine Sicherungsmanschette auf, welche mit dem Druckbehälter verbunden und/oder verbindbar ist, um den Körpergliedstumpf bei der Anfertigung eines Gipsabdrucks oder eines Datenmodells in der Vorrichtung zu fixieren. Diese Fixierung kann den Körpergliedstumpf während des Bewegens und während des Gipsabdrucks gegen ein Herausrutschen oder Verschieben in der Druckkammer in wenigstens einen Raumrichtung sichern.

In einigen beispielhaften Ausführungsformen ist die Sicherungsmanschette druckdicht ausgeführt. Der Begriff "druckdicht" ist im Rahmen der Messgenauigkeit eines Druckabfalls während eines Gipsabdrucks zu verstehen. Vorzugsweise wird ein Unterdruck in dem Zwischenraum zwischen der Sicherungsmanschette und dem Körpergliedstumpf angelegt oder aufgebaut, so dass der Körpergliedstumpf während der Bewegung auf einer Auflagefläche stabilisiert wird und nicht aus der Druckkammer herausrutschen kann. Eine Vorrichtung zum Erzeugen des Unterdrucks kann, z. B. als Luftpumpe, vorgesehen sein.

In einigen beispielhaften Ausführungsformen weist die erfindungsgemäße medizinische Vorrichtung eine Entlastungseinrichtung zum Druckentlasten des distalen Bereichs des Körpergliedstumpfs auf. Die Entlastungseinrichtung ist vorzugsweise innerhalb des Druckbehälters, auf der Membran, außerhalb der Druckkammer und/oder innerhalb eines von der Membran gebildeten Raums angeordnet oder zu einer solchen Anordnung bestimmt. Die Entlastungseinrichtung kann vorteilhaft empfindliche Weichteile im Bereich des distalen Endes des Körpergliedstumpfs vor einem durch das Fluid aufgebauten Druck schützen.

In manchen beispielhaften Ausführungsformen ist die Entlastungseinrichtung ein Hohlgefäß mit wenigstens einer Öffnung oder einer Vertiefung. Vorzugsweise ist die Öffnung oder Vertiefung zum distalen Bereich des Körpergliedstumpfs hin gerichtet oder ist hierzu bestimmt.

In einigen beispielhaften Ausführungsformen weist die Öffnung oder Vertiefung eine Deckschicht auf oder ist mit einer solchen bedeckt. Alternativ oder ergänzend ist das Hohlgefäß mit einem kompressiblen Material wenigstens bereichsweise gefüllt.

Das Hohlgefäß kann eine Becherform aufweisen. Es kann optional zylindrische Wände, und/oder oder einen konischen Querschnittsverlauf aufweisen.

Die konische Form mit sich verjüngendem Querschnitt kann an der Öffnung oder am Öffnungsquerschnitt einen größeren Durchmesser (bei einer exemplarischen runden und kreisförmigen Querschnittsform) zum Einbringen des Körpergliedstumpfs aufweisen. Der Durchmesser kann sich zum Boden des Hohlgefäßes hin verjüngen.

Die Öffnung oder Vertiefung ist vorzugsweise zum distalen Bereich des Körpergliedstumpfs oder zum oberen Stirnende der Vorrichtung hin offen und kann somit diesen empfindlichen Bereich des Körpergliedstumpfs vor einer möglichen Druckbelastung schützen. Der Körpergliedstumpf, der beim Einführen mit einer feuchten Gipsbinde umwickelt sein kann, kommt in distalen Abschnitten hiervon je nach Form der Öffnung nicht mit der Membran in Kontakt. Hierzu kann der distale Bereich des Körpergliedstumpfs auf den Seitenwänden, dem oberen Rand oder dem oberen Umfang der exemplarisch als Hohlgefäß ausgestalteten Entlastungseinrichtung ruhen.

Die Entlastungsvorrichtung wird vorzugsweise innerhalb des Druckbehälters, auf der Membran, außerhalb der Druckkammer angeordnet und ist hierzu bestimmt. Die Entlastungseinrichtung liegt vorzugsweise im Trocknen, also ohne direkten Kontakt zum Fluid.

In gewissen beispielhaften Ausführungsformen weist die Öffnung der Entlastungseinrichtung eine Membran auf oder ist mit dieser bedeckt. Die Membran kann ein inneres Material der Entlastungseinrichtung, die als Hohlgefäß ausgeführt sein kann, nach außen abdecken oder schützen. Ein inneres Material, mit das Hohlgefäß vollständig oder teilweise gefüllt sein kann, ist beispielsweise ein weiches, kompressibles oder komprimierbares Material. Eine Entlastungseinrichtung, die als Hohlgefäß mit einem weichen Material gefüllt und mit einer Membran abgedeckt ist, kann vorteilhaft als Druckentlastungseinrichtung für das distale Ende des Körpergliedstumpfs genutzt werden. Dadurch kann ein direkter Kontakt des distalen Endes des Körpergliedstumpfs mit der Membran der Druckkammer vermieden werden.

In bestimmten beispielhaften Ausführungsformen ist die Entlastungseinrichtung in die Membran integriert und/oder integraler Bestandteil der Membran. Beispielsweise kann die Membran im distalen Bereich des Körpergliedstumpfs zweilagig oder doppelwandig ausgeführt sein. Zwischen einer inneren und einer äußeren Schicht der doppelwandigen Membran kann die Entlastungseinrichtung eingebracht oder integriert sein.

Die Entlastungseinrichtung kann in die doppelwandige Membran eingebracht oder eingeschoben sein, wobei sich die Entlastungseinrichtung innerhalb der doppelwandigen Membran bewegen oder ausrichten kann.

Die Entlastungseinrichtung kann gleichfalls mit einer oder beiden Schichten der doppelwandigen Membran stoffschlüssig verbunden sein. Ein Stoffschluss kann eine Klebung, eine Schweißung oder eine andere Art von Stoffschluss sein.

Eine in die Membran integrierte Entlastungseinrichtung kann die Anfertigung eines Gipsabdrucks oder eines Datenmodells eines Körpergliedstumpfs, insbesondere eines Unterschenkelstumpfs, mithilfe einer erfindungsgemäßen medizinischen Vorrichtung vereinfachen. Eine separate Ausrichtung der Entlastungseinrichtung in der Membran kann vorteilhaft entfallen. Mit einer in die Membran integrierten Entlastungseinrichtung ist die Entlastungseinrichtung nicht unabhängig von der Membran positionierbar und somit nicht beliebig verschiebbar, sondern nur in vorbestimmten Bereichen oder Grenzen.

In manchen beispielhaften Ausführungsformen ist die Entlastungseinrichtung ein Abschnitt der Membran. Die Membran bildet im distalen Bereich des Körpergliedstumpfs die Entlastungseinrichtung aus. Die Verbindung des Membranbereichs mit der Entlastungseinrichtung und dem übrigen, elastischen Membranbereichs kann eine stoffschlüssige Verbindung, beispielsweise eine Klebung oder eine Verschweißung.

In bestimmten beispielhaften Ausführungsformen weist die Vorrichtung einen Adapter zum Verbinden mit dem Druckbehälter auf, wobei der Adapter zum lösbaren Verbinden mit dem Körpergliedstumpf vorbereitet ist. Der Adapter weist wenigstens einen Haftstrumpf sowie eine Entlastungseinrichtung und/oder eine Einrichtung zum Übertragen von Zugkräften, welche mit dem Haftstrumpf in Kraft- und/oder Formschluss zum Übertragen von Zugkräften verbunden ist, auf oder besteht hieraus.

In einigen beispielhaften Ausführungsformen ist die Einrichtung zum Übertragen von Zugkräften, welche mit dem Haftstrumpf in Kraft- und/oder Formschluss zum Übertragen von Zugkräften verbunden ist, ein Klettverschluss, ein integral mit dem Haftstrumpf verbundenes Material, beispielsweise ein Kunststoff oder ein Kompositmaterial, welches mit dem Haftstrumpf verschweißt oder verklebt ist, ein mit einem in den Haftstrumpf eingearbeiteten oder materialverstärkten Bereich zur Übertragung von Zugkräften, z. B. mehrlagig gewebte Schichten des Haftstrumpfes, oder jede andere geeignete Ausführungsform zum Übertragen von Zugkräften.

In manchen beispielhaften Ausführungsformen weist das Set wenigstens eine erfindungsgemäße medizinische Vorrichtung und wenigstens einen Strumpf zum Überziehen (ein Überziehen kann auch ein Überstülpen bedeuten) über den Körpergliedstumpf auf, wobei der mit dem Körpergliedstumpf verbundene Strumpf kraftschlüssig, insbesondere reibschlüssig, mit einer Oberfläche, insbesondere mit der Oberfläche der Membran, am oder im Druckbehälter verbunden ist.

In manchen beispielhaften Ausführungsformen weist der Strumpf an seiner Außenseite, also nicht auf der zum Körperstumpf hin orientierten Innenseite, eine reibschlüssige Oberfläche auf. Eine reibschlüssige Oberfläche ist beispielsweise eine raue Oberfläche. Die Oberfläche kann strukturiert sein. Die Oberfläche kann beschichtet sein und/oder chemisch oder physikalisch behandelt sein, um eine reibschlüssige Oberfläche zu erzielen. Insbesondere verhindert eine reibschlüssige Oberfläche ein, ohne zusätzlichem Kraftaufwand, Herausrutschen des Körpergliedstumpfs aus dem Strumpf. Dadurch kann vorteilhaft ein Gipsabdruck oder ein Datenmodell eines Körpergliedstumpfs, insbesondere eines Unterschenkelstumpfs, angefertigt werden, ohne dass der Patient unvorhergesehene oder ungewollte Bewegungen während des Gipsabdrucks oder bei der Erstellung eines Datenmodells verursacht. Durch derartige Bewegungen kann die Erstellung des Gipsabdrucks oder des Datenmodells negativ beeinflusst werden. Alternative Verfahren, um derartige unvorhergesehene oder ungewollte Bewegungen des Patienten zu vermeiden, etwa zusätzliche Gewichtsbelastungen des Patienten, können vorteilhaft mittels des erfindungsgemäßen Strumpfs vermieden werden.

In einigen beispielhaften Ausführungsformen weist die medizinische Vorrichtung einen im Inneren des Druckbehälters angeordneten Formkörper auf. Der Formköper ist zum Aufnehmen und/oder zum Stützen und/oder zur Druckentlastung des distalen Bereichs des Körpergliedstumpfes ausgestaltet und vorbereitet. Der Formkörper ist innerhalb des Druckbehälters und/oder innerhalb eines von der Membran gebildeten Raums, insbesondere Innenraums, in welchen auch der Körpergliedstumpf eingeführt wird, angeordnet. Die äußere Oberfläche des Formkörpers liegt vorzugsweise wenigstens abschnittsweise - direkt oder indirekt - reibschlüssig an der Oberfläche der Membran an.

In manchen beispielhaften Ausführungsformen ist der Formkörper schalenförmig ausgestaltet. Der Formkörper kann aus einem thermoplastischen Kunststoff herstellt sein oder einen solchen aufweisen. Ebenso kann der Formkörper aus einem anderen Material, beispielsweise aus einem faserverstärkten Verbundwerkstoff oder aus einem Metall hergestellt sein oder solche Materialien aufweisen.

In gewissen beispielhaften Ausführungsformen ist der Formkörper mehrteilig und/oder modulförmig aufgebaut. Die mehrteiligen und/oder modulförmigen Formkörperteile können formschlüssig und/oder kraftschlüssig zu einem Formkörper zusammengesetzt oder zusammengefügt oder verbunden werden. Wenigstens ein schalenförmiges Grundmodul oder Basismodul ist als Formkörper verwendbar. Das Grundmodul kann mit einem, zwei oder mehr Segmenten des mehrteiligen und/oder modulförmigen Formkörpers verbunden werden oder verbindbar sein.

In bestimmten beispielhaften Ausführungsformen umschließt der Formkörper ein kompressibles Material oder weist ein kompressibles Material auf. Das kompressible Material kann ein Schaumstoff sein. Das kompressible Material kann in den Formkörper beispielsweise eingelegt oder eingeklebt werden.

Die Einheit umfasst eine Druckbeaufschlagungs-Steuervorrichtung für die Druckkammer der erfindungsgemäßen medizinischen Vorrichtung und eine erfindungsgemäße medizinische Vorrichtung.

Die Druckbeaufschlagungs-Steuervorrichtung weist wenigstens ein Druckreservoir-Anschluss, ein Druckbegrenzungsventil und wenigstens einen Anschluss an die Druckkammer mit einem Regelventil zur Druckerhöhung und/oder einem Regelventil zur Druckverminderung in der Druckkammer auf.

Das Regelventil kann ein Steuerventil sein.

Der Druckreservoir-Anschluss ist in manchen erfindungsgemäßen, beispielhaften Ausführungsformen ein Anschluss an eine Druckquelle, insbesondere an eine Wasserleitung. Mittels des Druckreservoir-Anschlusses kann Fluid, insbesondere Wasser, aus einer Wasserleitung oder einem anderen Reservoir der Druckkammer zugeführt werden.

In gewissen beispielhaften Ausführungsformen begrenzt das Druckbegrenzungsventil den Druck aus der Fluidzuleitung an dem Druckreservoir-Anschluss auf einen Wert zwischen 0,7 bar und 0,9 bar, insbesondere auf max. 0,8 bar.

In bestimmten beispielhaften Ausführungsformen weist die Einheit, insbesondere die Druckbeaufschlagungs-Steuervorrichtung, eine Druckanzeige auf. Eine Druckanzeige kann ein Manometer sein. Mittels der Druckanzeige kann der Druck in der Druckkammer überprüft und/oder überwacht werden. Die Druckanzeige kann eine Redundanz-Funktion oder eine Sicherheitsfunktion gegenüber einer weiteren Druckanzeige haben, die direkt an der Druckkammer angeordnet sein kann.

In einigen beispielhaften Ausführungsformen weist die Einheit, insbesondere die Druckbeaufschlagungs-Steuervorrichtung, eine Not-Aus-Einrichtung auf. Mittels der Not-Aus-Einrichtung kann der Druckreservoir-Anschluss geschlossen oder blockiert werden. Beispielsweise können unvorhergesehene Druckspitzen oder ein plötzlicher Druckanstieg oder Druckabfall in der Zuleitung zu dem Druckreservoir-Anschluss auftreten, der aus Sicherheitsgründen, insbesondere zum Schutz des Körpergliedstumpfs, schnell unterbrochen werden soll. Dies kann mit einem Betätigen der Not-Aus-Einrichtung vorteilhaft einfach und schnell erfolgen.

In manchen beispielhaften Ausführungsformen weist die Einheit, insbesondere Druckbeaufschlagungs-Steuervorrichtung, eine Saug-Düse auf. Die Saug-Düse kann stromab des Regelventils zur Druckminderung des Drucks in der Druckkammer angeordnet sein. An der Saug-Düse kann ein Unterdruck anliegen oder mittels der Saug-Düse erzeugt werden. Beispielsweise kann eine Saugpumpe oder Unterdruckpumpe an die Saug-Düse angeschlossen sein. Ebenso kann eine sogenannte Venturi-Düse oder ein Venturi-Rohr an der Saug-Düse angeschlossen sein. Die Saug-Düse kann eine Venturi-Düse oder ein Venturi-Rohr sein. Mittels der Saug-Düse kann ein Fluid, insbesondere Wasser, aus der Druckkammer der erfindungsgemäßen Vorrichtung abgesaugt oder leergesaugt werden.

In gewissen beispielhaften Ausführungsformen weist die Einheit, insbesondere die Druckbeaufschlagungs-Steuervorrichtung, keine elektrischen Komponenten auf. Die Druckbeaufschlagungs-Steuervorrichtung kann vorzugsweise keinen Stromanschluss aufweisen, insbesondere keine elektrischen Komponenten und/oder keinen Stromanschluss, welche zur Druckveränderung innerhalb der mit der Druckbeaufschlagungs-Steuervorrichtung verbundenen Druckkammer genutzt werden würde.

Einige oder alle erfindungsgemäßen Ausführungsformen können einen oder mehrere der oben oder im Folgenden genannten Vorteile aufweisen.

Es liegt auf der Hand, dass der Prothesenschaft dem Patienten aufgrund der zurück gewonnenen Mobilität insbesondere im Stehen und im Gehen von Nutzen ist. Im Stehen und im Gehen, wenn der Schaft bestimmungsgemäß belastet wird, muss der Schaft daher im besonderen Maße bequem sitzen. Die bislang bekannt gewordenen Verfahren zum Fertigen des Gipsabdrucks oder zum Vermessen des Stumpfs werden dem allerdings nicht auf optimale Weise gerecht, da sie die Weichteilverschiebungen im Stumpf etwa relativ zum knöchernen Anteil des Stumpfs, wie sie bei Belastung des Schafts später auftreten, mangels Belastung des Stumpfs während des Vermessens oder des Gipsens nicht berücksichtigen können. Das Ergebnis kann zu einer Ungenauigkeit beim Fertigen des Schaftes führen, welche selbst bei Abnehmen von Körpermaßen in genau definierten Höhen und Bereichen im cm-Bereich liegt. Dies ist bei Verwendung der erfindungsgemäßen Vorrichtung bei stehendem Patienten vorteilhaft nicht der Fall - der Stumpf unterliegt während des Vermessens oder des Gipsens nahezu identischen Belastungen und Weichteilverlagerungen wie in der späteren Belastung im Schaft.

Mittels der Erfindung ist es bei während des Anfertigens des Gipsabdrucks oder des Vermessens stehendem und den Stumpf belastendem Patienten vorteilhaft erstmals möglich, zuverlässig und vor allem auch reproduzierbar einen Gipsabdruck des Stumpfs des Patienten zu fertigen oder den Stumpf zu vermessen um ein Datenmodell, welches den Stumpf widerspiegelt, zu erhalten.

Damit erlaubt es die vorliegende Erfindung, einen Gipsabdruck zu fertigen oder ein Stumpfmodell zu erhalten als Basis für einen Schaft, welcher dem Träger vor allem im Gehen und Stehen bequem passt. Dabei ist weniger handwerkliches Geschick als bisher erforderlich.

Somit kann die vorliegende Erfindung das Herstellen von Schäften für Prothesen der oberen und unteren Extremitäten des Menschen auf eine objektive Art, basierend auf direkt gewonnenen Messdaten ermöglichen. Die Erfindung ermöglicht somit die Herstellung eines gut angepassten Prothesenschafts, wobei jedoch die rein subjektiven und manuell ausgeführten kostenintensiven Tätigkeiten, die bei den Verfahren des Stands der Technik nötig sind, vermieden werden können.

Dabei ist es vorteilhaft möglich und in bestimmten Ausführungsformen vorgesehen, eine rein äußerliche Abtastung oder Vermessung oder Bildgebung einzusetzen. Diese ist wesentlich weniger aufwendiger, sowohl was die Auswertung ihrer Messungen als auch die Kosten für die Anschaffung der erforderlichen Einrichtungen betrifft.

Mittels der Erfindung ist es bei während des Anfertigens des Gipsabdrucks stehendem und dabei den Stumpf realitätsnah belastendem Patienten vorteilhaft auch erstmals möglich, bereits beim Anfertigen des Gipsabdrucks oder beim Vermessen eine Rückmeldung des Patienten über drückende oder schmerzende Stellen zu erhalten. Die spätere Tragesituation des Schafts wird vom Patienten bei Verwenden der erfindungsgemäßen Vorrichtung im Stehen sozusagen bereits während des Erstellens des Gipsabdrucks oder während der Vermessung "voraus"empfunden; unbefriedigend passende Abschnitte des späteren Schafts werden so frühzeitig erkannt bzw. antizipiert. Änderungswünsche oder Polsterungswünsche können so bereits beim Fertigen des Gipsabdrucks beim Vermessen vom Patienten angemeldet und vom Orthopädietechniker vorbereitet werden. Dies kann über die Zeitspanne, die bis zum Vorliegen des passenden, finalen Schafts vergeht, erheblich Arbeit und Zeit einsparen (helfen).

In vielen beispielhaften Ausführungsformen bedarf es vorteilhaft keines Zugangs zu einer Quelle für elektrische Spannung, zu Druckluft oder zu einer Wasserleitung. Die Vorrichtung kann daher autark und mobil verwendet werden. Dies betrifft in einigen beispielhaften Ausführungsformen die gesamte Vorrichtung. In anderen beispielhaften Ausführungsformen weist der Druckbehälter keinen Zugang zu einer Quelle für elektrische Spannung auf, bestimmte Einrichtungen der Vorrichtung, etwa jene zum Vermessen, Abtasten usw. sind jedoch mit einem Zugang zu einem Spannungsnetz ausgestaltet. Der Patient kann das erfindungsgemäße Verfahren ohne Fachwissen und Unterstützung möglicherweise selbst durchführen. Zu seiner Durchführung bedarf es keines medizinisch geschulten Personals.

Da der Patient die Vorrichtung stehend verwenden kann und dabei die Membran mit Anteilen seines Körpergewichts belastet, bedarf es keiner Vorkehrung, um den Körpergliedstumpf davor zu bewahren, durch axiale Reaktionskräfte, welche durch die Druckkammer auf den Körpergliedstumpf wirken und diesen aus dem Inneren des Druckbehälters verdrängen könnten, verschoben zu werden. Der Patient muss mit anderen Worten, nicht in Stellung - bezogen auf den Druckbehälter - gehalten werden. Sein Körpergewicht kann dies übernehmen.

Die Erfindung ermöglicht gegenüber den herkömmlichen Verfahren des Stands der Technik, auf eine objektive Art und Weise Schäfte für Prothesen zu erstellen. Dies gewährleistet eine bessere Versorgung durch verbesserte Passform und kann die Herstellungskosten dadurch senken, dass es keiner oder nur geringer kostenintensiver manueller Nacharbeitung und Anpassung bedarf. Zudem kann die Patientenversorgung beschleunigt werden, da die auch zeitaufwendigen Anpassungsschritte zumindest anzahlmäßig stark verringert werden können oder sogar ganz entfallen.

Durch das erfindungsgemäße Verfahren kann eine Nachvollziehbarkeit erreicht werden, da der Gipsabdruck, das Datenmodell, Prothesenschaft und/oder auf überprüfbaren Grundlagen stattfindet.

Die vorliegende Offenbarung wird im Folgenden anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- Fig. 1: zeigt eine längs geschnittene Vorrichtung in einer ersten Ausführungsform von der Seite;
- Fig. 2: zeigt eine längs geschnittene Vorrichtung in einer zweiten Ausführungsform von der Seite;
- Fig. 3: zeigt eine längs geschnittene Vorrichtung in einer dritten Ausführungsform von der Seite;
- Fig. 4: zeigt eine längs geschnittene Vorrichtung in einer vierten Ausführungsform von der Seite;
- Fig. 5: zeigt eine längs geschnittene Vorrichtung in einer fünften Ausführungsform von der Seite;
- Fig. 6: zeigt eine längs geschnittene Vorrichtung in einer sechsten Ausführungsform von der Seite, welche auf einer Aufstellfläche oder einen Untergrund steht;
- Fig. 7: zeigt eine längs geschnittene Vorrichtung in einer siebten Ausführungsform von der Seite;
- Fig. 8: zeigt eine längs geschnittene erfindungsgemäße Vorrichtung in einer achten Ausführungsform von der Seite;
- Fig. 9: zeigt eine Membran einer nicht weiter dargestellten Vorrichtung in einer weiteren Ausführungsform von vorne mit leichter Perspektive von oben;
- Fig. 10: zeigt eine Membran einer nicht weiter dargestellten Vorrichtung in einer weiteren Ausführungsform;
- Fig. 10a: zeigt eine Membran einer nicht weiter dargestellten Vorrichtung in einer weiteren Ausführungsform;
- Fig. 10b: zeigt eine Membran einer nicht weiter dargestellten Vorrichtung in einer wiederum weiteren Ausführungsform;
- Fig. 11: zeigt Abschnitte eines Druckbehälters einer erfindungsgemäßen Vorrichtung in einer neunten Ausführungsform;
- Fig. 12: zeigt eine erfindungsgemäße Vorrichtung in einer zehnten Ausführungsform;
- Fig. 13: zeigt eine Vorrichtung in einer elften Ausführungsform;
- Fig. 14: zeigt eine erfindungsgemäße Vorrichtung in einer zwölften Ausführungsform;
- Fig. 15: zeigt eine erfindungsgemäße Vorrichtung in einer dreizehnten Ausführungsform;
- Fig. 16: zeigt eine Berechnungsvorrichtung;
- Fig. 17: zeigt eine Vorrichtung in einer vierzehnten Ausführungsform mit einer mehrteiligen Wandung;
- Fig. 18: zeigt die Vorrichtung der Fig. 17 in einem zusammengeschobenen Transportzustand;
- Fig. 19: zeigt eine Vorrichtung in einer fünfzehnten Ausführungsform mit einer nach außen gewölbten (oder gekrümmten) Bodenfläche;
- Fig. 20: zeigt eine Vorrichtung in einer sechzehnten Ausführungsform mit einer nach innen gewölbten Bodenfläche;
- Fig. 21: zeigt eine Vorrichtung in einer siebzehnten Ausführungsform mit einem kugelgelenkförmigen Ansatz in oder an der Bodenfläche;
- Fig. 22: zeigt ein System mit einer erfindungsgemäßen medizinischen Vorrichtung und einer hier von getrennt vorliegenden Auflagefläche;
- Fig. 23: zeigt ein System mit einer als Ellipsoid ausgestalteter Auflagefläche;
- Fig. 24: zeigt ein System mit einer als Kugel ausgestalteter Auflagefläche;
- Fig. 25: zeigt ein System mit einer mit der Bodenfläche der Vorrichtung verbundenen Auflagefläche;
- Fig. 26: zeigt ein System mit einer Sicherungsmanschette;
- Fig. 27: zeigt eine Vorrichtung in einer achtzehnten Ausführungsform mit einer Entlastungseinrichtung;
- Fig. 28: zeigt eine Vorrichtung in einer neunzehnten Ausführungsform mit einer weiteren Entlastungseinrichtung;
- Fig. 29: zeigt eine Vorrichtung in einer zwanzigsten Ausführungsform mit einer Zugvorrichtung zum Fixieren des Körpergliedstumpfs im Druckbehälter;
- Fig. 30: zeigt die Anordnung der Fig. 29 im fixierten Zustand des Körpergliedstumpfs im Druckbehälter;
- Fig. 31: zeigt die Vorrichtung mit dem Druckbehälter und einer schlauchförmigen Membran;
- Fig. 32: zeigt die Vorrichtung mit dem Druckbehälter und einer weiteren schlauchförmigen Membran;
- Fig. 33: zeigt die Vorrichtung mit Sensoren in dem Adapter und/oder in der Membran;
- Fig. 34: zeigt die Vorrichtung mit einer Einrichtung zum Fixieren und/oder Verstellen der Zugvorrichtung; und
- Fig. 35: zeigt die Vorrichtung mit einer weiteren Einrichtung zum Fixieren und/oder Verstellen der Zugvorrichtung;
- Fig. 36: zeigt ein Set mit einem Haftstrumpf und einer Vorrichtung;
- Fig. 37: zeigt eine erfindungsgemäße Einheit mit einer erfindungsgemäßen Vorrichtung und einer Druckbeaufschlagungs-Steuervorrichtung;
- Fig.38: zeigt eine erfindungsgemäße Vorrichtung mit einem Formkörper zum Aufnehmen des distalen Bereichs des Körpergliedstumpfs sowie einen Verbundstrumpf;
- Fig. 39: zeigt die Anordnung der Fig. 38 im zusammengeführten Zustand;
- Fig. 40: zeigt eine erfindungsgemäße Vorrichtung mit einem mehrteiligen Formkörper zum Aufnehmen des distalen Bereichs des Körpergliedstumpfs;
- Fig. 40a-c: zeigen unterschiedliche Größen eines kompressiblen Materials und dazugehörige Schalen zum Aufnehmen des distalen Bereichs des Körpergliedstumpfs;
- Fig. 40d-g: zeigen unterschiedliche Längen eines mehrteiligen Formkörpers zum Längenausgleich unterschiedlich langer Körpergliedstümpfe; und
- Fig. 41: zeigt eine schematische Anordnung von Bauteilen innerhalb der Druckbeaufschlagungs-Steuervorrichtung.

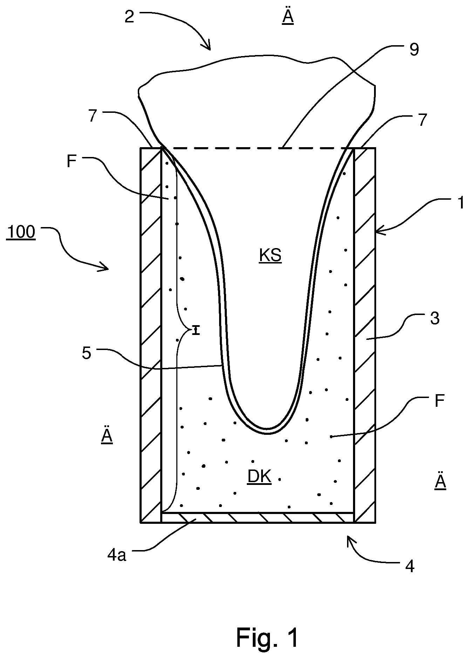

**Fig. 1** zeigt eine längs oder in Längsrichtung (also bezogen auf Fig. 1 von oben nach unten) geschnittene Vorrichtung 100 einer ersten Ausführungsform von der Seite.

Die erste Ausführungsform zeigt dabei die einfachste Ausgestaltung der Vorrichtung 100, in welcher diese wenigstens einen Druckbehälter 1 mit einer Wandung 3 und einer Membran 5 aufweist oder gar hieraus besteht.

Der Druckbehälter 1, welcher rein optional wie in Fig. 1 gezeigt zylindrisch ist, weist optional eine erste Stirnseite 2 (oben in Fig. 1) und eine zweite Stirnseite 4 (unten in Fig. 1) auf. Die zweite Stirnseite 4 ist in der exemplarischen Ausführungsform der Fig. 1 mit einer Bodenplatte oder Bodenfläche 4a fluiddicht gegenüber einem Äußeren Ä verschlossen. Die Bodenfläche 4a kann aus demselben Material wie die Wandung 3 gefertigt sein.

Die Membran 5 trennt eine Fluidkammer oder Druckkammer DK des Druckbehälters 1 fluiddicht gegenüber einem Äußeren der Fluidkammer oder Druckkammer DK, oder beispielsweise gegenüber dem Äußeren Ä, also einer Umgebung des Druckbehälters 1, oder, wie in Fig. 1 gezeigt, gegenüber einem in die Membran 5 eingeführten oder von dieser umgebenen Körpergliedstumpf KS.

Die Membran 5 kann an einem oberen, oftmals ringförmigen, rechteckigen, quadratischen oder auf andere Weise umlaufenden Rand 7 der Wandung 3, oder an anderer Stelle, mit dem Druckbehälter 1 fluiddicht verbunden sein.

Der obere Rand 7 liegt in einer Ebene, in welcher eine Einführöffnung 9 des Druckbehälters 1 liegt, oder begrenzt diese an deren Umfang. Die Einführöffnung 9 liegt in der mittels Strichlinie bezeichneten Ebene.

Die Einführöffnung 9 dient dem Einführen des mit der feuchten Gipsbinde umwickelten Körpergliedstumpfs KS, siehe unten, in ein Inneres I des Druckbehälters 1.

Das Innere I ist der von der Wandung 3 begrenzte Rauminhalt des Druckbehälters 1. Es reicht von der mit der Bodenfläche 4a fluiddicht verschlossenen, zweiten

Stirnseite 4 bis zur mit 9 bezeichneten, mittels Strichlinie angedeuteten Einführöffnung.

Die Druckkammer DK ist mit einem Fluid befüllt, hier exemplarisch mit einer durch Punkte angedeuteten Flüssigkeit F. Eine Befüllung mit Gas ist ebenfalls von der vorliegenden erfasst.

In Fig. 1 ist die Vorrichtung 100 in einem Zustand gezeigt, in welchem der äußerst schematisch angedeutete Körpergliedstumpf KS des stehenden Patienten in das Innere I derart eingeführt ist, dass er zumindest in seinem distalen Abschnitt von der Membran 5 umgeben ist. Die Membran 5 liegt an der Gipsbinde auf dem Körpergliedstumpf KS wie eine zweite Haut an, wobei zwischen Gipsbinde und Membran 5 weitere Schichten wie Liner oder dergleichen vorgesehen sein können.

Der Körpergliedstumpf KS wird vom stehenden Patienten mit dessen vollem Körpergewicht belastet. Die Menge der Flüssigkeit F ist in Kenntnis des Volumens des Inneren I oder des Druckbehälters 1 derart bemessen, dass der Körpergliedstumpf KS wenigstens derart tief durch die Einführöffnung 9 hindurch in den Druckbehälter 1 eintreten kann, dass die gesamte Fläche der Gipsbinde, jedenfalls aber soweit für den Abdruck relevant, mit der Membran 5 in Kontakt steht. Zugleich ist die Menge an Flüssigkeit F so bemessen, dass das distale Ende des Körpergliedstumpfs KS (unten in Fig. 1) den Boden des Druckbehälters 1 nicht berührt bzw. sich nicht hierauf abstützt. So ist sichergestellt, dass es der Druck des Fluids ist, auf welchem der Patient mit der eingeführten Extremität ruht, und dass die Gipsbinde mittels der Membran 5 an jeder Stelle denselben Druck erfährt.

Es ist der Figur entnehmbar, dass die Membran 5 dann, wenn kein Körpergliedstumpf KS in den Druckbehälter 1 eingeführt ist, durch den Druck des Fluids, hier der Flüssigkeit, aufschwimmt oder -treibt und sich ein in Fig. 1 nicht gezeigter Flüssigkeitsspiegel einstellt. Die Form der Membran 5, die in Fig. 1 gezeigt ist, stellt also jene Form dar, welche die Membran 5 bei Belastung annimmt, wenn sie an dem eingeführten Körpergliedstumpf KS anliegt und durch diesen - im Beispiel der Fig. 1 - unter elastischer Dehnung in die Tiefe des Inneren I in Richtung auf die Bodenfläche 4a hin "mitgenommen" wird.

Es ist ferner der Figur entnehmbar, dass Wandung 3 und die Membran 5 dadurch, dass sie einen Fluidaustausch zwischen der Druckkammer DK und dem Äußeren Ä verhindern oder ein Austreten von Fluid aus der Druckkammer DK verhindern, ermöglichen, dass sich der gewünschte Druck innerhalb der Druckkammer DK des Druckbehälters 1 aufbaut, nicht aber hieraus entweichen oder sich abbauen kann.

Wie Fig. 1 zu entnehmen ist wird die Druckkammer DK somit von der Membran 5 und wenigstens Teilen der Wandung 3 gebildet, zu welchen hier auch die Bodenfläche 4a der Stirnseite 4 zählt.

In anderen als der in Fig. 1 gezeigten beispielhaften Ausgestaltung der vorliegenden Offenbarung kann die Druckkammer DK aus einer rundum geschlossenen Membran, die ähnlich einem Ballon oder einer Blase im Inneren I des Druckbehälters 1 liegen kann, bestehen oder eine solche umfassen.

Für den in Fig. 1 oder in einer der folgenden Figuren gezeigten Druckbehälter 1 gilt ebenso wie für den Druckbehälter jeder anderen Ausführungsform, dass dieser anstelle eines runden Querschnitts einen nicht-runden, vorzugsweise einen eckigen, Querschnitt aufweisen kann. Der Querschnitt kann beispielsweise rechteckig, mehreckig oder quadratisch sein. Die letztgenannten Querschnitte können insbesondere bei der automatischen Vermessung des in die Vorrichtung 100 eingeführten Körpergliedstumpfs KS mittels Kamera oder dergleichen wie hierin beschrieben Artefakte, welche durch die gewölbte Umfangsfläche entstehen können, vorteilhaft vermeiden oder vermindern.

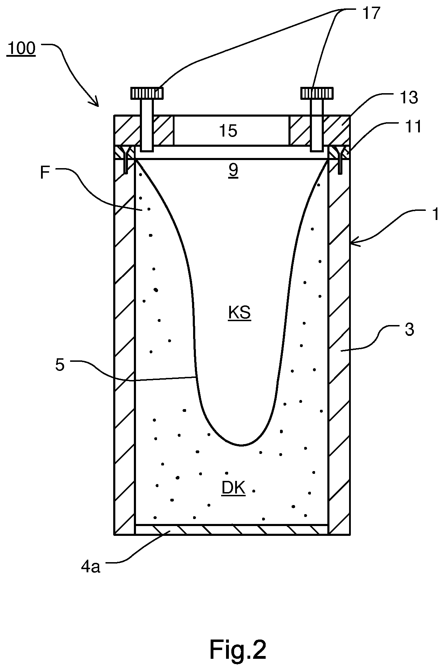

**Fig.** 2 zeigt eine längs geschnittene Vorrichtung 100 in einer zweiten Ausführungsform von der Seite.

Die zweite Ausführungsform entspricht im Wesentlich der in Fig. 1 gezeigten ersten Ausführungsform. Hinzugetreten ist gegenüber der Darstellung der Fig. 1 ein Klemmring 11, welcher z. B. auf dem oberen Rand der Wandung 3 des Druckbehälters 1, um mit diesem verklemmt zu sein oder zu werden, vorgesehen sein kann. Die Membran 5 ist dabei zwischen oberem Rand und Klemmring 11 lösbar mit dem Druckbehälter 1 fluiddicht gegenüber dem Inneren der Druckkammer verbunden. Diese lösbare Anordnung erlaubt es vorteilhaft, den Druckbehälter 1 rasch und mit geringer Mühe mit einer den konkreten Umständen oder dem konkreten Patienten angepassten Membran 5 zu verbinden. Die lösbare Anordnung erlaubt es ferner vorteilhaft, eine beschädigte Membran 5 auf einfache Weise austauschen zu können.

Ebenso optional wie der Klemmring 11 ist der in Fig. 2 gezeigte Begrenzungsring 13, hier ein Oberschenkeldichtring. Dieser kann ein- oder mehrteilig sein und derart angeordnet sein, dass er die Einführöffnung 9 mit Ausnahme einer Beinöffnung 15, verschließt. Die Größe und/oder Form der Beinöffnung 15 ist vorzugsweise ausgewählt, um dicht am Körpergliedstumpf KS oder Oberschenkel anzuliegen. Dies verhindert bei eingeführtem Körpergliedstumpf KS ein durch den im Druckbehälter 1 herrschenden Druck bedingtes Auftreiben der Membran 5 am Körpergliedstumpf KS vorbei und über die Einführöffnung 9 hinaus.

Der Begrenzungsring 13 kann mittels Schrauben 17 mit dem Druckbehälter 1 verschraubt oder anderweitig lösbar verbunden sein.

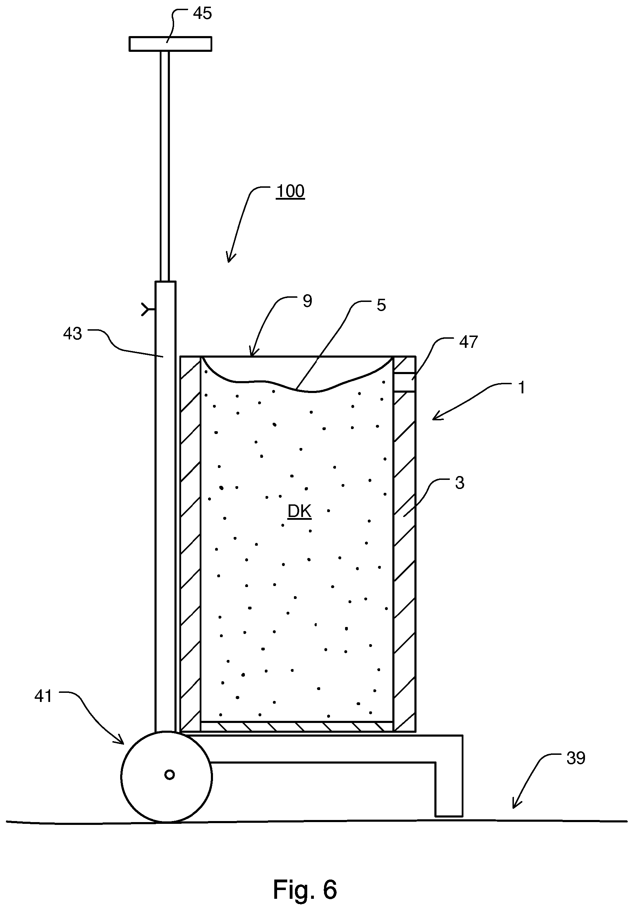

Fig. 1 und Fig. 2 zeigen wie Fig. 6 eine sehr einfache Ausführungsform der vorliegenden Offenbarung, bei welcher die Druckkammer DK eine unveränderliche, vorbestimmte Menge an Fluid aufweist. Der in ihr herrschende Druck steigt in dem Moment, in welchem der Patient mit dem Körpergliedstumpf KS durch die Einführöffnung 9 und auf die Membran 5 steigt. Ist die Menge des Fluids in Abhängigkeit von dessen Komprimierbarkeit geeignet ausgewählt, so bedarf es keines Auslasses, um durch eine Veränderung der Fluidmenge korrigierend einzugreifen. Die übrigen Figuren zeigen hingegen Ausführungsformen, bei welchen eine solche Veränderung dadurch möglich ist, dass ein Auslass 19 vorgesehen ist. Diese Ausführungsformen bieten vorteilhaft die Möglichkeit der Anpassung der Vorrichtung an den konkreten Patienten auch wenn dieser bereits mit seinem Körpergliedstumpf KS in der Vorrichtung 100 steht.

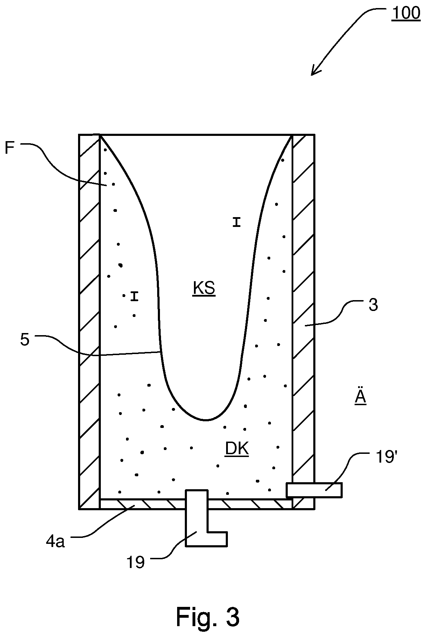

**Fig. 3** zeigt eine längs geschnittene Vorrichtung 100 in einer dritten Ausführungsform von der Seite.

Die dritte Ausführungsform entspricht im Wesentlich der in Fig. 1 gezeigten ersten Ausführungsform, wobei, wie bei den folgenden Figuren, auf schraffierte Darstellung verzichtet wurde. Hinzugetreten ist gegenüber der Darstellung der Fig. 1 ein Auslass 19 in der Bodenfläche 4a des Druckbehälters 1 und/oder ein Auslass 19' in der Seitenwand oder Wandung 3 des Druckbehälters 1.

Der Auslass 19, 19' verbindet das Innere I im Bereich der Druckkammer DK mit dem Äußeren Ä des Druckbehälters 1. Er erlaubt es, den Füllstand und/oder Druck innerhalb der Druckkammer DK gezielt zu verändern, beispielsweise indem Fluid aus der Druckkammer DK über den Auslass 19, 19' abgelassen wird. Der Auslass 19, 19' ist hierzu mit einem nicht näher dargestellten, fluiddicht schließbaren Ventil oder Sperrhahn versehen. Ungeachtet seiner Bezeichnung als Auslass kann dieser auch zum Einlassen von Fluid und damit zum Befüllen der Druckkammer DK verwendet werden.

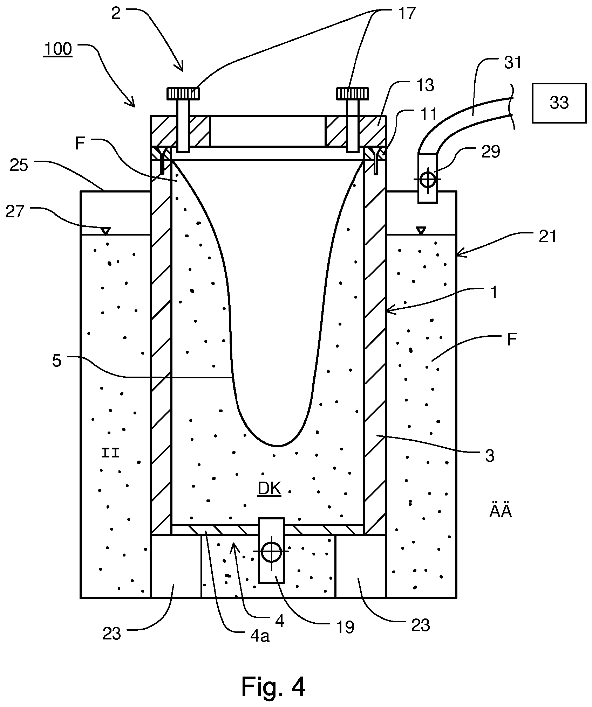

**Fig. 4** zeigt eine längs geschnittene Vorrichtung 100 in einer vierten Ausführungsform von der Seite.

Die vierte Ausführungsform basiert auf der in Fig. 2 gezeigten zweiten Ausführungsform. Die Vorrichtung 100 weist neben dem Druckbehälter 1 auch einen Vorratsbehälter 21 auf.

Der Vorratsbehälter 21 umgibt den in ihm beispielsweise auf Füßen 23 ruhenden Druckbehälter 1 in dieser exemplarischen Ausführungsform zumindest teilweise. Ein Inneres II des Vorratsbehälters 21 steht über den Auslass 19 (dieser kann ein Ventil oder einen Sperrhahn aufweisen) mit der Druckkammer DK des Druckbehälters 1 in Fluidverbindung. Die Fluidverbindung kann geöffnet oder geschlossen werden. Ein Fluidaustausch zwischen der Druckkammer DK des Druckbehälters 1 und dem Inneren II des Vorratsbehälters 21 kann somit zugelassen oder unterbunden werden.

Die Füße 23 sind eine mögliche Ausgestaltung einer Aufstellvorrichtung der Vorrichtung 100, mittels welcher der Druckbehälter 1 derart aufgestellt werden kann, dass seine erste Stirnseite 2 oben und seine zweite Stirnseite 4 unten (bezogen auf den Gebrauchszustand der Vorrichtung 100) angeordnet sind.

Das Innere II des Vorratsbehälters 21 kann optional fluiddicht gegenüber einem Äußeren ÄÄ des Vorratsbehälters 21 abgeschlossen sein, beispielsweise mittels eines Deckels 25.

Im Inneren II des Vorratsbehälters 21 liegt wie im Inneren I des Druckbehälters 1 ein Fluid F vor. Das Fluid F hat einen Spiegel 27. Der Vorratsbehälter 21 kann einen optionalen Einlass 29 aufweisen (welcher wiederum ein fluiddicht verschließbares Ventil oder einen Sperrhahn aufweisen kann), über welchen das Innere II des Vorratsbehälters 21 mittels einer wiederum optionalen Druckleitung 31 mit einer rein optionalen Druckquelle 33 verbunden sein kann. Wird mittels der Druckquelle 33 oder auf andere Weise bei geöffnetem Einlass 29 der Druck im Inneren II erhöht, so steigt er bei ebenfalls geöffnetem Auslass 19 auch in der Druckkammer DK, und umgekehrt. Auf diese Weise kann der Druck in der Druckkammer DK geändert und bei entsprechender Betätigung von Auslass 19 bzw. Einlass 29 eingestellt werden.

Das zum Aufbringen des gewünschten Drucks auf den Körpergliedstumpf KS erforderliche Fluid kann somit vollständig, und gegenüber dem Äußeren ÄÄ abgeschlossen, im Vorratsbehälter 21 der Vorrichtung 100 vorgesehen sein. Es bedarf beim Einsatz der Vorrichtung 100 in diesen Ausführungsformen keines Zuführens von Fluid aus externen Quellen wie Wasserleitungen, Druckluftquellen und dergleichen. Der Druck, welcher mittels der Druckleitung 31 und/oder dem Fluid aus dem Vorratsbehälter 21 aufgebracht wird, kann mittels Hand-Luftpumpe, Fussbalg oder dergleichen, insbesondere ohne elektrische Spannung zu benötigen, erzeugt werden.

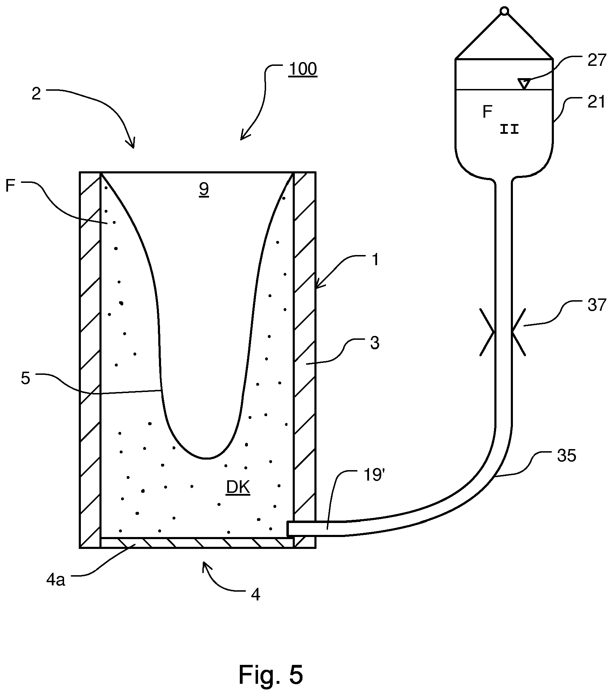

**Fig. 5** zeigt eine längs geschnittene Vorrichtung 100 in einer fünften Ausführungsform von der Seite.

Wie in der vierten Ausführungsform weist die Vorrichtung 100 sowohl einen dem Druckbehälter 1 als auch einen Vorratsbehälter 21 auf.

Während der Druckbehälter 1 in der in Fig. 4 gezeigten Ausführungsform jedoch im Inneren II des Vorratsbehälter 21 angeordnet ist, derart, dass die Druckkammer DK mittels des Auslasses 19 unmittelbar mit dem Inneren II in Fluidverbindung steht, liegen in der fünften Ausführungsform Druckbehälter 1 und Vorratsbehälter 21 getrennt oder unabhängig voneinander vor. Sie sind allerdings mittels einer Ausgleichsleitung 35 miteinander in Fluidkommunikation verbunden. Eine Flussregelvorrichtung, wie z. B. eine Klemme 37, kann vorgesehen sein, um den Fluidstrom innerhalb der Ausgleichsleitung 35 zu unterbinden. Zudem, oder alternativ, kann der Auslass 19' das o. g. Ventil oder den o. g. Sperrhahn aufweisen.

Wie in Fig. 4 kann der Vorratsbehälter 21 optional mit einer Druckquelle verbunden sein. Alternativ genügt es oftmals aber auch, zum Ändern des Drucks in der Druckkammer DK des Druckbehälters 1 den Vorratsbehälter 21 höher oder tiefer zu halten, oder ihn zu drücken oder den Druck auf ihn zu verringern.

Verschließbare oder nicht verschließbare Entlüftungsöffnungen können optional am Vorratsbehälter 21 vorgesehen sein. Sie können für einen Druckausgleich vorgesehen sein.

**Fig. 6** zeigt eine längs geschnittene Vorrichtung 100 in einer sechsten Ausführungsform von der Seite, welche auf einer Aufstellfläche oder einem Untergrund 39 steht.

Die Vorrichtung 100 der Fig. 6 hat, ungeachtet aller übrigen Merkmale, optional eine Rolleneinrichtung 41 mit wenigstens einer Rolle, einem Rad oder einer Walze. Die Rolleneinrichtung 41 erlaubt es, die Vorrichtung 100, welche insbesondere in einem mit Flüssigkeit gefüllten Zustand ein beachtliches Gewicht haben kann, mühelos von einem Behandlungsraum in den nächsten zu bewegen.

Die Vorrichtung 100 der Fig. 6 hat ferner, wiederum ungeachtet aller übrigen Merkmale, optional wenigstens eine Stütze 43, an welcher sich der mit seinem Körpergliedstumpf KS im Druckbehälter 1 stehende Patient abstützen kann. Die Stütze 43 kann zugleich zum Führen der Vorrichtung 100 benutzt werden, wenn diese mittels der Rolleneinrichtung 41, soweit vorgesehen, beispielsweise ähnlich einer Sackkarre, rollend über den Flur bewegt wird.

Die Stütze 43 kann optional verstellbar ausgestaltet sein. Sie kann höhenverstellbar sein (etwa mittels der in Fig. 6 angedeuteten Teleskopiereinrichtung), sie kann im Neigungswinkel (bezogen auf den Untergrund 39) einstellbar sein, und/oder sie kann einen Haltegriff 45 aufweisen.

Der Druckbehälter 1 kann in jeder Ausführungsform eine Luftablassöffnung 47 aufweisen, aus welcher beim Befüllen der Druckkammer DK des Druckbehälters 1 mit beispielsweise Wasser noch vom Herstellungsprozess und zum Transportieren eingebrachte Luft entweichen kann. Die Luftablassöffnung 47 dient nicht dem Einführen von Luft oder Druckluft. Sie dient vielmehr dazu, sicherzustellen, dass der Druckbehälter 1 oder seine Druckkammer DK nach seiner Befüllung mit einer Flüssigkeit luftfrei ist. Dies stellt sicher, dass der spätere Gipsabdruck nicht durch eventuell vorhandene Luftblasen Falten oder andere unerwünschte Oberflächenstrukturen aufweist oder das bei der Vermessung gewonnene Datenmodell oder die diesem zugrunde liegenden Daten keine vermeidbaren Fehler wegen Lufteinschlusses aufweisen.

Vorzugsweise liegt die Luftablassöffnung 47 in einem Abschnitt des Druckbehälters 1 oder seiner Wandung 3, welcher Teil der Druckkammer DK ist, an einer dem Auslass 19 oder 19' gegenüberliegender Fläche, Seite oder Stirnseite des Druckbehälters 1. So kann in die Druckkammer DK eingebrachtes Wasser auf einfachste und zugleich sicherste Weise vorhandene Luft unter Ausnutzung der unterschiedlichen Dichten zwischen Wasser und Luft vollständig verdrängen.

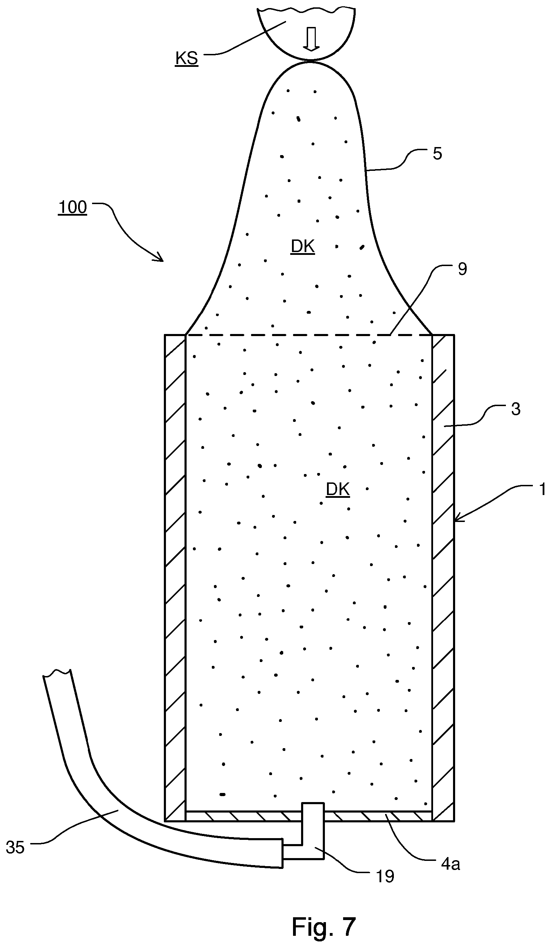

Fig. 7 zeigt eine längs geschnittene Vorrichtung 100 in einer siebten Ausführungsform von der Seite.

In Fig. 7 ist die Druckkammer DK des Druckbehälters 1 soweit mit Fluid, z. B. Wasser, gefüllt, dass sich die Membran über die Einführöffnung 9 hinaus nach außen, also zum Äußeren Ä des Druckbehälters 1, wölbt, wobei sich zwar nicht das Innere I des Druckbehälters 1, wohl aber das Volumen der Druckkammer DK vergrößert.

Der Patient kann nun auf besondere leichte Weise mit seinem Körpergliedstumpf KS durch die Einführöffnung 9 in den Druckbehälter 1 einsteigen. Er setzt hierzu das distale Ende des Körpergliedstumpfs KS auf die Spitze oder den oberen Bereich der Membran 5 auf, in etwa dort, wo der Doppelpfeil in Fig. 7 hinweist. Beim Senken des Körpergliedstumpfs KS in Pfeilrichtung tritt dieser durch die Einführöffnung 9 hindurch in den Druckbehälter 1 ein und nimmt die Membran 5 dabei an seiner Außenseite mit, indem er sie umstülpt. Die Membran 5 nimmt dabei schließlich ihre in z. B. Fig. 1 gezeigte Form ein. Dies scheint die bequemste Weise des Einführens des Körpergliedstumpfs KS in das Innere I des Druckbehälters 1 zu sein, wo er von Abschnitten der Druckkammer DK umgeben ist. Dies gilt vor allem angesichts der Tatsache, dass der Körpergliedstumpf KS gegen den Ausgangsdruck, welcher zu Beginn des Einführens des Körpergliedstumpfs KS in das Innere I bereits in der Druckkammer DK herrscht, in das Innere I zu führen ist, wobei sich dieser Druck während des Einführens aufgrund des Raumbedarfs des Körpergliedstumpfs KS im Inneren I erschwerend noch erhöhen kann. Das durch den Körpergliedstumpf KS aus der Druckkammer DK beim Einführen verdrängte Fluid oder Wasser kann mittels des Auslasses 19 abgelassen werden, etwa in einen in Fig. 7 nicht dargestellten Vorratsbehälter 21, mit welchem die Druckkammer DK mittels Ausgleichsleitung 35 in Fluidverbindung verbunden sein kann. Das zum Auswölben der Membran 5, wie in Fig. 7 gezeigt, erforderliche Fluid, kann vorab ebenfalls durch den Auslass 19 eingebracht worden sein. Es kann dem Vorratsbehälter 21 entnommen worden sein.

Der Auslass 19 wird geschlossen, wenn die gewünschte Einführtiefe des Körpergliedstumpfs KS im Inneren I des Druckbehälters 1 erreicht ist. Dass dies der Fall ist, kann beispielsweise anhand der o. g., in Fig. 7 nicht gezeigten Markierungen festgestellt werden. Zudem kann durch einen Seitenvergleich (linkes Bein zum rechten Bein oder Hüftenschrägstellung) festgestellt werden, wann die gewünschte Einführtiefe erreicht ist. Ist die gewünschte Einführtiefe erreicht und der Auslass 19 geschlossen, so kann der Patient den Körpergliedstumpf KS mit vollem Körpergewicht belasten. Der dann auf die Gipsbinde (falls vorhanden) oder den Körpergliedstumpf KS aufgrund der Druckverteilung innerhalb der Druckkammer DK gleichmäßig auf alle Flächeninhalte der Fläche der Gipsbinde oder des Körpergliedstumpfs KS wirkende Druck führt zu einem optimalen Datenmodell aus einer Vermessung, oder zu einer Modellierung des entstehenden Gipsabdrucks, welcher einem Prothesenschaft für die spätere Belastungssituation optimal entspricht. Diese Ausführungen sind nicht auf die in Fig. 7 gezeigte Ausführungsform beschränkt.

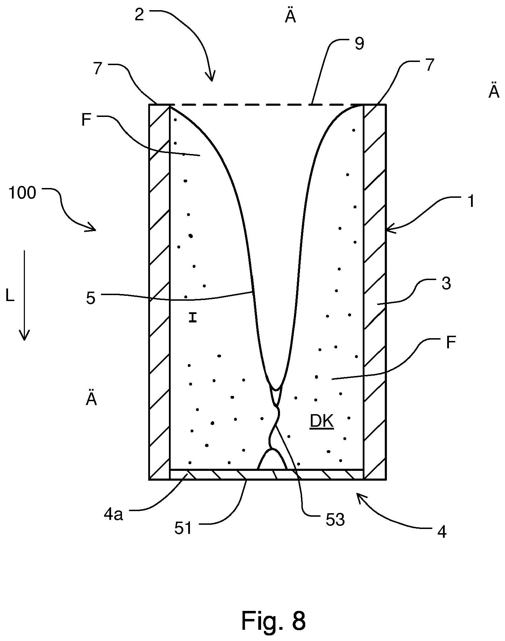

**Fig. 8** zeigt eine längs geschnittene erfindungsgemäße Vorrichtung in einer achten Ausführungsform von der Seite.

Die Druckkammer DK ist durch die geschnitten dargestellte Wandung 3, zu der hier die Bodenfläche 4a gezählt wird, und die Membran 5 gebildet.

Die Membran 5 ist - vorzugsweise in ihrem distal mittleren oder zentralen Abschnitt oder Bereich - mit einem Abschnitt 51 der Wandung 3 oder der Stirnfläche 4 kraftschlüssig verbunden. In der in Fig. 8 gezeigten Ausführungsform ist der Kraftschluss durch einen Verbinder 53 bewirkt, welcher sich von einem distalen Ende der Membran 5 bis zum Abschnitt 51, hier rein optional die Bodenfläche 4a der Druckkammer DK, erstreckt.

Der Verbinder 53 kann, wie in Fig. 8 exemplarisch gezeigt, ein Faden sein. Jeder andere geeignete Verbinder, etwa ein Band, ein Klettband oder dergleichen, ist ebenfalls von der vorliegenden Erfindung umfasst.

Der Verbinder 53 hält die Membran 5 vorzugsweise im Bereich der zweiten Stirnseite 4 des Druckbehälters 1, insbesondere im Bereich der Bodenfläche 4a und vorzugsweise in deren Mitte oder Zentrum, kraftschlüssig und/oder formschlüssig und/oder stoffschlüssig mit dem Druckbehälter 1 oder Bodenfläche 4a verbunden.

Bevorzugt ist, dass der Verbinder 53 zumindest im Gebrauchszustand der Vorrichtung 100, also bei in die Druckkammer DK eingeführtem Körpergliedstumpf KS ein Umströmen des distalen Endes des Körpergliedstumpfs KS vollständig oder im Wesentlichen vollständig zulässt. Eine Umströmung des Körpergliedstumpfs KS ist in Fig. 8 bis auf die Fläche, welche dem Querschnitt des Verbinders 53 entspricht, somit vorteilhaft noch möglich. Auf diese Weise kann unverändert ein Auftrieb oder das für Nachstellen oder Nachempfinden der späteren Belastungssituation im fertigen Schaft so wichtige Druckbeaufschlagen auch des distalen Abschnitts des Körpergliedstumpfs KS durch das Fluid erfolgen.

Neben dem in Fig. 8 gezeigten Verbinder 53 können mehrere oder weitere Verbinder vorgesehen sein. Diese können wie der Verbinder 53 mit der Bodenfläche 4a verbunden sein. Sie können alternativ oder ergänzend an einem anderen Abschnitt der Wandung 3 als der Stirnfläche oder Bodenfläche 4a mit der Druckkammer DK oder dem Druckbehälter 1 verbunden sein. Dies gilt auch für den in Fig. 8 gezeigten Verbinder 53.

Die Verbindung zwischen Verbinder 53 einerseits und Wandung 3 oder Bodenfläche 4a andererseits kann - wie unabhängig hiervon die Verbindung zwischen Verbinder 53 einerseits und Membran 5 andererseits - eine Klebverbindung, eine Schraubverbindung, eine Steckverbindung, eine Schnappverbindung, eine Rastverbindung oder dergleichen sein. Sie kann lösbar oder unlösbar sein.

Der in Fig. 8 gezeigte Verbinder ist, wie vorzugsweise auch jeder der Fig. 1, Fig. 11 und Fig. 12 nicht elastisch dehnbar. Er ist vorzugsweise nicht dehnbar.

Die in Fig. 8 gezeigte Membran 5 ist in Längsrichtung L ebenfalls vorzugsweise nicht dehnbar und/oder nicht elastisch.

Die in Fig. 8 gezeigte Form der Membran 5, in welcher der Körpergliedstumpf 5 noch nicht eingeführt ist, welche also leer ist, ist rein exemplarisch. Die Membran 5 kann sich aufgrund des Drucks des Fluids F stärker als in Fig. 8 gezeigt zusammenlegen. Tatsächlich können sich gegenüberliegende Abschnitte der Membran 5 - bei Betrachtung von oben, also in Richtung von der ersten Stirnseite 2 zur zweiten Stirnseite 4 - zumindest in Abschnitten der Membran 5, welche einigen Abstand von der Einführöffnung 9 haben, aneinander legen oder schlitzförmig aufeinander liegen. Bei Draufsicht auf die Einführöffnung kann man möglicherweise eine durch die Membran 5 zentral gebildete Eintrittsöffnung, siehe oben, sehen. Diese kann einen Durchmesser von 3 cm oder mehr haben.

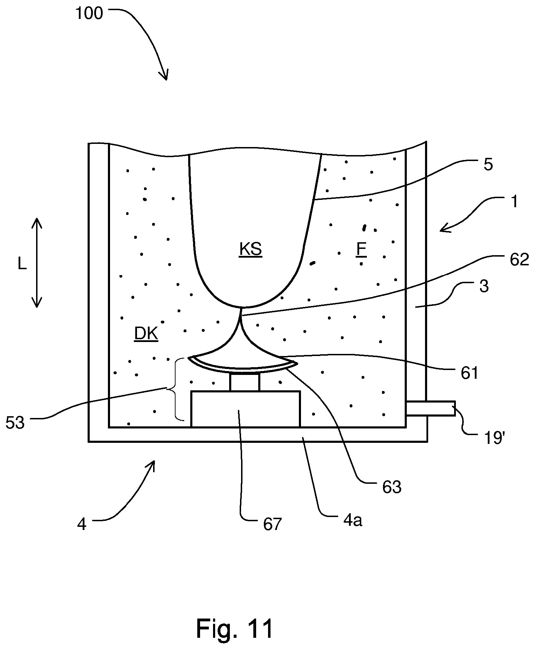

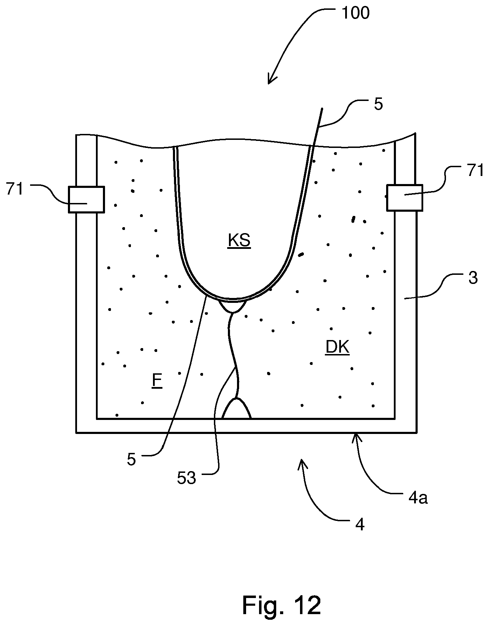

In Fig. 8 ist ein optional ebenfalls vorhandener Ein- und/oder Auslass 19 oder 19' nicht dargestellt, gleichwohl aber optional vorgesehen. Dies gilt auch für die Fig. 11 und die Fig. 12.

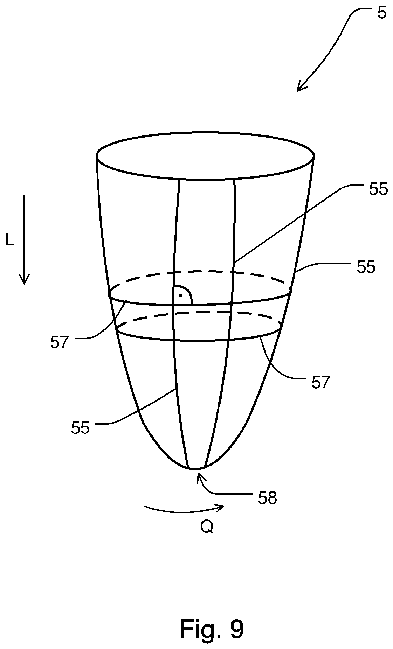

**Fig. 9** zeigt eine Membran 5 einer nicht weiter dargestellten Vorrichtung in einer weiteren Ausführungsform von vorne mit leichter Perspektive von oben. Die Membran 5 ist aus einem Material gefertigt, welches in einer ersten Richtung des Materials eine andere Elastizität hat als in einer zweiten, hier nur beispielsweise zur ersten senkrecht stehenden, Richtung. Die erste Richtung kann durch die Verläufe von Längsfasern 55 der Membran 5 beschrieben werden, die zweite Richtung durch Verläufe von Umfangs- oder Querfasern 57.

Die Längsfasern 55 können dabei weniger elastisch sein als die Umfangsfasern 57 oder (insbesondere bei identischer Kraft- oder Druckaufbringung) eine geringere Verformung der Membran in der Längsrichtung als in der Umfangsrichtung zulassen. Im Extremfall sind die Längsfasern 55 nicht elastisch und/oder nicht dehnbar, die Umfangsfasern 57 vorzugsweise hingegen schon. Die Membran 55 dehnt sich dahin in Längsrichtung L (von oben nach unten in Fig. 9) nicht oder nur wenig, während sie sich in Umfangsrichtung dem Körpergliedstumpf KS anlegen und hierzu im erforderlichen Umfang in einer Querrichtung Q dehnen kann.

Die Längsrichtung L kann der oben genannten Einführrichtung entsprechen.

Das Bezugszeichen 58 bezeichnet einen mittleren oder zentralen Bereich oder Abschnitt der Membran 55, hier die distale Spitze der Membran 5.

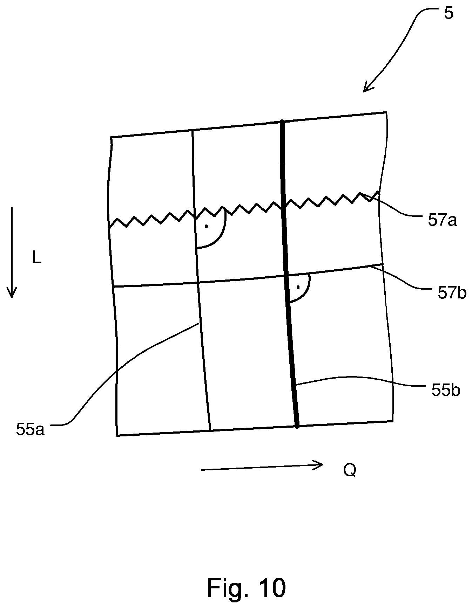

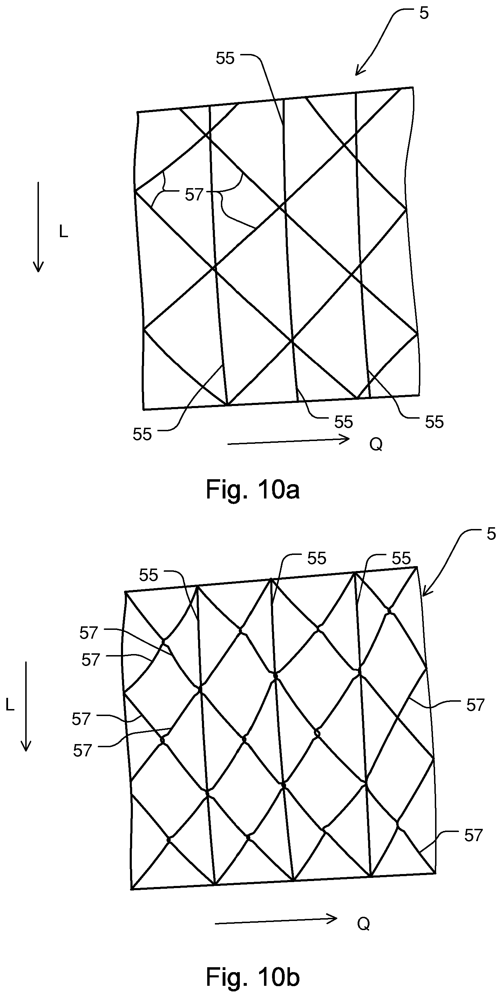

**Fig. 10** zeigt eine Membran 5 einer nicht weiter dargestellten Vorrichtung 100 in einer weiteren Ausführungsform.

Die Membran 5 ist im aufgeschnittenen Zustand gezeigt, in welchem sie nicht die Form annimmt, in welcher sie in der Vorrichtung 100 zum Einsatz kommt. Sie könnte durch Verbinden ihres linken und rechten Randes miteinander und unter Zuschneiden, wo erforderlich, in die Form gebracht werden, die in Fig. 9 gezeigt ist und welche gebrauchsfertig ist.

Zu erkennen sind rein exemplarisch zwei Paare von Längsfasern und Querfasern, welche jeweils mit 55a bzw. 57a und als 55b und 57b bezeichnet sind, die hier nur beispielhaft senkrecht zueinander stehen.

Die Längsfaser 55a ist gestreckt und erlaubt daher keine weitere Dehnung in Längsrichtung L. Die im Zusammenhang mit ihr zu betrachtende Querfaser 57a kann im Gegensatz hierzu aufgrund ihres geschlängelten oder gewellten Verlaufs oder Materials in der Matrix der Membran 5, welche neben den Fasern beispielsweise aus Silikon gefertigt ist oder Silikon aufweist, eine Dehnung der Membran 5 in Querrichtung Q erlauben. Damit unterscheiden sich die Längsfaser 55a und die Querfaser 57a in ihrer Dehnbarkeit voneinander.

Bei den beiden exemplarisch betrachteten, zugehörigen Fasern 55b und 57b kann derselbe Effekt auf andere Weise erzielt werden. Die Längsfaser 55b ist dick ausgeführt und kann sich deshalb nicht dehnen oder, verglichen mit der zugehörigen dünneren Querfaser 57b, weniger dehnen.

Es bedarf keiner Erwähnung, dass die Membran 5 mehr als die hier gezeigten Fasern aufweisen kann. Es bedarf auch keiner Erwähnung, dass die Membran 5 vorzugsweise Längsfasern und Querfasern 55a und 57a oder Längsfasern und Querfasern 55b und 57b aufweisen, vorzugsweise nicht aber beide Paare der hier diskutierten Längs- und Querfasern.

Die **Fig. 10a** und die **Fig. 10b** zeigen ähnlich der Darstellung der Fig. 10 Membranen 5 mit Längsfasern 55 und Querfasern 57, wobei die Querfasern 57 die Längsfasern 55 verbinden.

Die Längsfasern 55 verlaufen gerade, während die Querfasern 57 zwischen benachbarten (alternativ zwischen nicht benachbarten) Längsfasern 55 mit Quer- als auch Längserstreckung verlaufen.

Die Querfasern 57 verlaufen hierzu beispielsweise, wie in Fig. 10a und Fig. 10b gezeigt, in zig-zag-Form. Sie können dabei einen einfachen zig-zig-Verlauf, wie in Fig. 10a, ein doppelten zig-zag-Verlauf, wie in Fig. 10b, oder einen mehrfachen zig-zag-Verlauf, wie nicht gezeigt, annehmen.

Dabei können die Querfasern 57 die Längsfasern 55 durchaus auch aus demselben Material und/oder derselben Stärke sein. Es ist ihre Anordnung bezogen auf die Längsrichtung, die die Membran (in Querrichtung Q) dehnbar (oder mehr dehnbar) oder (in Längsrichtung L) nicht dehnbar (oder weniger dehnbar) macht.

Lässt man die Längsfasern 55 - anders als in Fig. 10a und 10b gezeigt - nicht gerade verlaufen, sondern erlaubt ihnen einen mehr oder weniger ausgeprägten zig-zag-Verlauf um eine Gerade in Längsrichtung L herum, so kann vorteilhaft eine gewünschte Verkürzung der Membran in Längsrichtung erzielt werden, wenn diese durch das Einführen des Körpergliedstumpfs KS belastet wird. Die mögliche Verkürzung kann dabei durch Abstimmung der Dimensionen und des Maßes des zig-zag-Musters der Längsfasern 55 und/oder der Querfasern 57 vorab "eingestellt" oder begrenzt werden. Eine Verkürzung kann dahingehend vorteilhaft sein, dass einer Längung der Membran 5 und/oder deren unerwünschtem Auftreiben auf struktureller Ebene einfach und doch wirkungsvoll entgegengewirkt werden kann.

**Fig. 11** zeigt einen unteren Abschnitt oder die zweite Stirnseite 4 eines Druckbehälters 1 einer Vorrichtung 100 in einer neunten Ausführungsform im Schnitt in Seitenansicht.

Die Membran 5 ist mit ihrem unteren Abschnitt 61 mit einem Verbinder 53 verbunden. Der Verbinder 53 ist wiederum mit der Bodenfläche 4a der Druckkammer DK verbunden, vorzugsweise mittig oder zentral. Auf diese Weise kann die Membran 5 in der Längsrichtung L daran gehindert werden, in Richtung zur Einführöffnung (in Fig. 11 nach oben) aufzutreiben. Sie kann sich auch nicht - oder nicht über ein gewünschtes Maß hinaus - aus der Einführöffnung hinaus in das Äußere Ä erstrecken.

Wie Fig. 11 zu entnehmen ist, könnte auch ein längerer Körpergliedstumpf KS in die Membran 5 eingeführt werden. Zwischen dem unteren Abschnitt 61 der Membran 5 und dem durch den Körpergliedstumpf KS gefüllten Bereich der Membran 5 liegt noch Material 62 der Membran 5 vor, welche durch den Druck des Fluids F zusammengedrückt wird.

Der Verbinder 53 weist hier rein exemplarisch eine Schale 63 auf, in welcher die Membran 5, hier exemplarisch mit ihrem unteren Abschnitt 61, verklebt oder anderweitig fest oder lösbar, etwa mittels einer Haken-Öse-Verbindung, verbunden ist.

Optional ist die Schale 63 einstückig mit der Membran 5 gefertigt.

Die Schale 63 ist im Beispiel der Fig. 11 mittels eines Gewindes 65 mit einem Sockel 67 verbunden. Der Sockel 67 ist mit der Bodenfläche 4a verschraubt, könnte aber oder anderweitig verbunden oder integraler Teil der Wandung 3, z. B. der Stirn- oder Bodenfläche 4a sein.

Mittels des Gewindes 65 kann die Membran 5 vorteilhaft von der Wandung 3 oder der Bodenfläche 4a gelöst werden. Andere lösbare oder nicht lösbare Verbindungen wie Steckverbindungen, Klemmverbinden, Schraubverbindungen usw. sind ebenfalls von der vorliegenden Erfindung umfasst.

**Fig. 12** zeigt den Druckbehälter 1 einer erfindungsgemäßen Vorrichtung 100 einer zehnten Ausführungsform im Schnitt in Seitenansicht.

Der Druckbehälter 1 weist in seiner Wandung 3 und/oder Bodenfläche 4a zwei oder mehr Kameras 71 (oder andere Einrichtungen wie oben beschrieben; die Kameras 71 sind rein exemplarisch gezeigt; sie stehen beispielhaft auch für allen anderen Einrichtungen, welche z. T. keine optischen Einrichtungen sind) auf zum Vermessen oder Abtasten des Körpergliedstumpfs KS.

Das Abtasten kann beispielsweise durch einfaches Ermitteln der Differenz oder des Abstands zwischen Kamera 71 (oder anderer Einrichtung, etwa einer Ultraschalleinrichtung oder einer Infraroteinrichtung) oder Wandung 3 einerseits und Membran 5 (oder Gipsoberfläche oder Hautoberfläche) andererseits erfolgen. Das Abtasten kann auf ausreichend vielen Ebenen unterschiedlicher Höhe (die Höhe kann von der Einführöffnung 9 oder deren Ebene oder von der Bodenfläche 4a aus bestimmt werden), beispielsweise mittels Infrarot oder Ultraschall oder anderen hierin genannten Einrichtungen/Messverfahren erfolgen. Aus diesen Abständen und der Kenntnis, unter welchem Winkel sie gemessen wurden, kann auf dem Fachmann bekannte Weise ein Volumenmodell oder ein Oberflächendatenmodell errechnet werden.

Das Abtasten kann ferner durch Verfahren, wie sie in den Offenlegungsschriften WO 2009/015455 A1, WO 2009/052602 A1 und WO 2010/111768 A1, ferner aus Ming Zhang et al., "Finite element modeling of a residual lower-limb in a prosthetic socket: a survey of the development in the first decade", Medical Engineering & Physics, Volume 20, No 5, 1998, Pages 360-373, Elsevier Science Ltd. ISSN: 1350-4533, und Douglas T et al: "Ultrasound imaging in lower limb prosthetics", IEEE Transactions on Neural Systems and Rehabilitation Engineering Bd. 10, Nr. 1, März 2002 (2002-03), Seiten 11-21, IEEE, USA, ISSN: 1534-4320 (XP11078071), beschrieben sind, erfolgen.

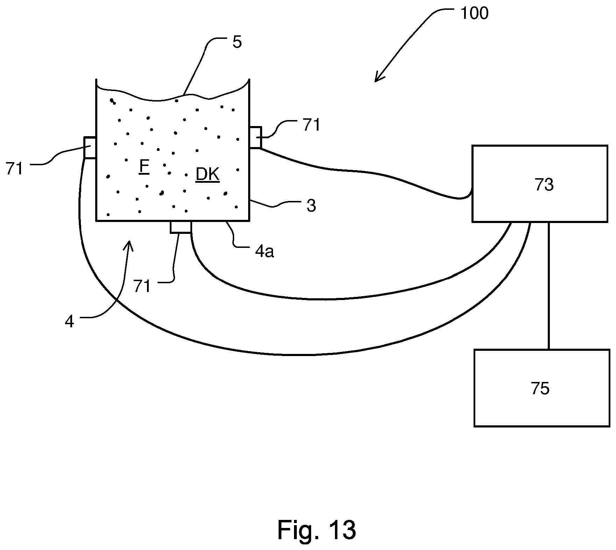

**Fig. 13** zeigt eine Vorrichtung 100 in einer elften Ausführungsform im Schnitt in Seitenansicht.

Kameras 71 sind mit einer Einrichtung 73 verbunden, welche konfiguriert ist, die mittels der Kameras 71 oder einer anderen hierin genannten Einrichtung erhobenen Daten in ein Datenmodell (Datensammlung, welche optional eine Matrixform haben kann) überzuführen. Das Datenmodell kann auf bekannte, geeignete Weise rechnerisch aufbereitet sein oder werden und die Oberfläche oder andere Informationen zur Geometrie u. a. des Körpergliedstumpfs KS widerspiegeln.

Die Einrichtung 73 kann konfiguriert sein, die ihm übermittelten Daten vor Erstellen des fertigen Datenmodells zu bearbeiten. Bei der Bearbeitung können Glättungsverfahren, Rekonstruktionsalgorithmen und Korrekturfaktoren zum Einsatz kommen.

Die Einrichtung 73 kann konfiguriert sein, das Datenmodell in einem bekannten Dateiformat abzulegen und/oder auszugeben. Eine entsprechende Ausgabeeinrichtung kann vorgesehen sein, ebenso eine geeignete Anzeigeeinrichtung zum Anzeigen des Datenmodells oder hierauf basierender Darstellung insbesondere des Körpergliedstumpfs oder des zu fertigen Schafts. Das so erstellte Datenmodell kann an anderer Stelle verwendet werden, um einen Schaft zu erstellen. Hierzu kann die Datei, welche das Datenmodell enthält, verschickt werden, beispielsweise an eine entfernt liegende Werkstatt für Prothesenschäfte, was ebenfalls von der vorliegenden Erfindung umfasst ist.

Das Datenmodell kann jedoch auch in Signalverbindung verbunden sein mit einer Formgebungs-Vorrichtung 75, die eingerichtet ist zum Herstellen des Prothesenschafts auf der Basis des Datenmodells des Körpergliedstumpfs KS oder des zu fertigenden Schafts für den Körpergliedstumpf KS. Die Signalverbindung kann leitergebunden oder "wireless" sein.

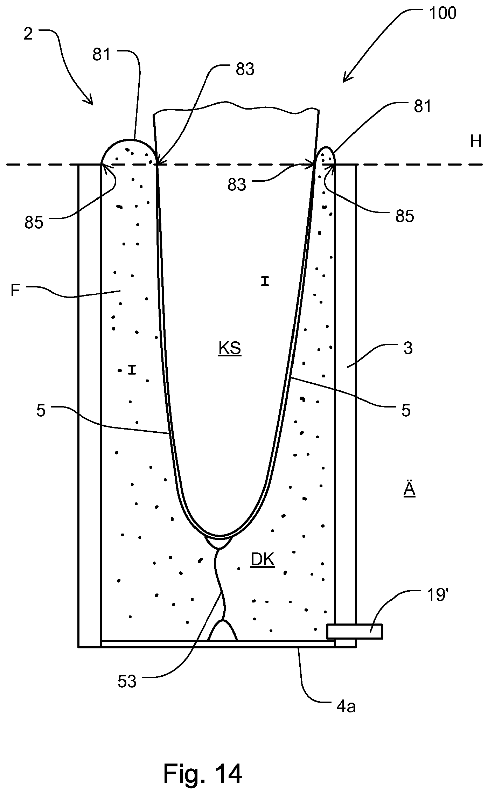

**Fig. 14** zeigt eine Vorrichtung 100 in einer zwölften Ausführungsform im Schnitt in Seitenansicht bei eingeführtem Körpergliedstumpf KS.

Die Membran 5 ist mittels eines Verbinders 51 mit der Bodenfläche 4a verbunden. Der Verbinder 51 ist nicht (oder nur in geringem Maße) elastisch oder dehnbar. Die Membran 5 ist in Längsrichtung ebenfalls nicht (oder nur in geringem Maße) elastisch oder dehnbar.

Der Verbinder 51 erlaubt ein Auftreiben der Membran 5 nach proximal (nach oben in Fig. 14). Daher stellt sich an der zweiten Stirnseite 2 eine mehr oder weniger ringförmige, jedenfalls aber in ihrem Umfang geschlossene, Auftreibung 81 ein. Die Auftreibung 81 ist bedingt durch die Schnittdarstellung der Fig. 14 als Erhöhungen links und rechts des Körpergliedstumpfs KS zu sehen.

Die Membran 5 liegt dem Körpergliedstumpf KS (oder einer über diesen gezogenen Gipsbinde, einem Liner oder dergleichen) in einer mehr oder weniger ringförmigen, jedenfalls aber in ihrem Umfang geschlossenen Kontaktpunktfläche 83 an. Die Kontaktpunktfläche 83 ist die im Umfang geschlossene Linie oder Fläche, welche die Punkte umfasst, an welchen der Körpergliedstumpf KS Kontakt nach proximal letztmalig oder abschließend Kontakt mit der Membran 5 hat. Die Kontaktpunktfläche könnte auch als Kontaktfläche bezeichnet werden.

Gleichzeitig liegt die Membran 5 an ihrem radialen Umfang im Bereich der zweiten Stirnseite 2 der Wandung 3 in einem in seinem Umfang ebenfalls geschlossenen Übergangsabschnitt 85 an oder ist hiermit befestigt. Der Übergangsabschnitt 85 kann als Bereich verstanden werden, in welchem die Begrenzung der Druckkammer DK von einer Begrenzung durch die Wandung 3 in eine Begrenzung durch die Membran 5 übergeht. Dies ist in Fig. 14 gut dadurch zu erkennen, dass die Druckkammer DK unterhalb der Pfeilspitze der Bezugszeichenlinie zum Bezugszeichen 85 allein durch die Wandung 3 begrenzt wird, oberhalb allein durch die Membran 5, die sich ihrerseits nicht gegen einen Abschnitt der Wandung 3 abstützen kann.

Die Auftreibung 81 kann durch Ablassen von Fluid oder Einführen von Fluid mittels des Auslasses oder Einlasses 19' im Zusammenwirken mit dem Verbinder 51 so eingestellt werden, dass sich die Kontaktpunktfläche 83 und der Übergangsabschnitt 85 in ein und derselbe Höhe H, angedeutet durch die Strichlinie in Fig. 14, befinden. Ist dies geschehen, so liegen nach Erfahrung des Erfinders der vorliegenden Erfindung die optimalen Druckverhältnisse vor zum Fertigen des Gipsabdrucks.

Selbst wenn der Verbinder nicht in seiner Länge verstellbar ist, stellt er vorteilhaft sicher, dass ein vorbestimmtes Maß an Auftreibung 81 nicht eintreten kann. Ein vorbestimmtes Maß kann 1 bis 4 cm betragen, besonders bevorzugt sind rund 2 cm.

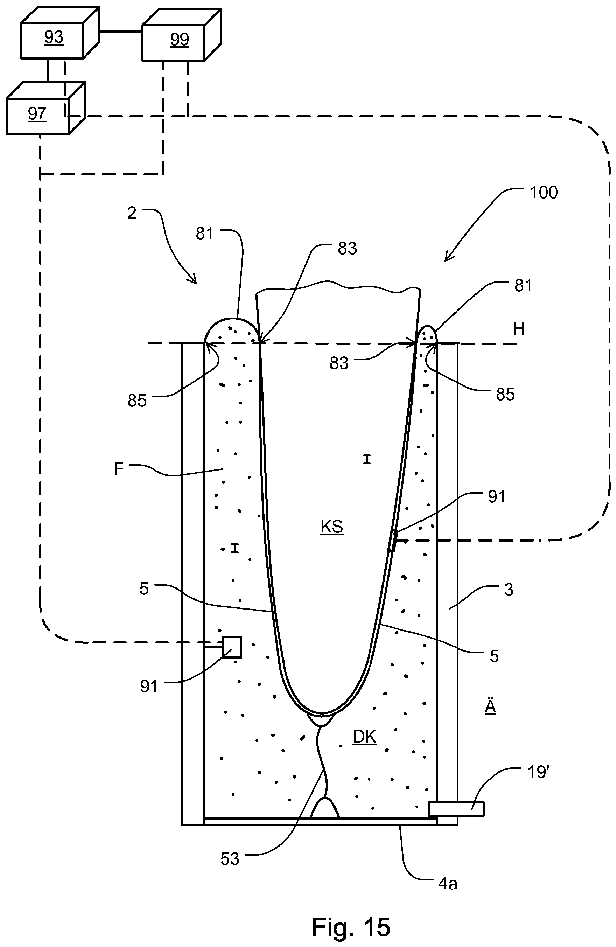

**Fig. 15** zeigt eine Vorrichtung 100 in einer dreizehnten Ausführungsform. Sie weist einen links in Fig. 15 gezeigten ersten Drucksensor 91 (oder mehrere Sensoren dieser Art) auf, welcher im Fluid der Druckkammer DK liegt und optional mit der Wandung 3 verbunden ist. Der wenigstens eine Drucksensor 91 ist angeordnet, konfiguriert und/oder eingebunden, um einen Druck zu messen, welcher den Druck in der Druckkammer DK misst. Der Drucksensor 91 kann kabellos oder leitergebunden mit der Vorrichtung 100 oder einer ihrer Einrichtungen verbunden sein oder in Signalkommunikation stehen.

Alternativ oder ergänzend kann ein rechts in Fig. 15 gezeigter Drucksensor 91 vorgesehen sein, welcher an oder in der Membran 5 oder auf einem Abschnitt der Oberfläche des von der Membran 5 im Gebrauch der Vorrichtung 100 bedeckten Körpergliedstumpfs KS und/oder zwischen Membran 5 und Körpergliedstumpf KS vorgesehen ist und den dort herrschenden Druck misst.

Optional weist die medizinische Vorrichtung 100 eine Anzeigevorrichtung 93 auf oder ist hiermit verbunden. Letztere ist konfiguriert, um von einer Druckmessvorrichtung wie z. B. vorstehend genanntem Drucksensor 91 oder von einer Auswertevorrichtung 95 ein einem Drucksignal entsprechendes Signal zu empfangen und anzuzeigen. Eine Verbindung zwischen Drucksensor 91 und Anzeigevorrichtung 93 ist jeweils mittels Strichlinie angedeutet. Sie kann leitergebunden oder kabellos sein.

Optional weist die medizinische Vorrichtung 100 eine Ermittlungsvorrichtung 97 auf oder ist hiermit verbunden. Letztere ist konfiguriert, um beispielsweise unter Einbezug eines von einer Druckmessvorrichtung wie dem wenigstens einen Drucksensor 91 ermittelten Druckwerts oder hierauf basierend das Gewicht zu ermitteln, mit welchem der Patienten beschwert werden muss, damit der Druck in der Druckkammer DK (welcher wiederum mittels der Druckmessvorrichtung wie dem Drucksensor 91 gemessen werden kann) in einem vorbestimmten Zielwertebereich für den Druck liegt oder sich in diesen hinein bewegt, wenn der Körpergliedstumpf KS in die medizinische Vorrichtung 100 eingeführt ist.

Dabei kann die medizinische Vorrichtung 100 optional eine Anzeigevorrichtung 99 aufweisen, welche konfiguriert ist, das Gewicht zu anzuzeigen, mit welchem der Patient, dessen Körpergliedstumpf KS in die medizinische Vorrichtung 100 eingeführt ist, beschwert werden muss, damit der Druck in der Druckkammer DK in einem vorbestimmten Zielwertebereich liegt.

Die Anzeigevorrichtung 99 kann gemeinsam mit der Anzeigevorrichtung 93 verwirklicht sein; es können - zu vorstehend genannten Zwecken - allerdings auch getrennte Anzeigevorrichtungen vorgesehen sein.

Die Anzeigevorrichtung 99 kann wie in Fig. 15 angedeutet direkt oder indirekt mit dem oder den Drucksensoren 91 verbunden sein.

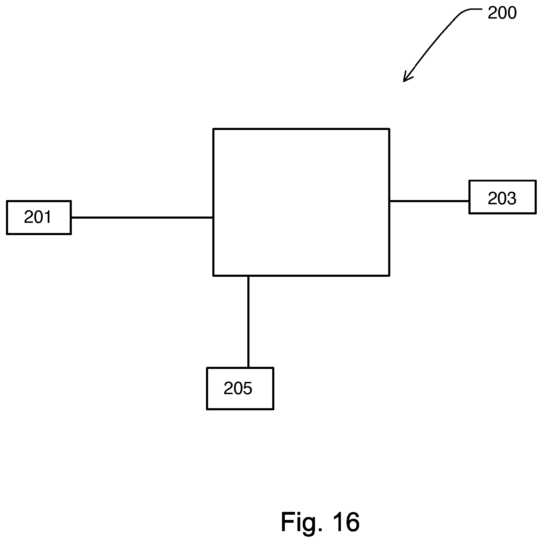

Fig. 16 zeigt eine erfindungsgemäße Berechnungsvorrichtung 200 in einer exemplarischen ersten Ausführungsform. Diese kann Teil der erfindungsgemäßen Vorrichtung 100 oder mit dieser körperlich oder in Signalverbindung verbunden sein. Sie kann allerdings auch unabhängig von der Vorrichtung 100 sein.

Die Berechnungsvorrichtung 200 weist eine Eingabevorrichtung 201 (beispielsweise eine Tastatur, einen Scanner, eine Maus, ein Interface, einen Stick, eine Speichereinheit, eine Schnittstelle (Bluetooth oder dergleichen), usw.) und eine Ausgabevorrichtung 203 (beispielsweise einen Monitor, eine Anzeige, ein Drucker, eine Speichereinheit, eine Schnittstelle (Bluetooth oder dergleichen), usw.) auf.

Die Berechnungsvorrichtung 200 ist konfiguriert, um anhand von patientenbezogenen Daten, welche über die Eingabevorrichtung 201 eingegeben werden können, das Gewicht zu ermitteln, um welches der Patient bei der Nutzung einer Vorrichtung zum Erstellen eines Gipsabdrucks, beispielsweise der hierin beschriebenen, mit einer Membran 5 und einer Druckkammer DK beschwert werden muss, um eine als vorteilhaft erkannte, vorab festgelegte Belastung der Membran 5 zu erzielen. Die vorab festgelegte Belastung kann ein Druck sein, welcher in einem vorbestimmten Zielwertebereich liegt wie hierin beschrieben.

Die Berechnungsvorrichtung 200 ist dabei optional konfiguriert, um auf hinterlegte Algorithmen zurückzugreifen. Alternativ oder ergänzend kann die Berechnungsvorrichtung 200 konfiguriert sein, um auf Referenzdaten oder -werte 205 zurückzugreifen, welche beispielsweise in einer Nachschlagetabelle hinterlegt sind.

Fig. 17 zeigt eine Vorrichtung 100 in einer vierzehnten Ausführungsform mit einer mehrteiligen Wandung. Die mehrteilige Wandung weist drei ineinander einschiebbare, zylindrische Wandungsteilabschnitte 101 auf. Der oberste Wandungsteilabschnitt 101 weist den größten Durchmesser auf, der mittlere Wandungsteilabschnitt 101 einen kleineren Durchmesser und der unterste Wandungsteilabschnitt 101 einen noch kleineren Durchmesser. Aufgrund dieser Abstufung können der mittlere Wandungsteilabschnitt 101 in den oberen und der untere in den mittleren Wandungsteilabschnitt 101 eingeschoben werden kann. Andere Konstellationen sind gleichfalls möglich, beispielsweise kann der mittlere Wandungsteilabschnitt 101 den kleinsten Durchmesser aufweisen.

Als Führungshilfe zum Zusammenschieben der zylindrischen Wandungsteilabschnitte 101 sind in diesem Ausführungsbeispiel Führungsstifte 105 und Langlöcher 107 vorgesehen. Der oberste und der mittlere Wandungsteilabschnitt 101 weisen jeweils wenigstens ein Langloch 107 an ihrem Umfang auf, in welche Führungsstifte 105, die am mittleren und am unteren Wandungsteilabschnitt 101 angeordnet sind, einschiebbar sind.

Die Anzahl an Wandungsteilabschnitten 101 ist dabei natürlich nicht auf drei beschränkt. Es können mehr oder weniger Wandungsteilabschnitte 101 vorgesehen sein.

Ferner können Arretierungseinrichtungen zum lösbaren Fixieren der relativ zueinander verschiebbaren Wandungsteilabschnitte 101 angeordnet sein, die in Fig. 17 nicht dargestellt sind. Ein Sicherungssplint kann beispielsweise in hierfür vorgesehene Öffnungen oder Bohrungen (in Fig. 17 nicht dargestellt) eingesteckt werden, wobei die Öffnungen beispielsweise senkrecht zur Bewegungsrichtung der verschiebbaren Wandungsabschnitte 101 und in diesen Wandungsabschnitten 101 angeordnet sein können. Weiterhin kann eine Arretiereinrichtung den Führungsstift 105 umfassen.

Beispielsweise kann der Führungsstift 105 drehbar oder kippbar angeordnet sein und in Langlöcher, die in einem vorgegebenen Winkel zur Bewegungsrichtung der verschiebbaren Wandungsabschnitte 101 angeordnet sind, eingeführt oder eingesteckt werden. Der Winkel kann beispielsweise senkrecht zur Bewegungsrichtung der verschiebbaren Wandungsabschnitte 101 angeordnet sein. Eine Arretierung der Wandungsabschnitte 101 kann im teilweise oder vollständig (wie in Fig. 17 dargestellt) ausgezogenen Zustand erfolgen, so dass ein ungewolltes Zusammenschieben der Wandungsabschnitte 101 verhindert werden kann. Eine Arretierung der Wandungsabschnitte 101 kann jedoch auch im zusammengeschobenen Zustand (wie in Fig. 18 dargestellt), oder im teilzusammengeschobenen Zustand erfolgen.

In der Ausführungsform der Fig. 17 sind die drei Wandungsteilabschnitte 101 in Längsrichtung L gleich lang. In anderen Ausführungsformen können die Längen unterschiedlich sein.

Ferner weist die Ausführungsform der Fig. 17 einen Schlauchabschnitt 103 auf, der als fluiddichte, insbesondere wasserdichte Membran oder Folie ausgeführt ist. Der Schlauchabschnitt 103 dichtet das Fluid F der Druckkammer DK gegen die Wandungsabschnitte 101 ab. Der Schlauchabschnitt 103 ist in diesem Ausführungsbeispiel rein exemplarisch am oberen Rand 7 des obersten Wandungsteilabschnitts 101 der Vorrichtung 100 und an der Bodenfläche 4a des untersten Wandungsteilabschnitts 101 befestigt. Ebenso kann der Schlauchabschnitt 103 beispielsweise mit seinem oberen Ende an der Innenseite des oberen Wandungsteilabschnitts 101 und/oder mit seinem unteren Ende an der Innenseite des unteren Wandungsteilabschnitts 101 befestigt sein. Andere Positionen zur Befestigung sind gleichfalls möglich.

Die Befestigung kann beispielsweise mittels einer mechanischen Befestigung, beispielsweise als Klemmung, mittels einer stoffschlüssigen Befestigung, beispielsweise mittels einer Klebung oder in einer anderen Weise ausgeführt sein.

Der Auslass 19' in dem unteren Wandungsteilabschnitt 101 ist mit der Druckkammer DK verbunden. Gegenüber den vorherigen Ausführungsformen durchstößt der Auslass 19' somit nicht nur die Seitenwand des Wandungsteilabschnitts 101, sondern ist durch die Wand, Membran oder Folie des Schlauchabschnitts 103 hindurch bis in die Druckkammer DK verlängert ausgeführt, so dass ein Fluidaustausch zwischen dem Äußeren und der Druckkammer DK erfolgen kann.

Der Auslass 19' kann beispielsweise mittels einer stoffschlüssigen Klebung mit dem Schlauchabschnitt 103 verbunden sein und so eine Fluidverbindung zwischen dem Inneren I und dem Äußeren der Vorrichtung 100 herstellen. Ungeachtet seiner Bezeichnung als Auslass kann dieser auch zum Einlassen von Fluid und damit zum Befüllen der Druckkammer DK verwendet werden.

Der übrige Aufbau entspricht im Wesentlichen der Vorrichtung 100 der in Fig. 3 dargestellten Ausführungsform, so dass auf diese Beschreibung verwiesen wird.

Der Schlauchabschnitt 103 liegt in Fig. 17 nicht den Wandungsteilabschnitten 101 an. Diese Darstellung dient allein der besseren Anschaulichkeit. In der Praxis wird der Fluiddruck des Fluids im Inneren I der Vorrichtung 100 den Schlauchabschnitt 101 radial nach außen gegen die Wandungsteilabschnitte 101 drücken.

Es sei ferner angemerkt, dass der Schlauchabschnitt 103 rein optional ist. Er dient der Abdichtung zwischen benachbarten Wandungsteilabschnitten 103. Sollten diese hingegen keine Abdichtung benötigen, so erübrigt sich der Schlauchabschnitt 103. Ebenso kann optional anstelle oder in Ergänzung des Schlauchabschnitts 103 auch jede andere Abdichteinrichtung vorgesehen sein, etwa Labyrinthdichtungen, Gummidichtungen, Reibdichtungen, oder dergleichen.

Fig. 18 zeigt die Vorrichtung der Fig. 17 in einem vollständig zusammengeschobenen Transportzustand. Gegenüber der Fig. 17 ist das Fluid F aus der Druckkammer DK durch den Auslass 19' entleert worden und die drei Wandungsteilabschnitte 101 ineinandergeschoben. Die Gesamtlänge L im in Fig. 18 gezeigten, zusammengeschobenen Transportzustand entspricht ca. einem Drittel der in Fig. 17 gezeigten Länge im vollständig auseinandergezogenen Zustand. Die erste Membran 5 und der Schlauchabschnitt 103 sind zusammengeschoben oder teilgefaltet.

Die Führungsstifte 105 sind im zusammengeschobenen Zustand in die Langlöcher 107 eingeführt.

Rein exemplarisch könnte die die Gesamtlänge im ausgezogenen Zustand (Fig. 17) beispielsweise ca. 50 cm betragen, und im zusammengeschobenen Zustand ca. 20 cm.

In einem exemplarischen, Ausführungsbeispiel kann ein Auseinanderschieben aus dem Transportzustand (Fig. 17) in den ausgezogenen Zustand oder Anwendungszustand der Fig. 16 dadurch erfolgen, dass das Fluid F durch den Einlass 19' in die Druckkammer DK eingefüllt wird. Das unter Druck eingeführte Fluid weitet den Raum der Druckkammer DK aus, wobei die Wandungsteilabschnitte 101 zunehmend vom in Fig. 18 gezeigten Zustand in den in Fig. 17 gezeigten Zustand übergehen. Mittels der Führungen der Führungsstifte 105 in den Langlöchern 107 können die Wandungsabschnitte 101 einfach und sicher ineinandergeschoben und auseinandergezogen werden. Ein Verkanten oder ein Verklemmen der Wandungsteilabschnitte 101 infolge eines nicht gerade in Längsrichtung L geführten Bewegens der Wandungsteilabschnitte 101 kann vorteilhaft verhindert werden.

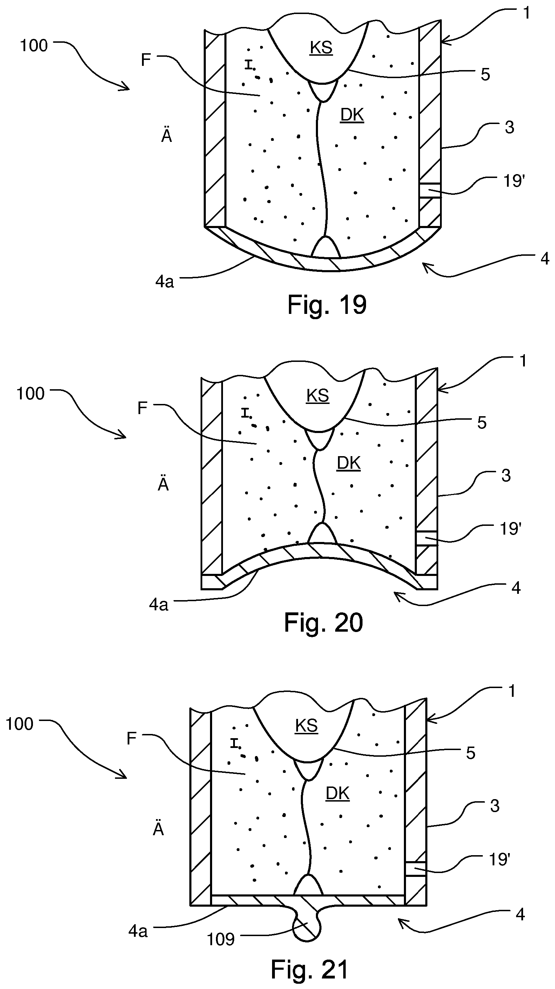

**Fig. 19** zeigt eine Vorrichtung 100 in einer fünfzehnten Ausführungsform mit einer nach außen gewölbten Bodenfläche 4a. Diese Form der Bodenfläche 4a kann als konvex Form bezeichnet werden.

Die nach außen gewölbte Bodenfläche 4a ermöglicht, vorzugsweise während der Anfertigung eines Gipsabdrucks oder eines Datenmodells des Körpergliedstumpfs KS, ein Abrollen oder Bewegen der medizinischen Vorrichtung. Somit kann beim Gipsabdruck nicht nur eine statische Belastung, wie sie ohne Bewegung der Vorrichtung auftritt, sondern auch eine dynamische Belastung erzeugt oder nachgestellt werden. Eine dynamische Belastung ermöglicht ein Einbeziehen oder ein Simulieren einer muskulären und/oder knöchernen Verschiebung die beim Gehen auftritt während des Ausfertigens eines Gipsabdrucks.

Die nach außen gewölbte Bodenfläche 4a ist in Fig. 19 exemplarisch als halbkugelförmige Kalotte in einer Schnittdarstellung dargestellt. Die Bodenfläche 4a kann alternativ andere Formen aufweisen, beispielsweise (teil-) zylinderförmig paraboloidförmige oder andere Wölbungsformen.

Die Wandung 3 ist in Fig. 19 einteilig dargestellt, kann jedoch ebenso mehrteilig ausgeführt sein, wie es in Fig. 17 und in Fig. 18 gezeigt ist. Ebenso kann das Ausführungsbeispiel einen Schlauchabschnitt 103 aufweisen, oder einzelne oder alle anderen vorstehend diskutierten Merkmale der anderen Ausführungsbeispiele.

**Fig. 20** zeigt eine Vorrichtung 100 in einer sechzehnten Ausführungsform mit einer nach innen gewölbten Bodenfläche 4a. Die Diskussion und Ausführungen zum Ausführungsbeispiel der Fig. 19 (konvexe Form der Bodenfläche 4a) gelten analog zu der Ausführungsform mit der innen gewölbten Bodenfläche 4a, die als konkave Form der Bodenfläche 4a bezeichnet werden kann.

Eine konvex geformte Bodenfläche 4a erfordert vorzugsweise eine besondere Form einer Auflagefläche, auf der sich die Vorrichtung beispielsweise kippbar, verdrehbar oder verschiebbar gegenüber der Auflagefläche bewegen kann. Beispielsweise eine kugelförmige Auflagefläche mit einem kleineren Radius gegenüber dem Radius der konkaven Form Bodenfläche 4a wäre eine bevorzugte Form einer Auflagefläche.

**Fig. 21** zeigt eine Vorrichtung 100 in einer siebzehnten Ausführungsform mit einem kugelgelenkförmigen oder zylindrischen Ansatz 109 in oder an der Bodenfläche 4a. Der Ansatz 109 ist als einteiliges Bauteil oder als integraler Bestandteil der Bodenfläche 4a ausgeführt. Der Ansatz 109 kann beispielsweise auf einem ebenen oder gewölbten Untergrund derart bewegt werden, dass sich die Vorrichtung in Kippbewegungen, Verdrehbewegungen oder Verschiebungen gegenüber dem Untergrund bewegen lässt und eine dynamische Belastung des Gipsabdrucks, wie zur Fig. 19 beschrieben, ermöglicht wird.

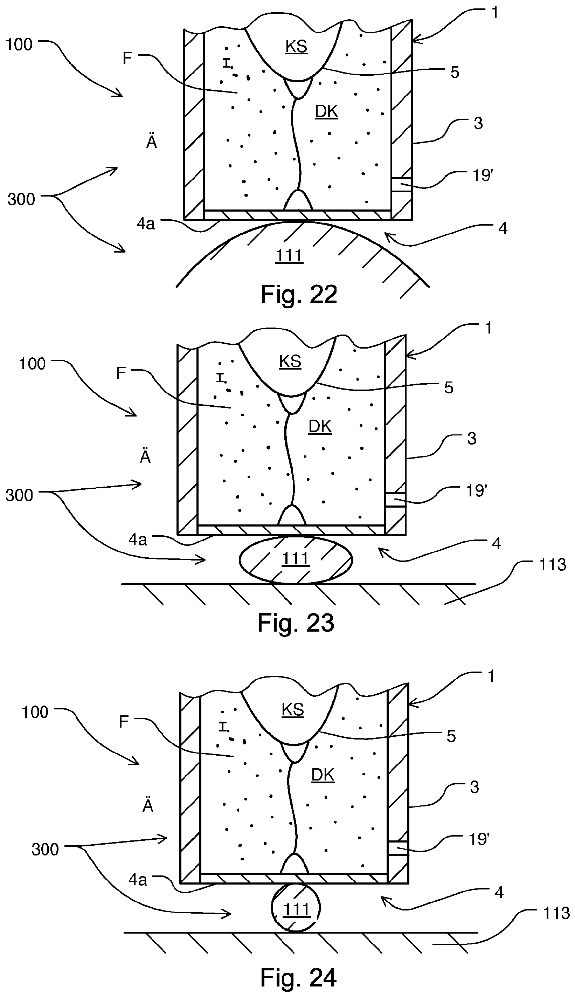

**Fig. 22** zeigt ein System oder Set 300 mit einer medizinischen Vorrichtung 100 und einer Auflagefläche 111. Die im Schnitt dargestellte Auflagefläche 111 kann eine dreidimensionale Form eines Paraboloids, einer Walze oder einer anderen Form aufweisen. Die Vorrichtung 100 kann auf der Auflagefläche 111 kippbar, verdrehbar oder verschiebbar gegenüber der Auflagefläche 111 bewegt werden, und somit eine dynamische Belastung, wie zur Fig. 19 beschrieben, erzeugt werden.

Die Auflagefläche 111 und ein mit dieser Auflagefläche 111 verbundener Körper kann ein Teil oder ein Abschnitt einer Bodenfläche oder eines Untergrunds sein. Ebenso kann der Körper der Auflagefläche 111 auf einem Untergrund angeordnet oder mit diesem, lösbar oder unlösbar, verbunden sein.

**Fig. 23** zeigt ein weiteres System 300 mit einer als Ellipsoid ausgestalteten Auflagefläche 111. Das Ellipsoid ist auf einem Untergrund 113 angeordnet. Vorzugsweise ist das Ellipsoid an oder auf dem Untergrund 113 fixiert, so dass bei einer Bewegung der Vorrichtung 100 auf der Auflagefläche 113 das Ellipsoid sich nicht selbst bewegt oder verschiebt.

**Fig. 24** zeigt ein weiteres System 300 mit einer als Kugel ausgestalteten Auflagefläche 111.

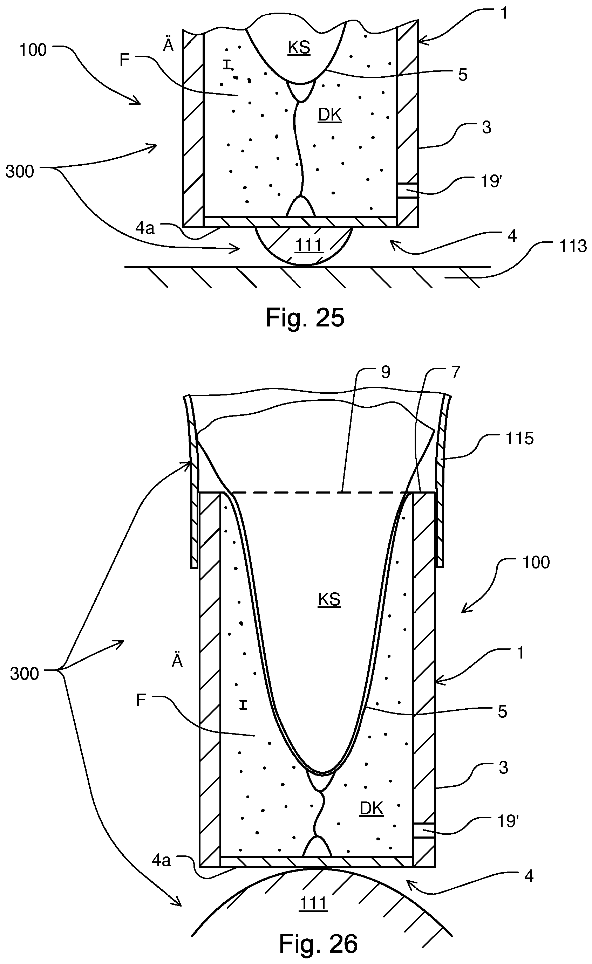

**Fig. 25** zeigt ein System 300 mit einer mit der Bodenfläche 4a der Vorrichtung 100 verbundenen Auflagefläche 111. Die Verbindung kann beispielsweise eine stoffschlüssige Verbindung, z. B. Klebung, oder eine formschlüssige Verbindung, z. B. mittels Scharnieren, sein. Die Verbindung kann eine Schraubverbindung oder eine andere Verbindung sein.

Das System 300 ist exemplarisch auf einem Untergrund 113 angeordnet. Das System 300 kann auf dem Untergrund 113 bewegt werden, beispielsweise kann es gekippt, verdreht oder verschoben werden.

**Fig. 26** zeigt ein System 300 mit einer Sicherungsmanschette 115, die schlauchförmig ausgebildet ist. Die Sicherungsmanschette 115 ist mit dem oberen Ende der Vorrichtung 100 verbunden, beispielsweise mittels einer Manschette, einer Klebung oder einer anderen Art von Befestigung. Die Sicherungsmanschette 115 umschließt einen nach oben aus der Einführöffnung 9 hinausragenden Teil des Körpergliedstumpfs KS. Dieses Umschließen sichert insbesondere den Körpergliedstumpf KS gegen ein ungewolltes Herausrutschen aus der Druckkammer DK bei einer Kippbewegung, Drehbewegung, Verschiebebewegung oder einer gemischt überlagerten Bewegung der genannten Bewegungen der Vorrichtung 100 auf der Auflagefläche 111.

Die Sicherung des Körpergliedstumpfs KS gegen ein ungewolltes Herausrutschen aus der Druckkammer DK kann zusätzlich durch ein Anlegen eines Unterdrucks in Zwischenraum zwischen der Sicherungsmanschette 115 und dem Körpergliedstumpf KS verbessert werden. Für diesen Zweck sind unter anderem das Material der Sicherungsmanschette 115, die Verbindung der Sicherungsmanschette 115 mit der Vorrichtung 100 und alle übrigen Verbindungsstellen vorzugsweise druckdicht und/oder fluiddicht ausgeführt.

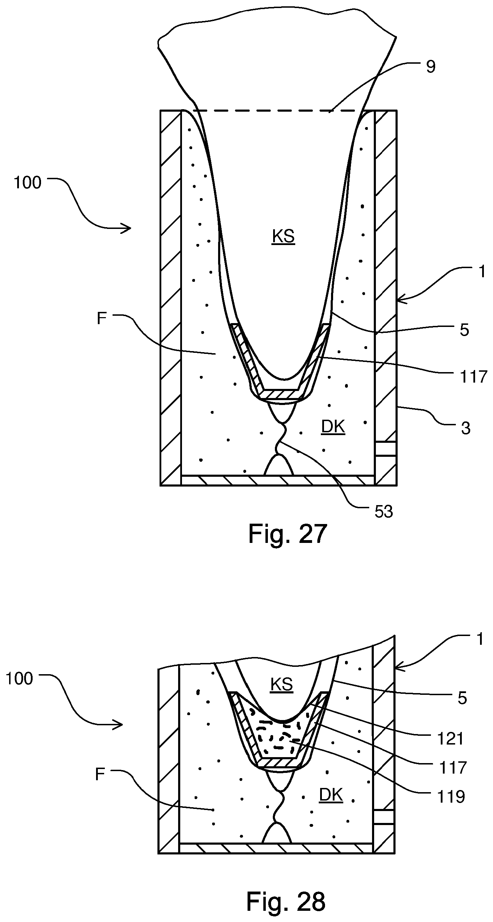

**Fig. 27** zeigt eine Vorrichtung 100 in einer achtzehnten Ausführungsform mit einem nach oben offenen Hohlgefäß 117, welches eine Entlastungseinrichtung zum Druckentlasten des distalen Bereichs des Körpergliedstumpfs KS ist. Das Hohlgefäß 117 weist eine becherartige Form auf. Die Druckentlastung wird durch den Hohlraum zwischen dem distalen Ende des Körpergliedstumpfs KS und dem Boden des Hohlgefäßes 117 erreicht. Dieser Hohlraum entlastet die empfindlichen Weichteile am distalen Ende des Körpergliedstumpfs KS, indem keine Druckbelastung durch das Fluid F auftritt, welche von manchen Patienten als unangenehm empfunden wird.

Das Hohlgefäß 117 wird vor dem Einbringen des Körpergliedstumpfs KS in die Einführöffnung 9 und in die Membran 5 hineingelegt oder hineingeschoben. Anschließend kann das Hohlgefäß 117 in dem unteren Bereich des Druckbehälters 1, etwa in unmittelbarer Nähe des Verbinders 53, positioniert werden. Nachfolgend kann der Körpergliedstumpf KS in den Druckbehälter 1 eingeführt werden. Dabei kann das Hohlgefäß 117 jedoch durch den Körpergliedstumpf KS selber in die Tiefe der Membran 5 geschoben werden.

**Fig. 28** zeigt eine Vorrichtung 100 mit einer weiteren Entlastungseinrichtung. Die Entlastungseinrichtung ist als Hohlgefäß 117 mit einem vorzugsweise weichen und kompressiblen oder komprimierbaren Material 119 gefüllt. Das Hohlgefäß 17 wird rein optional nach oben von einer Deckschicht 121 abgeschlossen, die als Membran ausgeführt sein kann. Das Material 119 kann offene oder geschlossene Poren aufweisen, und beispielsweise ein Schaumstoff sein.

In Fig. 28 berührt der Körpergliedstumpf KS den oberen Rand der Entlastungsvorrichtung nicht, er ruht auf dem Material 119. Dies ist allerdings rein exemplarisch zu verstehen. Der Rand kann auch in der in Fig. 28 gezeigten Ausführungsform zum Abstützen verwendet werden.

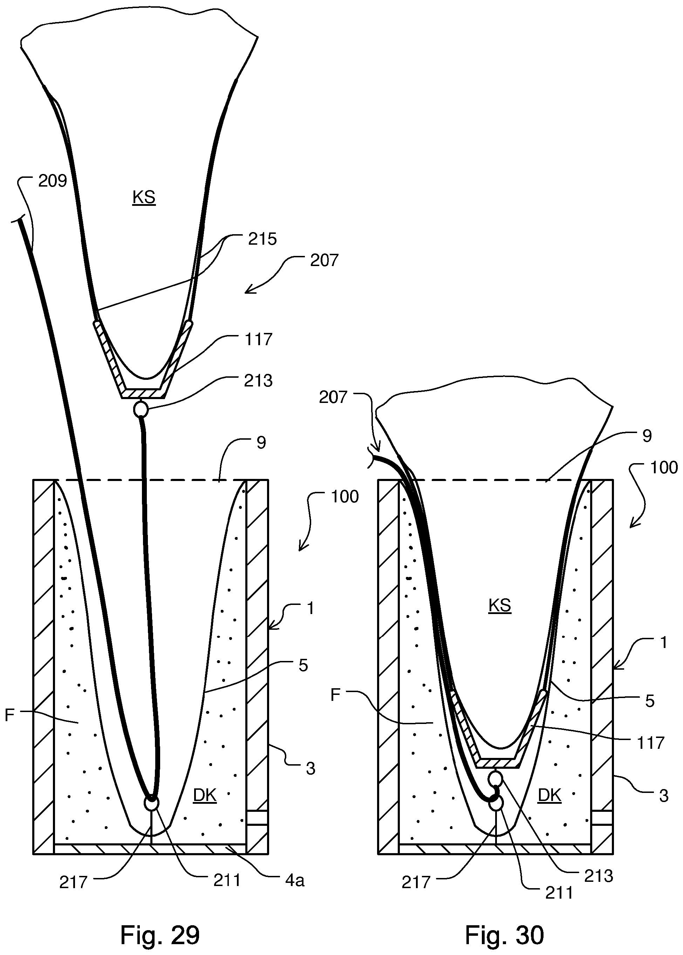

**Fig. 29** zeigt eine Vorrichtung 100 in einer zwanzigsten Ausführungsform mit einem Adapter 207 zum Fixieren des Körpergliedstumpfs KS im Druckbehälter 1.

Die Adapter 207 weist im Beispiel der Fig. 29 eine als Hohlgefäß 117 ausgestaltete Entlastungseinrichtung, einen Haftstrumpf 215, einen Gurt 209, einen Befestigungsring 213 und einen Umlenkring 211 auf. Der Gurt 209 kann gleichfalls ein Seil oder eine andere, geeignete Einrichtung zum Ziehen oder Halten sein. Der Adapter 207 könnte zusätzlich eine Einrichtung 223 zum Fixieren und/oder Verstellen des Gurtes 209 aufweisen.

Der Gurt 209 ist an seinem einen Ende an dem Hohlgefäß 117 mittels des Befestigungsrings 213 fixiert. Das Hohlgefäß 117 ist mit dem Haftstrumpf 215 (der hier als sogenannter Liner bezeichnet wird) verbunden. Der Haftstrumpf 215 kann beispielsweise am oberen Rand des Hohlgefäßes 117 befestigt sein, z. B. mittels Klebung oder einer mechanischen Fixierung, oder das gesamte Hohlgefäß 117 an dessen Außen- und/oder Innenseite umschließen (in Fig. 29 nicht dargestellt). Der Haftstrumpf 215 ist über den Körpergliedstumpf KS (oder dessen distales Ende) gestülpt oder diesem übergezogen.

Der Körpergliedstumpf KS kann vorzugsweise nicht ohne zusätzlichen Kraftaufwand aus dem Haftstrumpf 215, mit oder ohne Hohlgefäß 117, herausrutschen.

Das Hohlgefäß 117 stellt in dem in Fig. 29 gezeigten Beispiel gemeinsam mit dem Haftstrumpf 215 einen Teil des Adapters 207 dar. In anderen Ausführungsformen des Adapters 207 kann letzterer weitere Komponenten aufweisen, etwa den Gurt 209 und/oder den Befestigungsring 213. In wiederum anderen Ausführungsformen des Adapters 207 weist letzterer kein Hohlgefäß 117 auf, sondern beispielsweise nur den Haftstrumpf 215 und beispielsweise einen hieran befestigten Befestigungsring 213, mit oder ohne den Gurt 209.

Der Gurt 209 wird über den Umlenkring 211, der hier exemplarisch innerhalb des Druckbehälters 1 angeordnet ist, weiter nach außen geführt. Der Umlenkring 211 ist mittels eines gemeinsamen Verbinders 217 mit dem Boden des Druckbehälters 1 verbunden. Der gemeinsame Verbinder 217 fixiert gleichzeitig die Membran 5 am Boden des Druckbehälters 1.

Der gemeinsame Verbinder 217 kann in seiner Länge unterschiedlich ausgeführt sein. Je nach der ausgewählten Länge des Verbinders 217 kann der Körpergliedstumpf KS unterschiedlich weit in dem Druckbehälter 1 nahe an der Bodenfläche 4a positioniert werden. Der gemeinsame Verbinder 217 kann weiterhin in seiner Länge einstellbar sein, beispielsweise verschiedener Stufen, die an der Bodenfläche 4a fixiert und festgestellt werden können.

Die Membran 5 weist in Fig. 29 eine Sackform auf. Gleichfalls könnte die Membran 5 eine andere Form, beispielsweise eine Schlauchform, aufweisen.

Zum Erstellen oder Anfertigen eines Gipsabdrucks wird der Körpergliedstumpf KS, welcher im Adapter 207 steckt, also im Haftstrumpf 215 mit dem Hohlgefäß 117, zusammen mit dem Adapter 207 in den Druckbehälter 1 eingeführt. Gleichzeitig wird der Gurt 209 nachgeführt, wobei in der Regel nicht aktiv an dem Gurt 209 gezogen wird.

Nachdem der Körpergliedstumpf KS (zusammen mit dem Hohlgefäß 117 und dem Haftstrumpf 215) in gewünschter Tiefe im Druckbehälter 1 platziert ist (dargestellt in Fig. 30), kann der Körpergliedstumpf KS mittels der Zugvorrichtung 209 während des Aufbauens von Druck in der Druckkammer DK und während des Aushärtens des Gipsabdrucks fixiert oder im Druckbehälter 1 festgehalten werden. Dies kann beispielsweise mittels manueller Fixierung des äußeren Endes des Gurtes 209 erfolgen. Der Körpergliedstumpf KS wird vorteilhaft mittels des Adapters 207 daran gehindert, durch die auf ihr wirkenden, von der Membran 5 aufgebrachten Kräfte sowie durch ein Auftreiben aufgrund des Fluids entgegen der Eintrittsrichtung oder Längsrichtung L (siehe Fig. 8) bewegt zu werden.

Mittels des Adapters 207 kann der Körpergliedstumpf KS während des Anfertigens (oder Aushärtens) des Gipsabdrucks vorteilhaft in dem Druckbehälter 1 gehalten oder fixiert werden, ohne dass der Patient selbst ständig aktiv Druck auf den Körpergliedstumpf KS ausüben muss. Der Körpergliedstumpf KS wird auch bei höherem, in der Druckkammer DK herrschendem Druck im Druckbehälter gehalten. Er kann sich dem zum Erstellen eines passenden Gipsabdrucks erforderlichen Druck nicht nach axial entziehen.

**Fig.** 30 zeigt die Anordnung der Fig. 29 im fixierten Zustand des Körpergliedstumpfs KS im Druckbehälter 1.

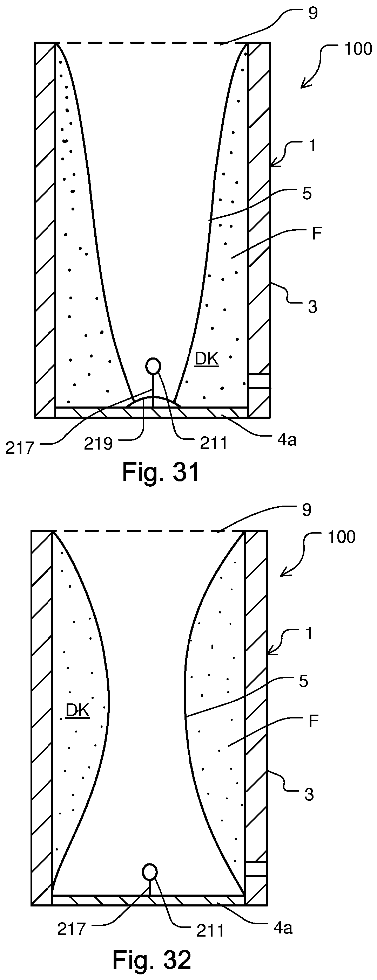

Fig. 31 zeigt die Vorrichtung 100 mit dem Druckbehälter 1 und einer schlauchförmigen Membran 5. Die schlauchförmige Membran 5 ist mittels eines optionalen Zwischenstücks 219 an der Bodenfläche 4a des Druckbehälters 1 fixiert.

Fig. 32 zeigt die Vorrichtung 100 mit dem Druckbehälter 1 und einer weiteren schlauchförmigen Membran 5. Die schlauchförmige Membran 5 ist am äußeren Rand der Bodenfläche 4a des Druckbehälters 1 fixiert oder befestigt. Der Verbinder 217 ist diesem Ausführungsbeispiel ohne einem Zwischenstück 219 (siehe Fig. 31) an der Bodenfläche 4a befestigt.

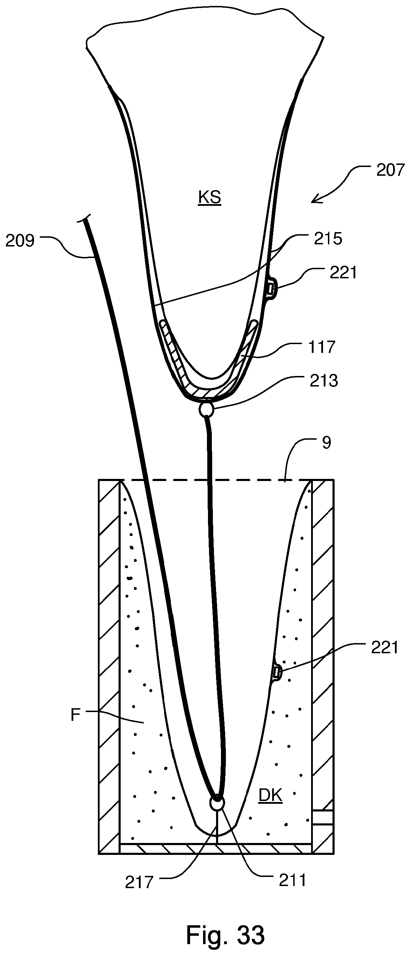

Fig. 33 zeigt die Vorrichtung 100 mit wenigstens einem Sensor 221, in diesem Ausführungsbeispiel mit zwei Sensoren 221 ausgeführt. Der wenigstens eine Sensor 221 kann mit dem Adapter 207 und/oder mit der Membran 5 verbunden sein. Insbesondere ist der Sensor 221 in den Haftstrumpf 215 und/oder in die Membran 5 integriert, beispielsweise in einem Zwischenraum einer doppelwandigen Ausführung des Haftstrumpfes 215 und/oder der Membran 5. Der Positionierung und die Anwendung des Sensors 221 ist ausdrücklich nicht auf die Ausführungsform der Fig. 33 beschränkt, sondern kann in jeder anderen beschriebenen Ausführungsform angewendet oder verwendet werden. Der Sensor 221 kann ein Sensor 221 zum Erfassen physiologischer Daten des Körpergliedstumpfs KS sein. Beispielsweise kann im Rahmen einer Oxymetrie mit einem Oxymeter als Sensor 221 der Sauerstoffgehalt des Blutes zur Überprüfung der Gefäßdurchblutung in dem Körpergliedstumpf KS ermittelt werden. Die Überprüfung der Gefäßdurchblutung in dem Körpergliedstumpf KS kann ebenso mit anderen Sensor-Messverfahren durchgeführt werden, beispielsweise mit dem Dopplerprinzip als Messverfahren oder dem Ultraschallprinzip.

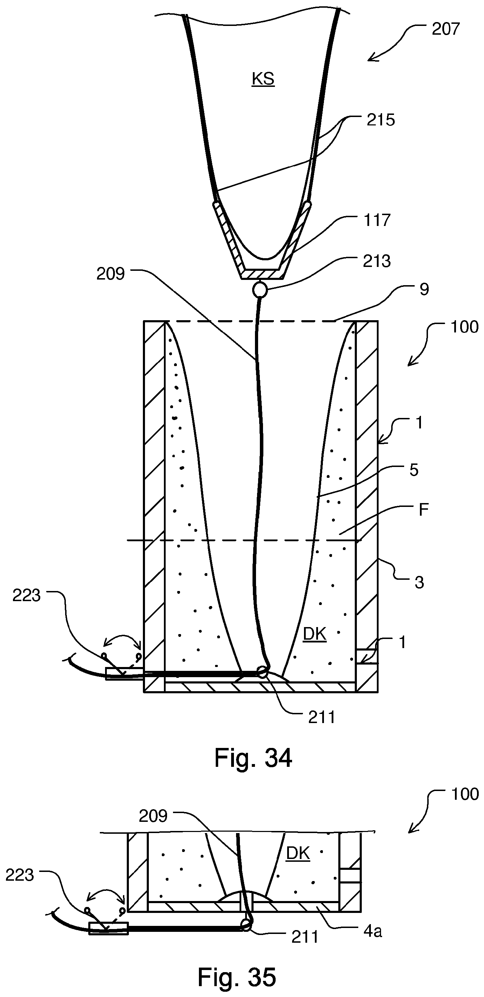

**Fig. 34** zeigt die Vorrichtung 100 mit einer Einrichtung 223 zum Fixieren und/oder Verstellen des Adapters 207. Die Zugvorrichtung des Adapters 207 kann der Gurt 209 sein.

Mittels der Einrichtung 223 kann der Gurt 209 fixiert werden, beispielsweise wenn der Körpergliedstumpf KS in dem Druckbehälter 1 fixiert werden soll (siehe Fig. 30). Ebenso kann der Gurt 209 mittels einer Einrichtung 223 zum Verstellen, insbesondere kontrolliert bewegt werden, beispielsweise mittels einer stufenförmigen Bewegung. Eine derartige Einrichtung kann als sogenannte "Ratsche" bezeichnet werden.

Fig. 35 zeigt einen Ausschnitt der Vorrichtung 100 mit einer weiteren Einrichtung 223 zum Fixieren und/oder Verstellen des Adapters 207. In diesem Ausführungsbeispiel wird der Gurt 209 über einen unterhalb der Bodenfläche 4a des Druckbehälters 1 angeordneten Umlenkring 211 nach außen zur Einrichtung 223 geführt. Damit kann vorteilhaft die Gurtanordnung und die Umlenkung mittels des Umlenkrings 211 außerhalb der Membran 5 und der Flüssigkeit F positioniert werden.

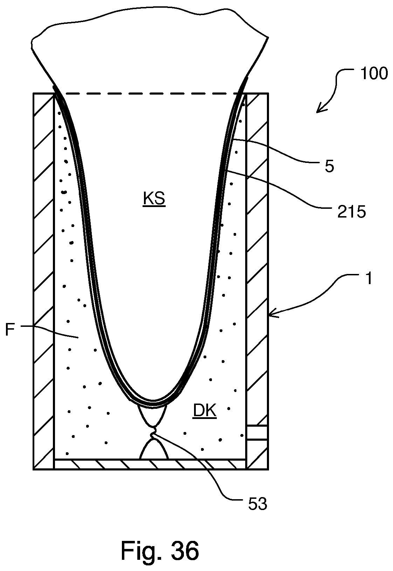

Fig. 36 zeigt ein mit einem Haftstrumpf 215 und einer Vorrichtung 100. Der Haftstrumpf 215 wurde vor dem Einführen des Körpergliedstumpfs KS in den Druckbehälter 1 über den Körpergliedstumpf KS übergezogen oder übergestülpt. In der Anordnung der Fig. 36 ist der Haftstrumpf 215 an seiner Außenseite reibschlüssig, beispielsweise durch eine aufgeraute, strukturierte, beschichtete und/oder oberflächenbehandelte Oberfläche, mit der Membran 5 kraftschlüssig verbunden. Die Membran 5 wird durch den Verbinder 53 in dem Druckbehälter 1 fixiert. Mittels dieser Anordnung kann der Körpergliedstumpf KS nicht ohne zusätzlichen Kraftaufwand aus dem Druckbehälter 1 herausrutschen oder sich ungewollt aus dem Druckbehälter 1 herausbewegen. Damit kann vorteilhaft ein Gipsabdruck oder ein Datenmodell eines Körpergliedstumpfs, insbesondere eines Unterschenkelstumpfs, angefertigt werden, ohne dass der Patient mit dem Körpergliedstumpf KS unvorhergesehene oder ungewollte Bewegungen während des Gipsabdrucks oder bei der Erstellung eines Datenmodells verursacht. Durch derartige Bewegungen kann die Erstellung des Gipsabdrucks oder des Datenmodells negativ beeinflusst werden.

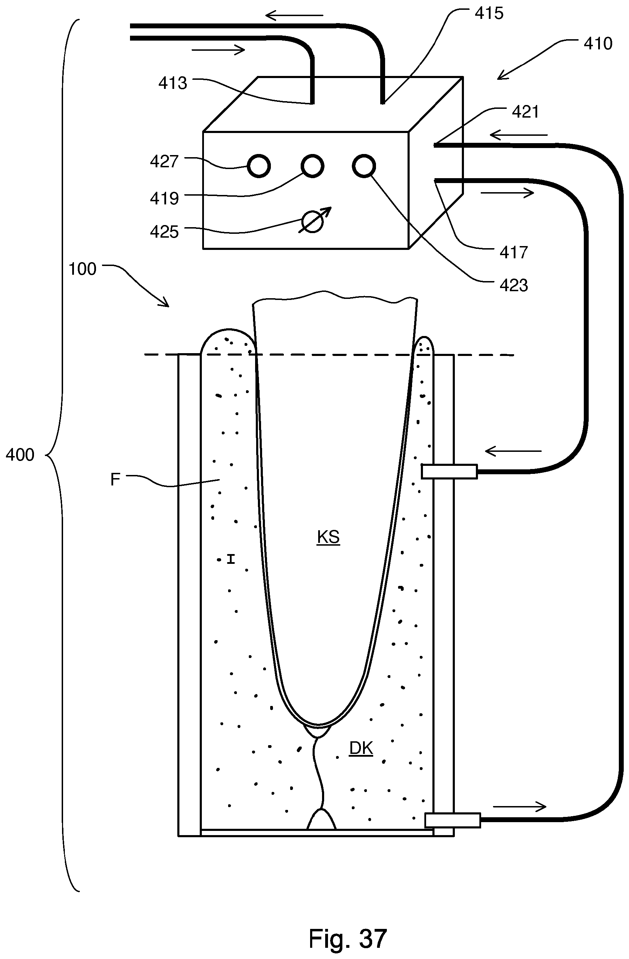

Fig. 37 zeigt eine erfindungsgemäße Einheit 400 mit einer erfindungsgemäßen medizinischen Vorrichtung 100 und einer erfindungsgemäßen Druckbeaufschlagungs-Steuervorrichtung 410. Die Druckbeaufschlagungs-Steuervorrichtung 410 wird im Folgenden abgekürzt als Steuervorrichtung 410 bezeichnet.

Die Steuervorrichtung 410 umfasst einen Druckreservoir-Anschluss 413, der als Anschluss an eine Druckquelle, beispielsweise an eine Wasserleitung, ausgeführt sein kann. Weiterhin umfasst die Steuervorrichtung 410 in der hier gezeigten Ausführungsform einen separaten Abfluss-Anschluss 415 zum Entleeren des Fluids aus der Druckkammer DK. Der Abfluss könnte alternativ beispielsweise mittels eines Mehrwegventils, welches an den Druckreservoir-Anschluss 413 angeschlossen werden kann, erfolgen.

Die Steuervorrichtung 410 der Fig. 37 umfasst weiterhin ein optionales Druckbegrenzungsventil, welches innerhalb der Steuervorrichtung 410 angeordnet ist (in Fig. 37 nicht dargestellt). Das Druckbegrenzungsventil kann den im Druckreservoir-Anschluss 413 herrschenden Druck begrenzen, so dass der stromab des Druckbegrenzungsventils anliegende Druck einen vorbestimmbaren oder optional vom Benutzer einstellbaren Wert nicht übersteigt. Damit kann vorteilhaft ein zu hoher Druck in der Druckkammer DK vermieden oder verhindert werden, der beispielsweise eine zu hohe Druckbelastung des Körpergliedstumpfs KS, und damit eine Schädigung und Schmerzen, ferner eine Verzerrung des Gipsabdrucks bedingen könnte. Das Druckbegrenzungsventil kann den Druck rein exemplarisch auf max. 0,8 bar begrenzen.

Die Steuervorrichtung 410 umfasst weiterhin einen Druckkammer-Anschluss 417, der mit einem Regelventil 419 zur Druckerhöhung in der Druckkammer DK verbunden ist oder ein solches aufweist. Das Regelventil 419 kann bei, beispielsweise manueller, Betätigung, den Druck in der Druckkammer DK, z. B. schrittweise, bis zu einem erwünschten oder bis zum maximalen Druck, der durch das Druckbegrenzungsventil limitiert ist, erhöhen.

Weiterhin umfasst die Steuervorrichtung 410 einen Druckkammer-Rückstromanschluss 421, der mit einem weiteren Regelventil 423 zur Druckverminderung in der Druckkammer DK verbunden ist oder ein solches aufweist. Das weitere Regelventil 423 kann, beispielsweise bei manueller Betätigung, den Druck in der Druckkammer DK z. B. schrittweise, je nach Bedarf, verringern. Nach dem Abschluss der Anfertigung des Gipsabdrucks kann der Druck in der Druckkammer DK soweit verringert werden, dass der Patient den Körpergliedstumpfs KS aus der Vorrichtung 100 herausziehen kann.

In der dargestellten Ausführungsform umfasst die Steuervorrichtung 410 weiterhin eine optionale Zulauf-Druckanzeige 425 und eine ebenfalls optionale Notaus-Einrichtung 427. Die Zulauf-Druckanzeige 425 kann als Manometer bezeichnet werden und als eine zusätzliche Anzeige, beispielsweise als Sicherheitsanzeige oder Redundanz-Anordnung zur Überwachung des Drucks in der Druckkammer DK, vorgesehen sein. Die Zulauf-Druckanzeige 425 ist beispielsweise mit, oder in, einer Verbindungsleitung in der Steuervorrichtung 410 zwischen dem Regelventil 419 (zur Druckerhöhung in der Druckkammer DK) und dem Druckkammer-Anschluss 417 angeordnet.

Ein Entleeren der Druckkammer DK mittels des Druckkammer-Rückstromanschlusses 421 und des Abfluss-Anschlusses 415, gesteuert über das weitere Regelventil 423, kann auf unterschiedliche Art und Weise erfolgen. Beispielsweise kann - z. B. stromab des Abfluss-Anschlusses 415 - eine Saugpumpe angeschossen werden, die bei einem Öffnen des weiteren Regelventils 423 Fluid F aus der Druckkammer DK heraussaugt und gegebenenfalls leersaugt. Ebenso kann - z. B. stromab des Abfluss-Anschlusses 415 - eine Venturi-Düse oder ein VenturiRohr zum Absaugen oder Leersaugen des Fluids F aus der Druckkammer DK angeschlossen werden. Eine Venturi-Düse kann beispielsweise an einen externen Wasseranschluss, beispielsweise an einen Wasserhahn an einem Waschbecken, angeschlossen werden. Wird der Wasserhahn geöffnet und fließt Wasser durch die Venturi-Düse, wird in einer mit dem Abfluss-Anschluss 415 verbundenen Leitung ein Unterdruck erzeugt und Fluid F, bei geöffnetem weiterem Regelventil 423, abgesaugt. Eine Venturi-Düse benötigt vorteilhaft keinen elektrischen Anschluss, wie dies bei einer Saugpumpe beispielsweise notwendig wäre.

Die hier beschriebene exemplarische Ausführungsform der Steuervorrichtung 410 benötigt vorteilhaft keine elektrischen Komponenten und somit keine Stromversorgung und keinen Stromanschluss.

In anderen Ausführungsformen als den hierin beschriebenen, besteht die Druckbeaufschlagungs-Steuervorrichtung aus einer Vorrichtung zum Aufbringen von Druck auf ein Fluidreservoir, etwa den Versorgungsbehälter 21 der Fig. 5, oder weist eine solche auf.

Angedacht ist hierzu beispielsweise, eine mechanische oder hydraulische Pressvorrichtung vorzusehen, mittels welcher das Fluid mit dem gewünschten, jedenfalls aber ausreichenden Druck aus dem Versorgungsbehälter 21 bzw. der Druckbeaufschlagungs-Steuervorrichtung ausgebracht werden kann.

Eine derartige Mechanik kann einen Kurbelmechanismus, einen Press- oder Klemmmechanismus mit Klemmflächen, eine Fussbetätigungsvorrichtung, deren Aufbau jenem eines Blasebalgs oder einer Fahrradpumpe entspricht oder ähnelt, oder dergleichen aufweisen. Mittels einer solchen Druckbeaufschlagungs-Steuervorrichtung kann einerseits der erforderliche Druck erzielt werden, andererseits ist der Benutzer der Vorrichtung nicht auf externe Druckquellen wie Wasseranschlüsse oder dergleichen angewiesen.

Die Druckbeaufschlagungs-Steuervorrichtung kann elektrisch betrieben werden. Dies ist jedoch nicht erforderlich. Sie kann vorgesehen sein, ohne Stromzufuhr zu arbeiten.

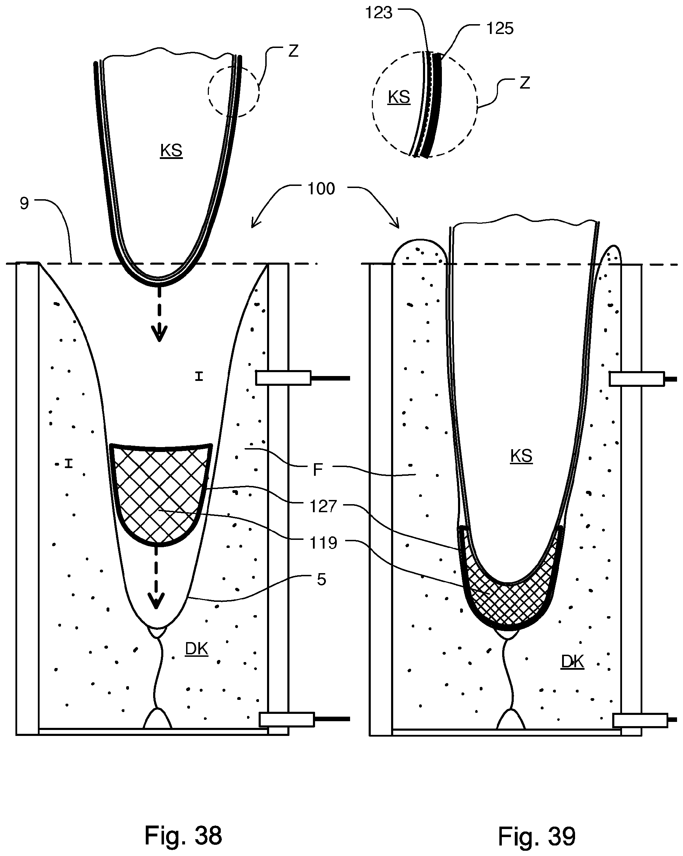

Fig. 38 zeigt eine medizinische Vorrichtung 100 mit einem Formkörper 127 zum Aufnehmen des distalen Bereichs des Körpergliedstumpfs KS, sowie einen Verbundstrumpf 123, gekennzeichnet in der Einzelteilvergrößerung Z.

Der Verbundstrumpf 123 ist auf den Körpergliedstumpf KS aufgezogen, übergezogen oder übergestülpt und liegt direkt an der Haut des Körpergliedstumpfs KS an. Über den Verbundstrumpf 123 wird außen die Gipsbinde 125 aufgetragen oder aufgewickelt. Optional kann in einem Zwischenschritt eine dünne Kunststofffolie über den Verbundstrumpf 123 aufgetragen werden, insbesondere um den Verbundstrumpf 123 vor dem feuchten Gips der Gipsbinde 125 zu schützen und zusätzliche spätere Reinigungsschritte zu vermeiden. Der Verbundstrumpf 123 weist an seiner zum Körpergliedstumpf KS hin orientierten Innenseite eine glatte oder glattere Oberfläche auf und auf seiner zur Gipsbinde 125 hin orientierten Außenseite eine raue oder rauere und/oder strukturierte oder strukturiertere Oberfläche auf. Die glatte Innenseite soll eine reibschlüssige Haftung oder Verbindung zum Körpergliedstumpf KS herstellen, um ein Verrutschen oder ein Herausrutschen des Körpergliedstumpf KS, insbesondere bei Druckaufbringung während des Gipsabdrucks in der Vorrichtung 100 und einer Druckerhöhung in der Druckkammer DK bis hin zum maximalen Druck, der durch das Druckbegrenzungsventil limitiert ist, sicherstellen. Die raue und/oder strukturierte Oberfläche des Verbundstrumpfs 123 soll eine möglichst gute und kraftschlüssige und/oder formschlüssige Verbindung zur Gipsbinde 125 sicherstellen.

Die Gipsbinde 125 ist bei ihrem Anlegen an den Körpergliedstumpf KS feucht und formbar, so dass sich die Innenseite des Gipsabdrucks an die raue und strukturierte Form anpassen und Teil des getrockneten Gipsabdrucks werden kann.

Der Formkörper 127 weist eine harte und formstabile Schale auf, deren Außenseite vorzugsweise glatt ist. Die Außenseite soll eine hohe Haftreibung des Formkörpers 127 an der Membran 5 im Einbauzustand sicherstellen und gewährleisten. Damit soll vorteilhaft ein Herausdrücken oder Herausschieben des Formkörpers 127, und damit des in dem Formkörper 127 angeordneten Körpergliedstumpfs KS, bei einer Druckaufbringung während des Anfertigens des Gipsabdrucks in der Vorrichtung 100 und einer Druckerhöhung in der Druckkammer DK bis hin zum maximalen Druck, der ggf. durch das Druckbegrenzungsventil limitiert ist, sicherstellen.

Im Formkörper 127 ist ein kompressibles, vorzugsweise weiches, Material 119 angeordnet. Das Material 119 kann beispielsweise Schaumstoff sein. Dieses Material 119 kann vorteilhaft den distalen Bereich des Körpergliedstumpfs KS schützen und druckentlasten, insbesondere bei einer Druckerhöhung in der Druckkammer DK bis hin zum maximalen Druck.

Der Formkörper 127 kann beispielsweise aus einem thermoplastischen Kunststoff herstellt sein oder einen solchen aufweisen. Thermoplastische Kunststoffe sind beispielsweise Acrylnitril-Butadien-Styrol (ABS), Polyamid (PA), Polylactat (PLA), Polymethylmethacrylat (PMMA), Polycarbonat (PC), Polyethylenterephthalat (PET), Polyethylen (PE), Polypropylen (PP), Polystyrol (PS), Polyetheretherketon (PEEK) oder Polyvinylchlorid (PVC). Ebenso kann der Formkörper 127 aus einem anderen Material, beispielsweise aus einem faserverstärkten Verbundwerkstoff, hergestellt sein oder einen solchen aufweisen.

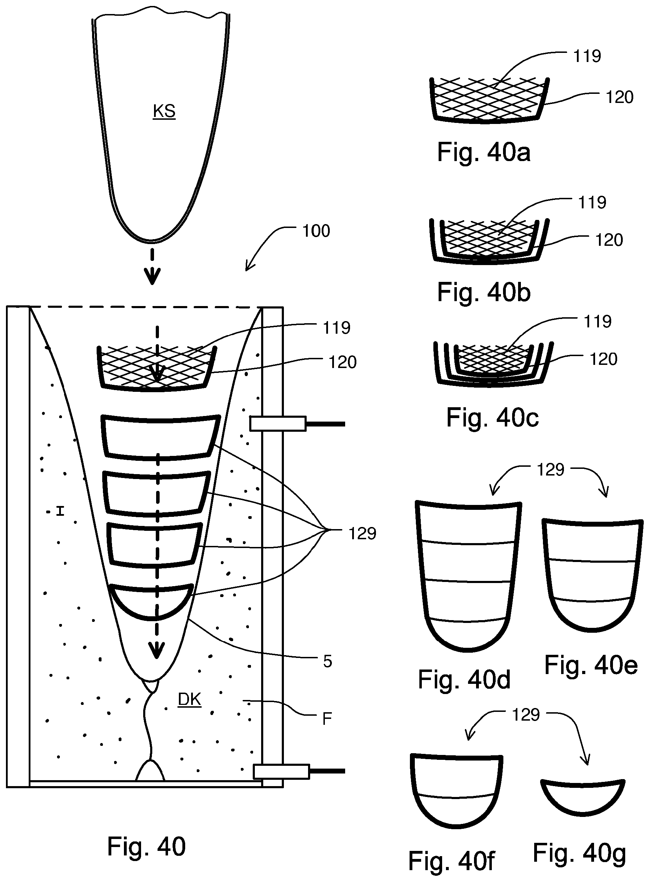

Die gestrichelten Pfeile der Fig. 40 zeigen die Richtung des Körpergliedstumpfs KS und des Formkörpers 127 bei seinem Einführen oder Einbringen in die Vorrichtung 100.

Fig. 39 zeigt die Anordnung der Fig. 38 im zusammengeführten Zustand. Das distale Ende des Körpergliedstumpfs KS ist in das kompressible Material des Formkörpers 127 eingeführt oder eingedrückt oder eingeschoben. Aufgrund des Drucks in der Druckkammer DK wölbt oder beult sich die Membran 5 oberhalb der Einführöffnung 9 (siehe Fig. 38) auf.

Fig. 40 zeigt eine Vorrichtung 100 mit einem mehrteiligen Formkörper 129 zum Aufnehmen des distalen Bereichs des Körpergliedstumpfs KS.

Mittels des mehrteiligen und modulförmigen Formkörpers 129 können unterschiedliche Längen des Körpergliedstumpfs KS ausgeglichen oder angepasst werden. Der grundsätzliche Aufbau und die Materialwahl ist analog dem Formkörper 127, der zur Fig. 39 ausführlich beschrieben wurde. Beispielsweise weisen die einzelnen Module des Formkörpers 129 glatte Oberflächen an der Außenseite auf, um eine hohe Haftreibung an der Membran 5 zu erzielen.

Das kompressible Material 119 hat die gleiche Funktion wie sie bereits in Fig. 39 beschrieben wurde. Das Volumen des Materials 119 kann je nach der endgültigen Größe und Höhe des modularen Formkörper 129 angepasst werden. Bei einem Schaumstoff als möglichem Material 119 kann das Volumen mittels Zuschneidens angepasst werden.

Analog zur Fig. 38 kann der Körpergliedstumpf KS mit einem Verbundstrumpf 123 und dessen weiter oben beschriebenen Eigenschaften und einer Gipsbinde 125 umhüllt werden. Der Einfachheit halber ist in Fig. 40 nur eine Umhüllung dargestellt.

Die gestrichelten Pfeile zeigen die Richtung des Körpergliedstumpfs KS, des Materials 119 und des mehrteiligen Formkörpers 129 bei einem Einführen oder Einbringen in die Vorrichtung 100.

**Fig. 40a****-c** zeigen unterschiedliche Größen eines kompressiblen Materials 119 und dazugehörige Schalen 120 zum Aufnehmen des distalen Bereichs des Körpergliedstumpfs KS. Das kompressible Material 119, beispielsweise Schaumstoff, kann vorgefertigt vorbereitet oder angepasst werden oder sein, beispielsweise mittels Zuschneidens. Vorgefertigte Materialien 119 können als konfektionierte Materialien 119 bezeichnet werden.

Die Schalen 120 können, wie in Fig. 40a, Fig. 40b und Fig. 40c dargestellt, ineinander gestapelt werden. Je nach Größe der innersten Schale 120 kann ein entsprechend großes kompressibles Material 119 eingelegt werden.

Unterschiedlich große Schalen 120 und das entsprechend der Größe eingelegte Material 119 können vorteilhaft für unterschiedlich große, distale Endmaße des Körpergliedstumpfs KS verwendet werden. Damit kann der Kompressionsdruck auf druckempfindliche, distale Bereiche des Körpergliedstumpfs KS angepasst werden.

Die Schalen 120 können an den mehrteiligen Formkörper 129 modulartig angepasst und zusammengeführt werden.

**Fig. 40d****-g** zeigen unterschiedliche Längen eines mehrteiligen Formkörpers 129 zum Längenausgleich unterschiedlich langer Körpergliedstümpfe KS. Das unterste Modul ist kalottenförmig zum Einlegen in die Membran 5 ausgestaltet. Die daran nach oben sich modulförmig anschließenden Formkörper 129 können je nach Länge des Körpergliedstumpfs KS angepasst werden. Die einzelnen, sich modular oder baukastenartig anschließenden Formkörper 129 können als Scheiben oder scheibenförmig bezeichnet werden. Sie können mittels Paßsitze einander angepasst werden.

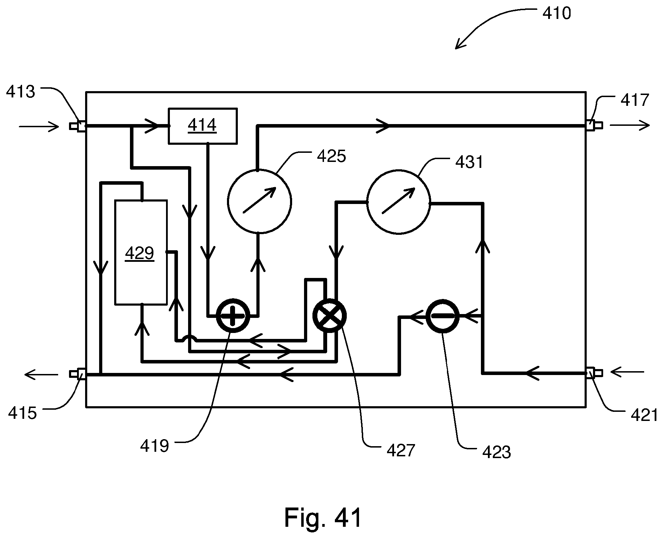

**Fig. 41** zeigt eine schematische Anordnung von Bauteilen innerhalb der Druckbeaufschlagungs-Steuervorrichtung 410 einer exemplarischen Ausführungsform.

Ergänzend zu der Beschreibung zur Figur 37 werden nachfolgend nur zusätzliche Bauteile oder Funktionen beschrieben.

Als eintretendes Fluid am Anschluss 413 wird im Folgendes Wasser beschrieben, ohne jedoch hierdurch auf Wasser als Fluid beschränkt zu sein.

Das eintretende Wasser kann zunächst in das optionale Druckbegrenzungsventil 414 eintreten bzw. einfließen. Das Druckbegrenzungsventil 414 kann einen am Anschluss 413 anliegenden Wasserdruck begrenzen oder reduzieren. Rein exemplarisch kann der Wasserdruck am Anschluss 413 8 bar oder 10 bar betragen und mittels des Druckbegrenzungsventils 414 auf beispielsweise 2 bar oder 1 bar reduziert werden.

Stromab des Druckbegrenzungsventils 414 fließt das Wasser über eine Leitung in ein erstes Regelventil 419 ein (oder durch dieses hindurch), mit dem die Leitung beispielsweise je nach Bedarf geöffnet und (insbesondere ganz oder teilweise) geschlossen werden kann. Damit kann die Zufuhr des Wassers in die nicht gezeigte Druckkammer DK der Vorrichtung 100 bzw. das Befüllen der Vorrichtung 100 gesteuert werden. Die Steuerung kann beispielsweise manuell mittels Betätigung des Regelventils 419 erfolgen.

Weiter stromab ist optional eine Zulauf-Druckanzeige 425 angeordnet. Bei geöffnetem Regelventil 419 wird der Wasserdruck stromab des Druckbegrenzungsventil 414 angezeigt. Bei geschlossenem Regelventil 419 wird der Druck in der Druckkammer DK des Druckbehälters 1 angezeigt. Weiter stromab fließt das Wasser über den Druckkammer-Anschluss 417 zur nicht gezeigten Druckkammer DK.

Ein weiteres Regelventil 423 regelt in gleicher Weise durch Öffnen und Schließen den Durchlauf zwischen dem von der Druckkammer DK rückführenden Druckkammer-Rückstromanschluss 421 und dem Abfluss-Anschluss 415.

Vor dem Druckbegrenzungsventil 414 ist eine optionale Leitungsabzweigung zu einer Notaus-Einrichtung 427 und weiter stromab (unterer Abzweig an der Notaus-Einrichtung 427) zu einer wiederum optionalen Venturi-Düse 429 dargestellt. Die Notaus-Einrichtung 427 ist in der Regel immer offen, es sei denn, sie wird im Notfall betätigt und geschlossen. Weiter stromab, aus der Venturi-Düse 429 abgehend, fließt das Wasser weiter zum Abfluss-Anschluss 415. Innerhalb oder mittels der Venturi-Düse 429 wird ein Unterdruck erzeugt (beispielsweise mittels einer Laval-Düse). An diesen Unterdruck wird eine weitere Leitung zum Absaugen von Wasser aus der Druckkammer DK angeschlossen. Dieser Anschluss zweigt zunächst stromauf des weiteren Regelventils 423 ab und führt zu einem Unterdruck-Anzeiger 431, beispielsweise einem Manometer. Am Ausgang des Unterdruck-Anzeigers 431 führt diese Leitung weiter, optional über die Notaus-Einrichtung 427, zur Venturi-Düse 429 am zuvor beschriebenen Unterdruck-Anschluss.

Die Venturi-Düse 429 mit den beschriebenen Anschlussleitungen kann bei Bedarf (zu-)geschaltet oder angeschaltet werden, um die Druckkammer DK zu entleeren. Dies wird beispielsweise durch optionale Absperrventile oder ähnliches realisiert, die jedoch aus Gründen der Übersichtlichkeit in Fig. 41 nicht eingezeichnet sind.

Mittels der Venturi-Düse 429 kann die Druckkammer DK vorteilhaft ohne die Druckbeaufschlagungs-Steuervorrichtung 410 mit einer Quelle für elektrischen Strom/elektrische Spannung verbinden zu müssen, vorteilhaft entleert werden.

Die Druckbeaufschlagungs-Steuervorrichtung 410 der Fig. 41 sowie anderer Ausführungsformen weist keinen (elektrischen) Stromanschluss auf.

### Bezugszeichenliste

- 100: Vorrichtung
- 1: Druckbehälter
- 2: erste Stirnseite
- 3: Wandung
- 4: zweite Stirnseite, kann optional mit der Bodenfläche verschlossen sein
- 4a: Bodenfläche
- 5: Membran
- 7: oberer Rand
- 9: Einführöffnung
- 11: Klemmring
- 13: Begrenzungsring oder Oberschenkeldichtring
- 15: Beinöffnung
- 17: Schrauben
- 19, 19': Auslass, Einlass
- 21: Vorratsbehälter
- 23: Füße oder Aufstellvorrichtung
- 25: Deckel
- 27: Spiegel des Fluids/Fluidspiegel
- 29: Einlass
- 31: Druckleitung
- 33: Druckquelle
- 35: Ausgleichsleitung, Fluidverbindung
- 37: Klemme
- 39: Aufstellfläche oder Untergrund
- 41: Rolleneinrichtung
- 43: Stütze
- 45: Haltegriff
- 47: Luftablassöffnung
- 51: Unterer Abschnitt der Wandung
- 53: Verbinder
- 55: Längsfaser
- 55a: Längsfaser
- 55b: Längsfaser
- 57: Umfangsfaser oder Querfaser
- 57a: Umfangsfaser oder Querfaser
- 57b: Umfangsfaser oder Querfaser
- 58: zentraler oder mittlerer Bereich oder Abschnitt der Membran
- 61: Unterer Abschnitt der Membran
- 63: Schale
- 65: Gewinde
- 67: Sockel
- 71: Kamera als Bespiel einer Einrichtung zum Vermessen, Abtasten oder Erfassen
- 73: Einrichtung zum Erstellen oder Errechnen eines Modells des Körpergliedstumpfs oder eines zu fertigenden Schafts für den Körpergliedstumpf
- 75: Formgebungs-Vorrichtung
- 81: Auftreibung der Membran
- 83: Kontaktpunktfläche
- 85: Übergangsabschnitt
- 91: Drucksensor
- 93: Anzeigevorrichtung
- 95: Auswertevorrichtung
- 97: Ermittlungsvorrichtung
- 99: Anzeigevorrichtung
- 101: Wandungsteilabschnitt
- 103: Schlauchabschnitt
- 105: Führungsstift
- 107: Langloch
- 109: kugelförmiger Ansatz
- 111: Auflagefläche
- 113: Untergrund
- 115: Sicherungsmanschette
- 117: Hohlgefäß
- 119: Material
- 120: Schale
- 121: Deckschicht
- 123: Verbundstrumpf
- 125: Gipsbinde
- 127: Formkörper
- 129: mehrteiliger Formkörper
- 200: Berechnungsvorrichtung
- 201: Eingabevorrichtung
- 203: Ausgabevorrichtung
- 205: Referenzdaten
- 207: Adapter
- 209: Gurt
- 211: Umlenkring
- 213: Befestigungsring
- 215: Haftstrumpf
- 217: gemeinsamer Verbinder
- 219: Zwischenstück
- 221: Sensor
- 223: Einrichtung zum Fixieren und/oder Verstellen
- 300: System mit Vorrichtung/ Auflagefläche/ Sicherungsmanschette
- 400: Einheit
- 410: Druckbeaufschlagungs-Steuervorrichtung
- 413: Druckreservoir-Anschluss
- 414: Druckbegrenzungsventil
- 415: Abfluss-Anschluss
- 417: Druckkammer-Anschluss
- 419: Regelventil
- 421: Druckkammer-Rückstromanschluss
- 423: weiteres Regelventil
- 425: Zulauf-Druckanzeige
- 427: Notaus-Einrichtung
- 429: Venturi-Düse
- 431: Unterdruck-Anzeiger
- DK: Druckkammer des Druckbehälters
- I: Inneres des Druckbehälters
- II: Inneres des Vorratsbehälters
- F: Fluid oder Flüssigkeit
- Ä: Äußeres des Druckbehälters
- ÄÄ: Äußeres des Vorratsbehälters
- KS: Körpergliedstumpf
- L: Längsrichtung
- Q: Querrichtung

## Patentansprüche

1. Medizinische Vorrichtung (100) zur Verwendung bei der Anfertigung eines Gipsabdrucks oder eines Datenmodells eines Körpergliedstumpfs, insbesondere eines Unterschenkelstumpfs, wobei die Vorrichtung (100) wenigstens aufweist:
- einen Druckbehälter (1) mit einer Fluidkammer oder einer Druckkammer zur Aufnahme oder Speicherung eines unter Druck stehenden, Fluids wobei der Druckbehälter (1) eine Wandung (3) aus einem ersten Material aufweist, wobei die Wandung (3) ein Inneres des Druckbehälters (1) gegenüber einem Äußeren begrenzt, wobei der Druckbehälter (1) eine Einführöffnung (9) zum Einführen des Körpergliedstumpfs in das Innere des Druckbehälters (1) aufweist;
- eine fluiddichte Membran (5) aus einem zweiten Material, welche angeordnet ist, um die Fluidkammer oder die Druckkammer zu bilden oder zu begrenzen; und welche direkt oder indirekt in fluiddichter Verbindung mit Abschnitten der Wandung (3) des Druckbehälters (1) verbunden ist; sowie wenigstens einen Verbinder, wobei wenigstens ein Abschnitt (58) der Membran (5) mittels wenigstens des besagten Verbinders (53) an einem Abschnitt der Wandung (3), vorzugsweise lösbar, befestigt ist, **dadurch gekennzeichnet, dass** der Verbinder (53) mit der Membran (5) in einem Abschnitt der Membran (5) verbunden ist, welcher nicht im Bereich der Einführöffnung (9) liegt.

2. Medizinische Vorrichtung (100) nach einem der vorangegangenen Ansprüche, wobei wenigstens ein, vorzugsweise zentraler oder mittlerer, Abschnitt (58) der Membran (5) mittels wenigstens eines Verbinders (53), vorzugsweise lösbar, an einer Bodenfläche (4a) oder an der zweiten Stirnseite (4) des Druckbehälters (1), und vorzugsweise in einem mittleren oder zentralen Bereich oder Abschnitt hiervon, befestigt ist.

3. Medizinische Vorrichtung (100) nach einem der Ansprüche 1 oder **2,** wobei der Verbinder (53) nicht elastisch und/oder nicht dehnbar ist.

4. Medizinische Vorrichtung (100) nach einem der vorangegangenen Ansprüche, wobei der Verbinder (53), die Membran (5) und/oder die Bodenfläche (4a) wenigstens ein Gewinde (65) zum Verschrauben der Membran (5) oder eines hiermit verbundenen Elements, direkt oder indirekt, mit dem Druckbehälter (1) aufweist.

5. Medizinische Vorrichtung (100) nach einem der vorangegangenen Ansprüche, wobei die Membran (5) aus einem Material gefertigt ist oder ein solches aufweist, welches in einer ersten Richtung und/oder in einer zweiten Richtung des Materials Fasern (55a, 57a; 55b, 57b) aufweist, welche vorzugsweise in eine Matrix eingebettet oder vorzugsweise mit dieser auf andere Weise verbunden sind.

6. Medizinische Vorrichtung (100) nach einem der vorangegangenen Ansprüche, wobei die Membran (5) in einer ersten Richtung hiervon und/oder in einer zweiten Richtung hiervon nicht dehnbar oder nicht elastisch ist.

7. Set mit
- wenigstens einer medizinischen Vorrichtung (100) nach einem der Ansprüche 1 bis 6; und
- wenigstens zwei Membranen (5), welche sich in wenigstens einer Eigenschaft voneinander unterscheiden, und/oder wenigstens einem Gewicht zum vorübergehenden Erhöhen oder Beaufschlagen des Körpergewichts des Patienten und/oder wenigstens einer Entlastungseinrichtung, und/oder wenigstens einem Adapter (207) zum lösbaren Verbinden oder Fixieren des Körpergliedstumpfs mit dem oder am Druckbehälter (1); und
- optional wenigstens einem Haftstrumpf (215) zum Überziehen und/oder Überstülpen über den Körpergliedstumpf wobei der mit dem Körpergliedstumpf verbundene Haftstrumpf (215) kraftschlüssig und/oder reibschlüssig mit einer Oberfläche, insbesondere mit der Oberfläche der Membran (5), am oder im Druckbehälter (1) verbunden ist.

8. Verfahren zum Anpassen eines Gipsabdrucks an einen Körpergliedstumpf oder zum Vermessen des Körpergliedstumpfs eines Patienten, mit den Schritten:
- Bereitstellen einer medizinischen Vorrichtung (100) nach einem der Ansprüche 1 bis 6 und optional wenigstens einer Auflagefläche (111), wobei die Vorrichtung (100), wenn sie auf der Auflagefläche (111) aufliegt, relativ zur Auflagefläche (111) kippbar, verdrehbar und/oder verschiebbar ist;
- und . Befüllen der Fluidkammer oder Druckkammer des Druckbehälters (1) mit einem Fluid, insbesondere einer Flüssigkeit oder Verändern eines Flüssigkeitsspiegels innerhalb der Fluidkammer oder Druckkammer derart, dass die Membran (5) zumindest in Abschnitten hiervon um den gesamten Umfang dieser Abschnitte herum von der Flüssigkeit bedeckt ist, oder derart, dass sich die Membran (5) über die Einführöffnung (9) des Druckbehälters (1) hinaus in das Äußere des Druckbehälters (1) wölbt.

9. Verfahren zum Anpassen eines Gipsabdrucks an einen Körpergliedstumpf oder zum Vermessen des Körpergliedstumpfs eines Patienten, insbesondere nach Anspruch 8, mit den Schritten:
- Bereitstellen einer medizinischen Vorrichtung (100) nach einem der Ansprüche 1 bis 6 oder eines Sets nach Anspruch 7;
- Einführen des optional mit einer feuchten Gipsbinde (125) umwickelten Körpergliedstumpfs in die Membran (5) derart, dass der Körpergliedstumpf zumindest in Abschnitten hiervon um seinen gesamten Umfang herum von der Membran (5) umgeben ist;
- Ermitteln des Drucks, welcher auf die Membran (5), in der Druckkammer auf einem Abschnitt der Oberfläche des von der Membran (5) im Gebrauch der Vorrichtung (100) bedeckten Körpergliedstumpfs und/oder zwischen Membran (5) und Körpergliedstumpf herrscht, mittels einer Druckmessvorrichtung;
- Ermitteln eines Gewichts, mit welchem der Patient, dessen Körpergliedstumpf in die medizinische Vorrichtung (100) eingeführt ist, beschwert werden muss, damit der mittels der Druckmessvorrichtung gemessene Druck in einem vorbestimmten Zielwertebereich liegt.

10. Verfahren zum Ermitteln eines Zusatzgewichts beim Anpassen eines Gipsabdrucks an einen Körpergliedstumpf oder beim Vermessen des Körpergliedstumpfs eines Patienten, mit den Schritten:
- Bereitstellen von patientenbezogenen Daten;
- Eingeben der patientenbezogenen Daten in eine Ermittlungsvorrichtung (97);
- Ermitteln unter Bezugnahme der patientenbezogenen Daten, eines Gewichts, mit welchem der Patient, dessen Körpergliedstumpf in die medizinische Vorrichtung (100), nach einem der Ansprüche 1 bis 6, eingeführt ist, beschwert werden muss, damit ein Druck, welcher auf der Membran (5), in der Druckkammer, auf einem Abschnitt der Oberfläche des von der Membran (5) im Gebrauch der Vorrichtung (100) bedeckten Körpergliedstumpfs und/oder zwischen Membran (5) und Körpergliedstumpf herrscht, in einem vorbestimmten Zielwertebereich liegt.

11. Berechnungsvorrichtung (200), programmiert, konfiguriert und/oder eingerichtet, um ein Verfahren nach Anspruch 10 durchzuführen.

12. Einheit (400), umfassend wenigstens eine medizinische Vorrichtung (100) nach einem der Ansprüche 1 bis 6, und wenigstens eine Druckbeaufschlagungs-Steuervorrichtung (410) für die Druckkammer der medizinischen Vorrichtung (100), wobei die Druckbeaufschlagungs-Steuervorrichtung (410) vorzugsweise wenigstens aufweist:
- einen Druckreservoir-Anschluss (413);
- ein Druckbegrenzungsventil;
- wenigstens einen Anschluss (417, 421) an die Druckkammer mit einem Regelventil (419, 423) zur Druckerhöhung und/oder mit einem Regelventil (419, 423) zur Druckverminderung in der Druckkammer,
- optional: eine Druckanzeige (425);
- optional: eine Not-Aus-Einrichtung (427),
- optional: eine Saug-Düse,
wobei die Druckbeaufschlagungs-Steuervorrichtung (410) optional keine elektrischen Komponenten und/oder keinen Stromanschluss aufweist.

## Claims

1. A medical apparatus (100) for use in the fabrication of a plaster impression or of a data model of a limb stump, in particular a lower limb stump, the apparatus (100) comprising at least:
- a pressure vessel (1) with a fluid chamber or a pressure chamber for receiving or storing a fluid under pressure, the pressure vessel (1) comprising a wall (3) made of a first material, the wall (3) delimiting an interior of the pressure vessel (1) relative to an exterior, the pressure vessel (1) comprising an insertion opening (9) for inserting the limb stump into the interior of the pressure vessel (1);
- a fluid-tight membrane (5) made of a second material, which is arranged to form or delimit the fluid chamber or the pressure chamber; and which has a direct or indirect fluid-tight connection to portions of the wall (3) or the pressure vessel (1); and at least one connector,
wherein at least one portion (58) of the membrane (5) is fixed, preferably detachably, to a portion of the wall (3) by means of the connector (53), **characterized in that** the connector (53) is connected to the membrane (5) in a portion of the membrane (5) that is not located in the region of the insertion opening (9).

2. The medical apparatus (100) according to any one of the preceding claims, wherein at least one, preferably central or median, portion (58) of the membrane (5) is fixed, preferably detachably, to a bottom surface (4a) or to the second front side (4) of the pressure vessel (1) by means of at least one connector (53), and preferably in a median or central region or portion thereof.

3. The medical apparatus (100) according to any one of the claims 1 or 2, wherein the connector (53) is not elastic and/or not stretchable.

4. The medical apparatus (100) according to any one of the preceding claims, wherein the connector (53), the membrane (5) and/or the bottom surface (4a) comprise/s at least one thread (65) for screwing, directly or indirectly, the membrane (5) or an element connected thereto, to the pressure vessel (1).

5. The medical apparatus (100) according to any one of the preceding claims, wherein the membrane (5) is made of or comprises a material which, in a first direction and/or in a second direction of the material, comprises fibres (55a, 57a, 55b, 57b) being preferably embedded into a matrix or being preferably connected thereto by another way.

6. The medical apparatus (100) according to any one of the preceding claims, wherein the membrane (5) is not stretchable or elastic in a first direction and/or in a second direction thereof.

7. A kit comprising
- at least one medical apparatus (100) according to any one of the claims 1 to 6; and
- at least two membranes (5) that differ from one another in at least one characteristic, and/or at least one weight for temporarily increasing or applying the patient's body weight, and/or at least one relief device, and/or at least one adapter (207) for detachably connecting or securing the limb stump to or on the pressure vessel (1); and
- optionally, at least one adhesive sleeve (215) for pulling over and/or slipping onto the limb stump, wherein the adhesive sleeve connected to the limb stump (215) is connected by force and/or friction to a surface-in particular, the surface of the membrane (5)-on or inside the pressure vessel (1).

8. A method for fitting a plaster impression to a limb stump or for measuring a limb stump of a patient, comprising the steps of:
- providing a medical apparatus (100) according to any one of claims 1 through 6 and, optionally, at least one support surface (111), wherein the apparatus (100), when resting on the support surface (111), is capable of tilting, rotating, and/or sliding relative to the support surface (111);
- and filling the fluid chamber or pressure chamber of the pressure vessel (1) with a fluid, in particular a liquid, or changing a liquid level within the fluid chamber or pressure chamber such that the membrane (5) is covered by the liquid, at least in portions thereof, around the entire circumference of these portions, or such that the membrane (5) bulges outward beyond the insertion opening (9) of the pressure vessel (1) into the exterior of the pressure vessel (1).

9. A method for fitting a plaster impression to a limb stump or for measuring a limb stump of a patient, in particular according to claim 8, comprising the steps of:
- providing a medical apparatus (100) according to any one of the claims 1 to 6 or a kit according to claim 7;
- inserting the limb stump, which may optionally be wrapped in a damp plaster bandage (125), into the membrane (5) in such a way that the limb stump is surrounded by the membrane (5) around its entire circumference, at least in portions;
- determining the pressure acting on the membrane (5), in the pressure chamber, on a portion of the surface of the limb stump covered by the membrane (5) during use of the apparatus (100), and/or between the membrane (5) and the limb stump, by means of a pressure measuring device;
- determining the weight that must be applied to the patient whose limb stump is inserted into the medical apparatus (100), so that the pressure measured by the pressure measuring device falls within a predetermined target range.

10. A method for determining an additional weight when fitting a plaster impression to a limb stump or when measuring a limb stump of a patient, comprising the steps of:
providing patient-related data;
entering patient-related data into a data collection device (97);
determining, based on patient-specific data, a weight with which the patient whose limb stump is inserted into the medical apparatus (100) according to any one of claims 1 through 6 must be weighted so that a pressure is exerted on the membrane (5), in the pressure chamber on a portion of the surface of the limb stump covered by the membrane (5) during use of the device (100) and/or between the membrane (5) and the limb stump falls within a predetermined target range.

11. A computing device (200) programmed, configured, and/or set up to perform a method according to claim 10.

12. A unit (400) comprising at least one medical apparatus (100) according to anyone of the claims 1 to 6, and at least one pressurisation control apparatus (410) for the pressure chamber of the medical apparatus (100), wherein the pressurisation control apparatus (410) preferably comprises at least:
- one pressure reservoir connection (413);
- one pressure relief valve;
- at least one connection (417, 421) to the pressure chamber with a control valve (419, 423) for increasing the pressure and/or with a control valve (419, 423) for reducing the pressure in the pressure chamber,
- optionally: a pressure gauge (425);
- optionally: an emergency stop device (427),
- optionally: a suction nozzle,
- wherein the pressurisation control apparatus (410) optionally comprises no electrical component and/or no power supply.

## Revendications

1. Dispositif médical (100) destiné à être utilisé dans la fabrication d'une empreinte en plâtre ou d'un modèle numérique d'un moignon de membre, en particulier d'un moignon de jambe, dans lequel le dispositif (100) comprend au moins :
- un récipient sous pression (1) avec une chambre à fluide ou une chambre de pression permettant d'accueillir ou de stocker un fluide sous pression, dans lequel le récipient sous pression (1) présente une paroi (3) constituée d'un premier matériau, dans lequel la paroi (3) délimite un intérieur du récipient sous pression (1) par rapport à un extérieur, dans lequel le récipient sous pression (1) présente une ouverture d'insertion (9) permettant d'insérer le moignon de membre à l'intérieur du récipient sous pression (1) ;
- une membrane étanche aux fluides (5) constituée d'un second matériau, qui est agencée pour former ou délimiter la chambre à fluide ou la chambre de pression ; et qui est reliée directement ou indirectement de manière étanche aux fluides à des sections de la paroi (3) du récipient sous pression (1) ; et au moins un connecteur,
dans lequel au moins une section (58) de la membrane (5) est fixée, de manière préférée de manière amovible, à une section de la paroi (3) au moyen d'au moins ledit connecteur (53), **caractérisé en ce que** le connecteur (53) est relié à la membrane (5) dans une section de la membrane (5) qui ne se trouve pas dans la région de l'ouverture d'insertion (9).

2. Dispositif médical (100) selon la revendication précédente, dans lequel au moins une section (58), de manière préférée centrale ou médiane, de la membrane (5) est fixée, de manière préférée de manière amovible, à une surface inférieure (4a) ou à la seconde face d'extrémité (4) du récipient sous pression (1), et de manière préférée dans une région ou section médiane ou centrale de celle-ci, au moyen d'au moins un connecteur (53).

3. Dispositif médical (100) selon l'une quelconque des revendications 1 ou 2, dans lequel le connecteur (53) n'est pas élastique et/ou pas extensible.

4. Dispositif médical (100) selon l'une quelconque des revendications précédentes, dans lequel le connecteur (53), la membrane (5) et/ou la surface inférieure (4a) présente au moins un filetage (65) permettant de visser, directement ou indirectement, la membrane (5) ou un élément qui lui est relié, au récipient sous pression (1).

5. Dispositif médical (100) selon l'une quelconque des revendications précédentes, dans lequel la membrane (5) est fabriquée à partir d'un matériau, ou comprend un matériau, qui présente des fibres (55a, 57a ; 55b, 57b) dans une première direction et/ou dans une seconde direction du matériau, lesquelles fibres sont incorporées de manière préférée dans une matrice ou sont reliées de manière préférée à celle-ci d'une autre manière.

6. Dispositif médical (100) selon l'une quelconque des revendications précédentes, dans lequel la membrane (5) n'est pas extensible ou pas élastique dans une première direction (L) de celle-ci et/ou dans une seconde direction de celle-ci.

7. Ensemble comprenant
- au moins un dispositif médical (100) selon l'une quelconque des revendications 1 à 6 ; et
- au moins deux membranes (5) qui diffèrent l'une de l'autre par au moins une propriété, et/ou au moins un poids permettant d'augmenter ou d'amplifier temporairement le poids corporel du patient, et/ou au moins un dispositif de soulagement, et/ou au moins un adaptateur (207) permettant de relier ou fixer de manière amovible le moignon de membre au récipient sous pression (1) ; et
- éventuellement au moins un bas autofixant (215) à tirer et/ou à glisser sur le moignon de membre, dans lequel le bas autofixant (215) relié au moignon de membre est relié par complémentarité de force et/ou par friction à une surface, en particulier à la surface de la membrane (5), sur ou dans le récipient sous pression (1).

8. Procédé permettant de poser une empreinte en plâtre au niveau d'un moignon de membre ou de mesurer le moignon de membre d'un patient, comprenant les étapes consistant à :
- fournir un dispositif médical (100) selon l'une quelconque des revendications 1 à 6 et éventuellement au moins une surface de support (111), dans lequel le dispositif (100), lorsqu'il repose sur la surface de support (111), peut être incliné, tourné et/ou déplacé par rapport à la surface de support (111) ;
- et remplir la chambre à fluide ou la chambre de pression du récipient sous pression (1) avec un fluide, en particulier un liquide, ou modifier le niveau de liquide à l'intérieur de la chambre à fluide ou de la chambre de pression de telle manière que la membrane (5) est recouverte par le liquide au moins dans certaines sections de celle-ci sur toute la circonférence des dites sections, ou de telle manière que la membrane (5) déborde à l'extérieur du récipient sous pression (1) par l'ouverture d'entrée (9) du récipient sous pression (1).

9. Procédé permettant de poser une empreinte en plâtre au niveau d'un moignon de membre ou de mesurer le moignon de membre d'un patient, en particulier selon la revendication 8, comprenant les étapes consistant à :
- fournir un dispositif médical (100) selon l'une quelconque des revendications 1 à 6 ou un ensemble selon la revendication 7 ;
- insérer le moignon de membre, éventuellement enveloppé d'un bandage plâtré humide (125), dans la membrane (5) de telle manière que le moignon de membre est, au moins dans certaines sections de celui-ci, entouré par la membrane (5) sur toute sa circonférence ;
- déterminer la pression exercée sur la membrane (5) dans la chambre de pression, sur une section de la surface du moignon de membre recouvert par la membrane (5) lors de l'utilisation du dispositif (100) et/ou entre la membrane (5) et le moignon de membre, au moyen d'un dispositif de mesure de pression ;
- déterminer un poids avec lequel le patient, dont le moignon de membre est inséré dans le dispositif médical (100), doit être lesté afin que la pression mesurée par le dispositif de mesure de pression se situe dans une plage de valeurs cibles prédéterminée.

10. Procédé permettant de déterminer un poids d'ajout lors de la pose d'une empreinte en plâtre au niveau d'un moignon de membre ou lors de la mesure du moignon de membre d'un patient, comprenant les étapes consistant à :
fournir des données relatives aux patients ;
saisir les données relatives au patient dans un dispositif de caractérisation (97) ;
déterminer, en se référant aux données relatives au patient, un poids avec lequel le patient, dont le moignon de membre est inséré dans le dispositif médical (100) selon l'une quelconque des revendications 1 à 6, doit être lesté afin que la pression exercée sur la membrane (5), dans la chambre de pression sur une section de la surface du moignon de membre recouverte par la membrane (5) lors de l'utilisation du dispositif (100) et/ou entre la membrane (5) et le moignon de membre, se situe dans une plage de valeurs cibles prédéterminée.

11. Dispositif informatique (200), programmé, configuré et/ou conçu pour mettre en œuvre un procédé selon la revendication 10.

12. Unité (400) comprenant au moins un dispositif médical (100) selon l'une quelconque des revendications 1 à 6, et au moins un dispositif de commande de pression (410) destiné à la chambre de pression du dispositif médical (100), dans lequel le dispositif de commande de pression (410) présente de manière préférée au moins :
- un raccord de réservoir de pression (413) ;
- une soupape de limitation de pression ;
- au moins un raccord (417, 421) au niveau de la chambre de pression, avec une soupape de régulation (419, 423) permettant d'augmenter la pression et/ou avec une soupape de régulation (419, 423) permettant de réduire la pression dans la chambre de pression,
- éventuellement : un indicateur de pression (425) ;
- éventuellement : un dispositif d'arrêt d'urgence (427),
- éventuellement : un embout d'aspiration,
- dans laquelle le dispositif de commande de pression (410) ne présente éventuellement aucun composant électrique et/ou aucun raccord électrique.
